# EUROPEAN PATENT SPECIFICATION

(11) **EP 1 364 069 B1**
(45) Date of publication and mention of the grant of the patent: **22.04.2009**
(21) Application number: 02724190.0
(22) Date of filing: 01.03.2002
(51) Int. Cl.: C12Q 1/68

(54) **METHOD FOR THE DEVELOPMENT OF GENE PANELS FOR DIAGNOSTIC AND THERAPEUTIC PURPOSES BASED ON THE EXPRESSION AND METHYLATOIN STATUS OF THE GENES**
VERFAHREN ZUR ENTWICKLUNG VON GENSÄTZEN ZU DIAGNOSTISCHEN UND THERAPEUTISCHEN ZWECKEN AUF GRUNDLAGE DES EXPRESSIONS- UND METHYLIERUNGSSTATUS DER GENE
PROCEDE DE MISE AU POINT DE GROUPES D'ECHANTILLONS DE GENES A DES FINS DE DIAGNOSTIC ET DE THERAPIE QUI SONT BASES SUR L'EXPRESSION ET L'ETAT DE METHYLATION DES GENES

(30) Priority: 01.03.2001 US 272549 P
(43) Date of publication of application: 26.11.2003
(73) Proprietor: Epigenomics AG, 10178 Berlin (DE)
(72) Inventor: OLEK, Alexander, 10115 Berlin (DE); BERLIN, Kurt, 14532 Stahndorf (DE)
(74) Representative: Krauss, Jan
(86) International application number: PCT/EP2002/002255
(87) International publication number: WO 2002/070742

(56) References cited:
- WO-A-00/39347
- WO-A-00/44934
- WO-A-00/70090
- WO-A-01/18241
- HERMAN J G ET AL: "INCIDENCE AND FUNCTIONAL CONSEQUENCES OF HMLH1 PROMOTOR HYPERMETHYLATION IN COLORECTAL CARCINOMA" PROCEEDINGS OF THE NATIONAL ACADEMY OF SCIENCES OF USA, NATIONAL ACADEMY OF SCIENCE. WASHINGTON, US, vol. 95, June 1998 (1998-06), pages 6870-6875, XP002944676 ISSN: 0027-8424
- GUO YONGJING ET AL: "DNA methylation status of uPA promoter directly affects the expression of uPA in human breast cancer cells to alter their invasive potential" PROCEEDINGS OF THE ANNUAL MEETING OF THE AMERICAN ASSOCIATION FOR CANCER RESEARCH, NEW YORK, NY, US, vol. 42, March 2001 (2001-03), pages 625-626, XP002195787 ISSN: 0197-016X
- WU HUA-KANG ET AL: "Transcriptional activation of human LIM-HOX gene hLH-2 in chronic myelogenous leukemia is due to a cis-acting effect of Bcr-Abl." BIOCHEMICAL AND BIOPHYSICAL RESEARCH COMMUNICATIONS, vol. 234, no. 3, 1997, pages 742-747, XP002203941 ISSN: 0006-291X

## Description

### FIELD OF THE INVENTION

The present invention concerns a method for the development of gene panels for diagnostic and therapeutic purposes. The invention further concerns gene panels developed using the method of the present invention and their uses.

### BACKGROUND OF THE INVENTION

The levels of observation that have been well studied by the methodological developments of recent years in molecular biology include the gene itself, the translation of genes in RNA, and the resulting proteins. When, during the course of the development of an individual, a gene is switched on, and how the activation and inhibition of certain genes in certain cells and tissues is controlled, can be correlated with a high degree of probability with the extent and the character of the methylation of the gene or the genome. In this regard, it is reasonable to assume that pathogenic conditions are expressed in a modified methylation pattern of individual genes or of the genome.

### STATE OF THE ART

### 1. State of the art of molecular analysis of cell phenotypes

The study of gene expression can be at the RNA level or at the protein level. Both levels in principle reflect important phenotypic parameters. Protein assays using two-dimensional gels (McFarrel method) have been known for approximately 15 years. Using these assays, it is possible to elaborate the analysis of the chromatographic positions of several thousand proteins. Very early on, such electropherograms were already processed or evaluated with data processing means. In principle, the validity of the method is high, however, it is inferior to the modem methods of gene expression based on RNA analysis in two regards.

The global analysis of cellular proteins has recently been termed proteomics and is a key area of research that is developing in the post-genome era. Proteomics uses a combination of sophisticated techniques including two-dimensional (2D) gel electrophoresis, image analysis, mass spectrometry, amino acid sequencing, and bio-informatics to resolve comprehensively, to quantify, and to characterize proteins (for reviews, see Chambers G et al. "Proteomics: a new approach to the study of disease" J Pathol 2000 Nov;192(3):280-8; Banks RE et al. "Proteomics: new perspectives, new biomedical opportunities" Lancet 2000 Nov 18;356(9243):1749-56).

Proteomics is further said to contribute greatly to the understanding of gene function in the post-genomic era. Proteomics can be divided into three main areas: (1) protein micro-characterization for large-scale identification of proteins and their post-translational modifications; (2) 'differential display' proteomics for comparison of protein levels with potential application in a wide range of diseases; and (3) studies of protein-protein interactions using techniques such as mass spectrometry or the yeast two-hybrid system. Because it is often difficult to predict the function of a protein based on homology to other proteins or even their three-dimensional structure, determination of the components of a protein complex or of a cellular structure is central in functional analysis (Pandey A et al. "Proteomics to study genes and genomes." Nature 2000 Jun 15;405(6788):837-46).

Due to the complexity of higher eukaryotic cells, single-step characterization of a proteome is likely to be difficult to achieve. Jung et al. ("Proteomics meets cell biology: the establishment of subcellular proteomes" Electrophoresis 2000 Oct;21(16):3369-77) describe, that advantage can be taken of the macromolecular architecture of a cell, e.g., subcellular compartments, organelles, macromolecular structures and multiprotein complexes, to establish subcellular proteomes.

Despite the recent developments in the art, the detection of proteins that are of regulatory importance, from small quantities of cells still fails because of the fact that the sensitivity of the methods used is much too low. Indeed, in contrast to nucleic acids, proteins cannot be amplified. In addition, the method is very complex, not amenable to automation, and very expensive. In contrast, RNA analysis presents considerable advantages, and due to of the use of PCR it is more sensitive. Above all, each RNA species recognized to be important can be identified immediately by its sequence.

Overexpression or underexpression of individual RNAs with a known sequence can usually be easily detected; however, in connection with the applications discussed here, they are only valid in exceptional cases.

The method of "differential displays" at best allows a semiquantitative study of expression. Expression products amplified by PCR are separated by gel electrophoresis. The validity is limited as a result of the resolution of the gel electrophoresis. In addition, the method is insufficiently sensitive and robust for use in routine diagnosis (Liang, P. and Pardee, A. B., Science 257, 967-971).

Genes with high overexpression or underexpression are frequently identified by subtractive techniques. Here, cDNA clones of a cell or tissue species to be examined are plated. Against the clones, cDNA is hybridized as comparison material. Expression patterns cannot be reliably prepared using this technique.

Sturtevant et al. ("Applications of differential-display reverse transcription-PCR to molecular pathogenesis and medical mycology. Clin Microbiol Rev 2000 Jul;13(3):408-27) describe the characterisation of host-fungus interactions by changes in gene expression in both host and pathogen. Differential-display reverse transcription PCR (DDRT-PCR) is a PCR-based method that allows extensive analysis of gene expression among several cell populations. DDRT-PCR has been used to address biological questions in mammalian systems, including cell differentiation, cell activation, cell stress, and identification of drug targets. In microbial pathogenesis and plant pathogenesis, DDRT-PCR has allowed the identification of virulence factors, genes involved in cell death, and signaling genes. Further, To ("Identification of differential gene expression by high throughput analysis" Comb Chem High Throughput Screen 2000 Jun;3(3):235-41) describes the high throughput analysis of differential gene expression as a powerful tool that can be applied to many areas in molecular cell biology, including differentiation, development, physiology, and pharmacology. In recent years, a variety of techniques have been developed to analyze differential gene expression, including comparative expressed sequence tag sequencing, differential display, representational difference analysis, cDNA or oligonucleotide arrays, and serial analysis of gene expression. Similar strategies are described by Oetting ("Gene expression analysis. Pigment Cell Res 2000 Feb;13(1):21-7) and Watson ("Differential cDNA screening strategies to identify novel stage-specific proteins in the developing mammalian brain. Dev Neurosci 1993;15(2):77-86).

One activity of the American "Human Genome Project" is the systematic sequencing of expressed genes. The data obtained from this can be used to build expression chips, which allow the study of practically all expressed sequences of a cell or tissue type in a single experiment.

### 2. State of the art in the analysis of cancer diseases

Mutations in genes can trigger cancer diseases, that is, cell transformation. The causes of these mutations can be exogenous influences, or events in the cell. In a few exceptional cases, an individual mutation, which frequently affects larger regions of the genome (translocations, deletions), results in the degeneration of the cell; but in most cases a chain of mutations on different genes is involved, and it is only their combined effect that results in the malignant disease. These changes at the DNA level are also reflected on the RNA and protein levels. In this context, it is highly probable that a multiplication occurs, because it is certain that in many cases the quantity and type of one RNA influences the extent of the synthesis of several other RNA species. This leads to a change in the synthesis rates of the corresponding proteins, which, in turn, can result in the deregulation of metabolism, and thus initiate the mechanism of regulation and counter regulation. The result is a gene expression pattern of the cells in question, that has been modified in a very specific (but largely nondeterminable) manner,the specificity is for a certain carcinoma, for the stage of the carcinoma, and the degree of malignancy of the carcinoma. So far, such phenomena have been outside the realm of study of natural sciences. Indeed, it has been impossible to examine the gene expression or the metabolism of a cell in its totality. Chip technology for the first time provided such a possibility (Schena, M. et al., Science 270, 467-470).

If one wishes to solve the diagnostic problem of early diagnosis of tumors on the molecular level, then one is confronted, today, with an insurmountable difficulty. With very few exceptions, for most tumors, the knowledge of the molecular events, that is, the different mutations, is only fragmentary. Researchers do not know what to look for in medical examination material. This means it is absolutely impossible to apply the remarkable sensitivity and specificity of the polymerase chain reaction. Examples are certain intestinal tumors, Ewing's sarcoma, and certain forms of leukemia, which are in fact each defined by a single, precisely described mutation. In those cases, it is possible to identify the degenerated cell among millions of normal cells. However, even within these apparently unambiguously defined tumor groups, there are such differences in the behavior that the conclusion must be drawn that additional unknown genetic parameters (such as, for example, the genetic background of the individual) play an important role. Immunological tumor markers are helpful auxiliary parameters, but they continue to make only a modest contribution, in addition to the other conventional diagnostic parameters. However, they can be used for the purpose of preselecting suspect cells.

Histology plays an important and indispensable role in the identification of degenerated tissues, but not precisely in early diagnosis.

Thus, because most tumors are not sufficiently characterized for diagnostic purposes on the molecular level, as a rule, no possibilities exist to proceed to a subdivision into stages or even a subdivision by degrees of risk. Such a subdivision, however, is an absolute prerequisite for an improved selection of treatments and, above all, for the development of effective new drugs and of gene therapy.

### 3. State of the art in research on the number, type and properties of the possible stable states of cells of higher organisms

In recent times, there has been an increase in the number of indications that complex regulatory systems (an excellent example of which is cell regulation), when left alone, can exist in only a limited number of stable states, above a critical minimum complexity and below a critical maximum connectivity (of the average number of the components, with which any given component is connected) (Kauffman, S. A., Origins of Order, Oxford University Press, 1993). In this context, the word state should be understood as the concept of selection for the general phenomenon. In connection with cells as biological regulatory systems, one can also talk of differentiation state or cell type. Although no such connection has been demonstratedand even a mere limitation of the possible states for biological systems has not been demonstrated--the practical implications would be of very great importance: if, regarding the constant information content of the cells of an organism (de facto, such constancy essentially exists within one species), there were only a limited number of stable states, then it would be likely that degenerated cells could also be in only one of these states or in a transition between the possible states. At this time, it is not possible to define these states on a molecular basis. It is hardly possible to achieve a correlation between the individual states and the behavior of the cells according to the state of the art. However, such an analysis could make decisive contributions to the diagnosis and prognosis of diseases. It is even possible that a correlation could be established between the possible states of diseased cells and the best suited therapy. Furthermore, it is probable that such a method could also have a decisive influence in the selection of the time of treatment. For example, if one were to discover that the cells of a tumor are in a transition between possible states, one could assume that such a population of cells would be more likely to yield to the selection pressure resulting from the treatment, and thus could escape more easily. A cell population in such a scenario, within such transitional states, would have a considerably increased flexibility, and it would be easily forced into a possible stable state, in which the selection pressure would be eliminated, and the treatment would thus be without effect.

A method which could classify cells and cell groups according to states would then also contribute to recognizing, understanding and possibly solving such problems. However, according to the state of the art, it is not possible to determine whether only a limited number of states of cells exists. It follows that it is not possible to differentiate groups of cells according to an abstract criterion concerning their states, and to predict these states with a certain behavior of the cells.

### 4. Hereditary diseases

Today, the genetic map of the human genome comprises 2500 so-called microsatellites. These instruments are used to locate a multitude of genes, usually genes whose defect causes a genetic disease, per linkage analysis, and then to identify them. Common genetic diseases caused by a single defective gene are thus elucidated, from the point of view of the geneticist's principle, polygenic diseases should also be understood in this manner. Many polygenic diseases are very common, so common that they are included among the so-called widespread diseases. Asthma and diabetes are examples. Many carcinoma types are also included. The use of the above described strategy of linkage analysis also produced enormous initial successes. In many instances, numerous causal genes of important polygenic diseases such as diabetes, schizophrenia, atherosclerosis and obesity have been found. Besides the availability of the molecular biology laboratory techniques proper, the availability of a relatively large number of patients and relatives affected by each disease is a crucial prerequisite for genetic elucidation. In the past two years it has become apparent that the number of several hundred patients that were originally used for the linkage analysis of polygenic diseases very likely is too low by one order of magnitude. This applies, in any case, to cases where the entire spectrum of the causal gene is to be elucidated. Because the level of manual work required for such a linkage analysis is extraordinarily high, only very slow progress can be expected in the analysis of polygenic diseases. Alternative strategies are sought because it is precisely these diseases that are of enormous social and economic importance.

### 5. State of the art in methylation analysis

The modification of the genomic base cytosine to 5'-methylcytosine represents the epigenetic parameter which to date is the most important one and has been best examined. Nevertheless, methods exist today to determine comprehensive genotypes of cells and individuals, but no comparable methods exist to date to generate and evaluate epigenotypic information on a large scale.

In principle, there are three methods that differ in principle for determining the 5-methyl state of a cytosine in the sequence context.

The first method is based in principle on the use of restriction endonucleases (RE), which are methylation-sensitive". REs are **characterized in that** they produce a cut in the DNA at a certain DNA sequence which is usually 4-8 bases long. The position of such cuts can be detected by gel electrophoresis, transfer to a membrane and hybridization. Methylation-sensitive means that certain bases within the recognition sequence must be unmethylated for the step to occur. The band pattern after a restriction cut and gel electrophoresis thus changes depending on the methylation pattern of the DNA. However, most CpG that can be methylated are outside of the recognition sequences of REs, and thus cannot be examined.

The sensitivity of this method is extremely low (Bird, A. P., Southern, E. M., J. Mol. Biol. 118, 27-47). A variant combines PCR with this method; an amplification by two primers located on both sides of the recognition sequence occurs after a cut only if the recognition sequence is in the methylated form. In this case, the sensitivity theoretically increases to a single molecule of the target sequence; however, only individual positions can be examined, at great cost (Shemer, R. et al., PNAS 93, 6371-6376).

The second variant is based on the partial chemical cleavage of whole DNA, using the model of a Maxam-Gilbert sequencing reaction, ligation of adaptors to the ends thus generated, amplification with generic primers, and separation by gel electrophoresis. Using this method, defined regions having a size of less than thousands of base pairs can be examined. However, the method is so complicated and unreliable that it is practically no longer used (Ward, C, et al., J. Biol. Chem. 265, 3030-3033).

A new method for the examination of DNA to determine the presence of 5-methylcytosine is based on the specific reaction of bisulfite with cytosine. The latter is converted under appropriate conditions into uracil, which, as far as base pairing is concerned, is equivalent to thymidine, and which also corresponds to another base. 5-Methylcytosine is not modified. As a result, the original DNA is converted in such a manner that methylcytosine, which originally could not be distinguished from cytosine by its hybridization behavior, now can be detected by "normal" molecular biological techniques. All of these techniques are based on base pairing, which can now be completely exploited. The state of the art, as far as sensitivity is concerned, is defined by a method which includes the DNA to be examined in an agarose matrix, intended to prevent the diffusion and renaturing of the DNA (bisulfite reacts only with single-stranded DNA) and to replace all precipitation and purification steps by rapid dialysis (Olek, A., et al., Nucl. Acids. Res. 24, 5064-5066). Using this method, individual cells can be examined, which illustrates the potential of the method. However, so far only individual regions up to approximately 3000 base pairs in length have been examined, and an overall examination of cells to identify thousands of possible methylation events is not possible. However, this method is not capable of reliably analyzing minute fragments from small sample quantities. In spite of protection against diffusion, such samples are lost through the matrix.

### 6. State of the art with respect to Methylation and the diagnosis of human diseases

In the past, modification of the methylation pattern was analyzed in order to study and understand the genetic mechanisms which are involved in the outbreak or the progression of a disease. All this research was done in a piece-by-piece fashion by studying only one gene/chromosomal region at a time and no diagnosis/therapeutic treatment regimen was based on the methylation pattern modifications. In fact, the type of disease associated with the modification of the methylation pattern was known before methylation analysis was performed. Therefore, the following publications only indicate the widespread connection between modifications of the methylation patterns and human diseases. Modifications can include both hyper- or hypomethylation of selected sites of the DNA.

Disease associated with a modification of the methylation patterns are, for example:
- Leukemia (Aoki E et al. "Methylation status of the p15INK4B gene in hematopoietic progenitors and peripheral blood cells in myelodysplastic syndromes" Leukemia 2000 Apr;14(4):586-93; Nosaka K et al. "Increasing methylation of the CDKN2A gene is associated with the progression of adult T-cell leukemia" Cancer Res 2000 Feb 15;60(4):1043-8; Asimakopoulos FA et al. "ABL1 methylation is a distinct molecular event associated with clonal evolution of chronic myeloid leukemia" Blood 1999 Oct 1;94(7):2452-60; Fajkusova L. et al. "Detailed Mapping of Methylcytosine Positions at the CpG Island Surrounding the Pa Promoter at the bcr-abl Locus in CML Patients and in Two Cell Lines, K562 and BV 173" Blood Cells Mol Dis 2000 Jun;26(3):193-204; Litz CE et al. "Methylation status of the major breakpoint cluster region in Philadelphia chromosome negative leukemias" Leukemia 1992 Jan;6(1):35-41)
- Head and neck cancer (Sanchez-Cespedes M et al. "Gene promoter hypermethylation in tumors and serum of head and neck cancer patients" Cancer Res 2000 Feb 15;60(4):892-5)
- Hodgkin's disease (Garcia JF et al. "Loss of p16 protein expression associated with methylation of the p16INK4A gene is a frequent finding in Hodgkin's disease" Lab Invest 1999 Dec;79(12): 1453-9)
- Gastric cancer (Yanagisawa Y et al. "Methylation of the hMLH1 promoter in familial gastric cancer with microsatellite instability" Int J Cancer 2000 Jan 1;85(1):50-3)
- Prostate cancer (Rennie PS et al. "Epigenetic mechanisms for progression of prostate cancer" Cancer Metastasis Rev 1998-99;17(4):401-9)
- Renal cancer (Clifford SC et al. "Inactivation of the von Hippel-Lindau (VHL) tumor suppressor gene and allelic losses at chromosome arm 3p in primary renal cell carcinoma: evidence for a VHL-independent pathway in clear cell renal tumourigenesis" Genes Chromosomes Cancer 1998 Jul;22(3):200-9)
- Bladder cancer (Sardi I et al. "Molecular genetic alterations of c-myc oncogene in superficial and locally advanced bladder cancer" Eur Urol 1998;33(4):424-30)
- Breast cancer (Mancini DN et al. "CpG methylation within the 5' regulatory region of the BRCA1 gene is tumor specific and includes a putative CREB binding site" Oncogene 1998 Mar 5;16(9):1161-9; Zrihan-Licht S et al. "DNA methylation status of the MUC1 gene coding for a breast-cancer-associated protein" Int J Cancer 1995 Jul 28;62(3):245-51; Kass DH et al. "Examination of DNA methylation of chromosomal hot spots associated with breast cancer" Anticancer Res 1993 Sep-Oct;13(5A):1245-51)
- Burkitt's lymphoma (Tao Q et al. "Epstein-Barr virus (EBV) in endemic Burkitt's lymphoma: molecular analysis of primary tumor tissue" Blood 1998 Feb 15;91(4):1373-81)
- Wilms tumor (Kleymenova EV et al. "Identification of a tumor-specific methylation site in the Wilms tumor suppressor gene" Oncogene 1998 Feb 12;16(6):713-20)
- Prader-Willi/Angelman syndrome (Zeschnigh et al. "Imprinted segments in the human genome: different DNA methylation patterns in the Prader-Willi/Angelman syndrome region as determined by the genomic sequencing method" Human Mol. Genetics (1997) (6)3 pp 387-395; Fang P et al. "The spectrum of mutations in UBE3A causing Angelman syndrome" Hum Mol Genet 1999 Jan;8(1):129-35)
- ICF syndrome (Tuck-Muller et al. "CMDNA hypomethylation and unusual chromosome instability in cell lines from ICF syndrome patients" Cytogenet Cell Genet 2000;89(1-2):121-8)
- Dermatofibroma (Chen TC et al. "Dermatofibroma is a clonal proliferative disease" J Cutan Pathol 2000 Jan;27(1):36-9)
- Hypertension (Lee SD et al. "Monoclonal endothelial cell proliferation is present in primary but not secondary pulmonary hypertension" J Clin Invest 1998 Mar 1;101(5):927-34)
- Pediatric Neurobiology (Campos-Castello J et al. "The phenomenon of genomic "imprinting" and its implications in clinical neuropediatrics" Rev Neurol 1999 Jan 1-15;28(1):69-73)
- Autism (Klauck SM et al. "Molecular genetic analysis of the FMR-1 gene in a large collection of autistic patients" Hum Genet 1997 Aug;100(2):224-9)
- Ulcerative colitis (Gloria L et al. "DNA hypomethylation and proliferative activity are increased in the rectal mucosa of patients with long-standing ulcerative colitis" Cancer 1996 Dec 1;78(11):2300-6)
- Fragile X syndrome (Hornstra IK et al. "High resolution methylation analysis of the FMR1 gene trinucleotide repeat region in fragile X syndrome" Hum Mol Genet 1993 Oct;2(10):1659-65)
- Huntington's disease (Ferluga J et al. "Possible organ and age-related epigenetic factors in Huntington's disease and colorectal carcinoma" Med Hypotheses 1989 May;29(1):51-4)

In view of the above, despite the advance in the art of the analysis of gene expression, a screening and/or the diagnosis for a potential disease or medical condition is still a laborious and time consuming task, since in order to achieve a reliable result one has to analyse a vast number of differently expressed genes in parallel.

This makes the analyses unreliable, time consuming, expensive, non-automateable and limits it to the analysis of single genes. No methods exist so far which address these problems to reasonably scale down the effort which has to be applied in order to achieve a result while maintaining its statistical quality.

Therefore, it is an object of the present invention to provide a method which allows for a streamlining of the effort for a reliable cell expression analysis. This analysis shall then be used for less time consuming and more effective diagnostic and therapeutic purposes, in particular in personalised medical treatments.

A further object of the invention is to provide the gene panel which is obtained using a method according to the invention for the use in a treatment of a disease and/or medical condition.

A further object of the invention is to provide systems, methods and computer program products for performing any of the inventive methods.

A further object of the invention is to provide a treatment of a disease and/or medical condition, based on a gene panel according to the invention.

This object is solved according to the present invention by providing a method for the development of gene panels for diagnostic and therapeutic purposes, which comprises the steps of: a)isolating at least one biological sample from each of at least two groups of biological material containing mRNA and/or proteins; b) analysing the expression level of at least one gene in the at least one biological samples; c) selecting the gene(s) exhibiting a different expression level between said at least two groups of biological material, whereby a first knowledge base is generated; d) analysing the level of cytosine methylation in the methylation relevant regions of at least one gene of at least one of the biological samples of step a), wherein the gene is selected on the basis of the first knowledge base; e) selecting the gene(s) exhibiting a different level of cytosine methylation between said at least two groups of biological material, whereby a second knowledge base is generated; and f) adding selected genes from the second knowledge base to a gene panel.

Thus, the present invention provides a method for generating a gene panel combining only the advantages of the presently known expression analysis techniques, which results in a powerful tool for the fast and reliable diagnosis and/or therapy of a enormous number of diseases and/or medical conditions.

Celis et al. ("Gene expression profiling: monitoring transcription and translation products using DNA microarrays and proteomics" FEBS Lett 2000 Aug 25;480(1):2-16) describe a theoretical approach to combining different technologies such as DNA microarrays and proteomics, which have made possible the analysis of the expression levels of multiple genes simultaneously both in health and disease. In combination, these technologies are said to revolutionise biology, in particular in the area of molecular medicine as they are expected to reveal gene regulation events involved in disease progression as well as to pinpoint potential targets for drug discovery and diagnostics. Celis et al. review the current status of these technologies and highlight some studies in which they have been applied in concert to the analysis of biopsy specimens. Nevertheless, Celis et al. fail to describe or propose the combination of, in particular, data obtained in proteomics expression studies and methylation analyses in order to provide gene panels for further therapeutic or diagnostic purposes.

Other preferred embodiments of the invention will become apparent to the person skilled in the art after reading the features of the dependent claims.

In one embodiment of the method according to the invention, the biological sample is isolated by means of a biopsy, by means of an operation on an individual, by means of a dissection, derived from a preserved biological sample, collected from body fluid(s) and/or collected directly from the environment.

In another embodiment of the method according to the invention, the biological sample comprises a eucaryotic and/or procaryotic cell line, a biopsy sample, blood, sputum, faeces, urine, cerebral liquid, tissue embedded in paraffin, tissue derived from eyes, intestine, brain, heart, prostata, kidney, lung, breast or liver, histological samples or a combination thereof.

A preferred method according to the invention is characterised in that the at least one biological sample is derived from biological material of healthy and/or diseased individuals. Such diseases include all diseases and/or medical conditions which involve a modification of the expression of genes of the cell and include, for example, unwanted side effects of medicaments, cancers, metastasis, dysfunctions, damages or diseases of the central nervous system (CNS), aggressive symptoms or behavioural disorders, clinical, psychological and social consequences of brain injuries, psychotic disorders and disorders of the personality, dementia and/or associates syndromes, cardiovascular diseases, malfunctions or damages, diseases, malfunctions or damages of the gastrointestine, diseases, malfunctions or damages of the respiratory system, injury, inflammation, infection, immunity and/or reconvalescence, diseases, malfunctions or damages as consequences of modifications in the developmental process, diseases, malfunctions or damages of the skin, muscles, connective tissue or bones, endocrine or metabolic diseases, malfunctions or damages, headache, and sexual malfunctions or combinations thereof, leukemia, head and neck cancer, Hodgkin's disease, gastric cancer, prostate cancer, renal cancer, bladder cancer, breast cancer, Burkitt's lymphoma, Wilms tumor, Prader-Willi/Angelman syndrome, ICF syndrome, dermatofibroma, hypertension, pediatric neurobiological diseases, autism, ulcerative colitis, fragile X syndrome, and Huntington's disease.

A preferred method according to the invention is characterised in that the isolation of said biological sample comprises isolating subcellular compartments, organelles, macromolecular structures and multiprotein complexes, partial or complete preparation of the mRNA, reverse transcription or partial digestion of the material with an enzyme selected from proteases, RNAses and/or DNAses or combinations thereof. Such isolations and/preselections can initially even further limit the amount and complexity of the genes which take part in the inventive method.

Further preferred is a method according to the invention in which the analysis of the expression level of the at least one gene in the biological sample comprises determining the relative amount of mRNA or protein derived from said at least one gene. In general, all currently known methods for the expression analysis of genes except methylation analysis can be used for this first step of the inventive method. Preferred are analyses that comprise one or more of one- or two-dimensional gel electrophoresis, differential display, analysis of selected sets of tumour markers, subtractive hybridisation, mass spectrometry, comparative expressed sequence tag sequencing, representational difference analysis, cDNA or oligonucleotide arrays, serial analysis of gene expression, enzymatic, fluorescent, radioactive, dye and/or antibody labelling. Even more preferred are methods according to the invention which comprise measuring intensities of expression during one- or two-dimensional gel electrophoresis, differential display, subtractive hybridisation, DNA, RNA or protein sequencing, mass spectrometry, and enzymatic, radioactive, dye and/or antibody labelling.

In another embodiment of the method according to the invention, the analysis is at least partially performed by means of a suited automate, for example a robot and/or a computer device. Such device would be equipped with the necessary software for the analysis of the expression and could be connected to an inter- or intranet, be part of a neural network or the like. The necessary data/information for the analyses can be present on the system directly or at a remote source, to which the device is directly or indirectly connected, for example via the internet.

In a preferred method according to the invention, the expression levels of at least two genes are analysed in parallel. More preferably, at least 100 genes are analysed in parallel.

In another preferred embodiment of the method according to the invention the selection is based on a combination of the analysis of both mRNA level and protein expression. In another embodiment of the inventive method, the selection is based on the result of at least two individual rows of analyses. This provides for an internal control run of the data which is used for the selection and increases the preciseness of the results. This will further reduce the statistical error for the value of the expression of a selected gene with an only limited increase of the costs for the analysis.

In another preferred method according to the invention, the selection is performed in such a way as to give a first knowledge base comprising only one set of selected genes. Thus, the knowledge base will comprise only "on" and "off" type of data which allows for a very simple decision between expressed or non-expressed genes. In yet another embodiment of the inventive method, the selection is performed in such a way as to give a first knowledge base comprising different subsets of selected genes. Such classes can be referred to as "quality classes" which allows for a much more differentiated selection of the gene panel. The term "quality classes" as used herein comprises all different possibilities of groupings the different expression levels. Such grouping could, for example, include different importance for the selected sites for the analysis of the expression as well as statistical preciseness and/or quality of the analysis data of the selected gene.

In a preferred method according to the invention, the selection is at least partially performed automatically by means of a suited automate, like a computer device. Such device would be equipped with the necessary software for the analysis of the expression and could be connected to an inter- or intranet, be part of a neural network or the like. The necessary data/information for the analyses can be present on the system directly or at a remote source, to which the device is directly or indirectly connected, for example via the internet.

In a preferred method according to the invention, at least two genes are selected in parallel. More preferably, at least 100 sites are selected in parallel.

According to the invention it is further preferred to analyse methylation relevant regions comprising the complete genes and/or promoters, introns, first exons and/or enhancers of the genes to be analysed. From the analysis of the methylation sites which are relevant for the expression of a certain gene, but not localised inside the sequence of the gene itself, the effect of the site for the expression of the gene can be readily extrapolated by the person skilled in the art.

In another embodiment of the method according to the invention, the analysis of the level of cytosine methylation comprises chemical treatment with bisulphite, hydrogen sulphite or disulphite, polymerase chain reaction (PCR), hybridisation analyses, sequencing, mass spectrometry and fluorescent, enzymatic, radioactive, dye and/or antibody labelling. In general, all methods for the analysis of the methylation statuses at selected sites of the DNA can be employed. Such methods are known to the skilled artisan and are described in, for example, Dahl et al., "Analysis of in vivo methylation." Methods Mol Biol 2000;130:47-57; Zhou Y. et al., "Use of a single sequencing termination reaction to distinguish between cytosine and 5-methylcytosine in bisulfite-modified DNA." Biotechniques 1997 May;22(5):850-4; Yoder JA et al. "Genetic analysis of genomic methylation patterns in plants and mammals." C Biol Chem 1996 Oct;377(10):605-10 and others.

Preferred is a method according to the invention in which the analysis is at least partially performed by means of a suited automate, for example a robot. Such device would be equipped with the necessary software for the analysis of the expression and could be connected to an inter- or intranet, be part of a neural network or the like. The necessary data/information for the analyses can be present on the system directly or at a remote source, to which the device is directly or indirectly connected, for example via the internet.

Another preferred method according to the invention is characterised in that all potentially methylated sites of the DNA are analysed. Such sites usually include all so-called "CpG"-islands on a given DNA sequence and are readily detectable by the person skilled in the art. Preferably, the level of cytosine methylation of at least two genes are analysed in parallel. Preferably, the level of at least 100 cytosine methylation sites is analysed in parallel. The analysis of a multitude of sites in parallel allows for both a effective screening and a statistically highly relevant result of the method.

A further preferred method according to the invention is characterised in that the selection is based on the result of at least two individual rows of analyses. This will reduce the statistical error for the value of the methylation sensitivity of a selected site with an only limited increase of the costs for the analysis. In another preferred method according to the invention, the selection is performed in such a way to give a second knowledge base comprising only one set of selected genes. Thus, the knowledge base will comprise only "on" and "off" type of data which allows for a very simple decision between different methylation states.

In yet another embodiment of the inventive method, the selection is performed in such a way to give a second knowledge base comprising different subsets of selected genes. Such classes can be referred to as "quality classes" which allows for a much more differentiated analysis.

In a preferred method according to the invention, the selection is at least partially performed automatically by means of a suited automate, like a computer device. Such device would be equipped with the necessary software for the analysis of the methylation sites and could be connected to an inter- or intranet, be part of a neural network or the like. The necessary data/information for the analyses can be present on the system directly or at a remote source, to which the device is directly or indirectly connected, for example via the internet.

In a preferred method according to the invention, at least two genes are selected in parallel. More preferably, at least 100 genes are selected in parallel.

Another embodiment of the method according to the invention is characterised in that all or a part of the genes of the second knowledge base are added to the gene panel.

In another embodiment of the method according to the invention, additional information about methylation relevant regions of the selected genes is added to the gene panel. This additional information can comprise personal patient data, disease specific data, prior treatment data and/or additional methylation specific data.

Another embodiment of the method according to the invention is characterised in that steps a) to f) are repeated. Repeating the method of the invention suits several different purposes. First, as mentioned above, the statistical quality of the of the resulting data increases. Second, an internal control can be provided, whether the biological sample was taken correctly and resembles e.g. the tissue of interest. The number of repeating "cycles" of the invention can vary depending on the individual case, e.g. depending on the quality of the sample to be analyse. One possibility would be to repeat the method of the invention for at least 5 to 50 times. Preferably, such method according to the invention is characterised in that the method is at least partially performed by means of a suited automate, for example a robot and/or a computer system. The inventive method can be conveniently automated and/or computerized and respective devices and programs are readily known to the person skilled in the art.

In another aspect according to the method according to the invention, the identical biological material, different biological material or a combination thereof is used in step a). Further, in another aspect according to the method according to the invention, the steps are performed in the following order: step a), step d), step e), step b), step c), and step f). This simply interchanges the order of the different steps of the inventive method which should nevertheless lead to a similar or identical result. According to the invention, this method can also at least partially be performed by means of a suited automate, for example a robot.

Another aspect of the present invention is related to a gene panel which is obtained according to a method according to the invention. This gene panel can be further used for the diagnosis and/therapy of different diseases, like, for example, unwanted side effects of medicaments, cancers, metastasis, dysfunctions, damages or diseases of the central nervous system (CNS), aggressive symptoms or behavioural disorders, clinical, psychological and social consequences of brain injuries, psychotic disorders and disorders of the personality, dementia and/or associates syndromes, cardiovascular diseases, malfunctions or damages, diseases, malfunctions or damages of the gastrointestine, diseases, malfunctions or damages of the respiratory system, injury, inflammation, infection, immunity and/or reconvalescence, diseases, malfunctions or damages as consequences of modifications in the developmental process, diseases, malfunctions or damages of the skin, muscles, connective tissue or bones, endocrine or metabolic diseases, malfunctions or damages, headache, and sexual malfunctions or combinations thereof, leukemia, head and neck cancer, Hodgkin's disease, gastric cancer, prostate cancer, renal cancer, bladder cancer, breast cancer, Burkitt's lymphoma, Wilms tumor, Prader-Willi/Angelman syndrome, ICF syndrome, dermatofibroma, hypertension, pediatric neurobiological diseases, autism, ulcerative colitis, fragile X syndrome, and Huntington's disease.

Further preferred is a gene panel according to the invention which comprises additional information data about methylation relevant regions of the selected genes, like the regions of the complete genes and/or promoters, introns, first exons and/or enhancers. According to the invention, the gene panel can be present in the form of a knowledge base on a computer disc, RAM, ROM, or a printed table or listing.

According to the present invention, a "gene panel" designates a knowledge base, listing, table or other information source, that contains information about selected genes, herein also designated as "candidate genes". According to the present invention, the term "gene panel" should not be understood as merely containing information about the names or designations of the candidate genes. The panel further can contain additional information about the candidate genes, like sequence data, information about the origin (heredity) of the gene, species information, and information about the genetic elements and/or factors that influence expression of the candidate gene(s). Such elements can be the complete genes and/or promoters, introns, first exons and/or enhancers of the candidate genes. Factors can be growth conditions, developmental stage of the biological material from which the candidate gene is derived or other medical data.

In another aspect of the invention, the gene panel can be used for the diagnosis of a disease. Such diseases can include unwanted side effects of medicaments, cancers, metastasis, dysfunctions, damages or diseases of the central nerval system (CNS), aggressive symptoms or behavioural disorders, clinical, psychological and social consequences of brain injuries, psychotic disorders and disorders of the personality, dementia and/or associates syndromes, cardiovascular diseases, malfunctions or damages, diseases, malfunctions or damages of the gastrointestine, diseases, malfunctions or damages of the respiratory system, injury, inflammation, infection, immunity and/or reconvalescence, diseases, malfunctions or damages as consequences of modifications in the developmental process, diseases, malfunctions or damages of the skin, muscles, connective tissue or bones, endocrine or metabolic diseases, malfunctions or damages, headache, and sexual malfunctions or combinations thereof. Particularly preferred is a use according to the invention which is characterised in that the genes are related with leukemia, head and neck cancer, Hodgkin's disease, gastric cancer, prostate cancer, renal cancer, bladder cancer, breast cancer, Burkitt's, lymphoma, Wilms tumor, Prader-Willi/Angelman syndrome, ICF syndrome, dermatofibroma, hypertension, pediatric neurobiological diseases, autism, ulcerative colitis, fragile X syndrome, and Huntington's disease.

Another aspect of the present invention is related to a device for the generation of a gene panel according to the invention, which comprises means for generating a first and second knowledge base according to invention and means for adding selected genes from the second knowledge base to a gene panel.

Another aspect of the present invention is related to a method for the diagnosis of a disease, comprising the following steps: a) providing a gene panel according to the invention; b) analysing the level of cytosine methylation at selected sites of the DNA based on said gene panel in biological material of at least one diseased individual with a known disease or medical condition and/or at least one healthy individual, thereby generating a first knowledge base; c) analysing the level of cytosine methylation at selected sites of the DNA based on said gene panel in biological material of at least one diseased individual with an unknown disease or medical condition, thereby generating a second knowledge base; and d) providing a third knowledge base comprising a plurality of expert rules for comparing the first and second knowledge base; e) selecting a type of disease or medical condition for the at least one diseased individual with an unknown disease or medical condition based on said first to third knowledge bases.

A preferred method for the diagnosis of a disease is characterised in that the selected sites of the genes to be analysed are located in the promoters, introns, first exons and/or enhancers or combinations thereof. The inventive method can further comprise the analysis of the level of cytosine methylation comprises chemical treatment with bisulphite, hydrogen sulphite or disulphite, polymerase chain reaction (PCR), hybridisation analyses, sequencing, mass spectrometry and fluorescent, enzymatic, radioactive, dye and/or antibody labelling. Preferably, the method according to the invention is characterised in that the analysis is at least partially performed by means of a suited automate, for example a robot.

Further, in another embodiment of the inventive method, steps b) to d) are repeated before performing step e). Preferably, the identical biological material, different biological material or a combination thereof is used in step c).

In a preferred embodiment, the method according to the invention is at least partially performed by means of a suited automate, for example a robot.

In another aspect of the invention, one embodiment of the inventive method is used for the diagnosis of unwanted side effects of medicaments, cancers, dysfunctions, damages or diseases of the central nerval system (CNS), aggressive symptoms or behavioural disorders, clinical, psychological and social consequences of brain injuries, psychotic disorders and disorders of the personality, dementia and/or associates syndromes, cardiovascular diseases, malfunctions or damages, diseases, malfunctions or damages of the gastrointestine, diseases, malfunctions or damages of the respiratory system, injury, inflammation, infection, immunity and/or reconvalescence, diseases, malfunctions or damages as consequences of modifications in the developmental process, diseases, malfunctions or damages of the skin, muscles, connective tissue or bones, endocrine or metabolic diseases, malfunctions or damages, headache, and sexual malfunctions or combinations thereof. Particularly preferred is a use for the diagnosis of leukemia, head and neck cancer, Hodgkin's disease, gastric cancer, prostate cancer, renal cancer, bladder cancer, breast cancer, Burkitt's lymphoma, Wilms tumor, Prader-Willi/Angelman syndrome, ICF syndrome, dermatofibroma, hypertension, pediatric neurobiological diseases, autism, ulcerative colitis, fragile X syndrome, and Huntington's disease.

In an even further aspect, the invention provides a method for the treatment of a disease or medical condition which comprises a) providing at least one diagnosis according to a method as mentioned above; and b) installing a specific treatment for said at least one diagnosed disease or medical condition. Preferably, said specific treatment is disease specific and/or personalised. In one embodiment this method is used for the treatment of unwanted side effects of medicaments, cancers, dysfunctions, damages or diseases of the central nerval system (CNS), aggressive symptoms or behavioural disorders, clinical, psychological and social consequences of brain injuries, psychotic disorders and disorders of the personality, dementia and/or associates syndromes, cardiovascular diseases, malfunctions or damages, diseases, malfunctions or damages of the gastrointestine, diseases, malfunctions or damages of the respiratory system, injury, inflammation, infection, immunity and/or reconvalescence, diseases, malfunctions or damages as consequences of modifications in the developmental process, diseases, malfunctions or damages of the skin, muscles, connective tissue or bones, endocrine or metabolic diseases, malfunctions or damages, headache, and sexual malfunctions or combinations thereof. Most preferred is a use for the treatment of leukemia, head and neck cancer, Hodgkin's disease, gastric cancer, prostate cancer, renal cancer, bladder cancer, breast cancer, Burkitt's lymphoma, Wilms tumor, Prader-Willi/Angelman syndrome, ICF syndrome, dermatofibroma, hypertension, pediatric neurobiological diseases, autism, ulcerative colitis, fragile X syndrome, and Huntington's disease.

The invention shall now be explained in more detail by the following examples with reference to the attached listing of genes and the accompanying drawings without limiting the scope of the concept of the invention.

The attached sequence listing shows a panel of genes, whose methylation statuses are altered depending on cellular states as indicated above each subgroup of the listing.

### EXAMPLES

### Example 1

### Proteomics plus subsequent methylation screening

In this example, a proteomics-derived step was used in order to analyse the expression level of a set of proteins. First, a 2-D Gelelectrophoresis according to standard protocols (see above) was perfomed for both a prostate cancer cell line and cells derived from a healthy prostate in which a staining with Sypro Ruby dye was used. Then, the resulting gels were scanned using a CCD-camera and the scanned picture were analysed using a computer-based analysis software, e.g. "Imagemaster" (Amersham-Pharmacia) or "Z3" (Compugen).

Proteins that were differently expressed in both analysis pattern were excised by a robot (Flexys robot, genomic solutions) and tryptically digested. The peptides were analysed using a MALDI-TOF mass spectrometry. The resulting fragments were analysed via peptide mapping and compared to the protein database (which one). Finally, the differently expressed proteins were listed and displayed. This listing included several differently expressed proteins.

On the basis of the listing obtained in the first step, a methylation analysis was performed according to the methods described above. The analysis revealed, that the methylation statuses of the several proteins were significantly different compared to the non-prostate cancer cell line.

Taken together, these selected genes can be further used in diagnosis and/or therapy as a minimal set of markers for prostate cancer while providing a result of maximal reliably.

### Listing of genes for panels

### Angiogenesis

ADM (Adrenomedullin); ANG (Angiogenin, ribonuclease, RNase A family, 5); ANGPT1 (Angiopoietin 1); ANGPT2 (Angiopoietin 2); ANGPT3 (Angiopoietin 3); ANGPT4 (Angiopoietin 4); ANPEP (Alanyl (membrane) aminopeptidase (aminopeptidase N, aminopeptidase M, microsomal aminopeptidase, CD 13, p150)); ARNT (Aryl hydrocarbon receptor nuclear translocator); BDK (Bradykinin); BDKRB2 (Bradykinin receptor B2); BTN (Butyrophilin); CD14 (CD14 antigen); CD19 (CD19 antigen); CD1D (CDID antigen, d polypeptide); CD2 (CD2 antigen (p50), sheep red blood cell receptor); CD20 (CD20 antigen); CD22 (CD22 antigen); CD33 (CD33 antigen (gp67); CD34 (CD34 antigen); CD37 (CD37 antigen); CD38 (CD38 antigen (p45)); CD39 (CD39 antigen); CD4 (CD4 antigen (p55)); CD47 (CD47 antigen (Rh-related antigen, integrin-associated signal transducer); CD48 (CD48 antigen (B-cell membrane protein); CD53 (CD53 antigen); CD59 (CD59 antigen p18-20 (antigen identified by monoclonal antibodies 16.3A5, EJ16, EJ30, EL32 and G344); CD63 (CD63 antigen (melanoma 1 antigen); CD68 (CD68 antigen); CD7 (CD7 antigen (p41)); CD72 (CD72 antigen); CD8A (CD8 antigen, alpha polypeptide (p32)); CD9 (CD9 antigen (p24)); CD97 (CD97 antigen); CDW52 (CDW52 antigen (CAMPATH-1 antigen)); COL4A2 (Collagen, type IV, alpha 2); COL4A4 (Collagen, type IV, alpha 4); DAF (Decay accelerating factor for complement (CD55, Cromer blood group system)); DPP4 (Dipeptidylpeptidase IV (CD26, adenosine deaminase complexing protein 2)); EGF (Epidermal growth factor); EGFR (Epidermal growth factor receptor (avian erythroblastic leukemia viral (v-erb-b) oncogene homolog)); EMAPII (Endothelial monocyte-activating polypeptide); EMAPL (Echinoderm microtubule-associated protein-like); ENG (Endoglin (Osler-Rendu-Weber syndrome 1)); F3 ( (Coagulation factor III (thromboplastin, tissue factor)); F8C (Coagulation factor VIIIc, procoagulant component (hemophilia A)); FAP (Fibroblast activation protein, alpha); FGF 1 (Fibroblast growth factor 1 (acidic)); FGF2 (Fibroblast growth factor 2 (basic)); FGFR1 (Fibroblast growth factor receptor 1 (fms-related tyrosine kinase 2, Pfeiffer syndrome)); FGFR2 (Fibroblast growth factor receptor 2 (bacteria-expressed kinase, keratinocyte growth factor receptor, craniofacial dysostosis 1, Crouzon syndrome, Pfeiffer syndrome, Jackson-Weiss syndrome)); FIGF (C-fos induced growth factor (vascular endothelial growth factor D)); FLT1 (Fms-related tyrosine kinase 1 (vascular endothelial growth factor/vascular permeability factor receptor)); FLT4 (Fms-related tyrosine kinase 4); FN1 (Fibronectin 1); GRB2 (Growth factor receptor-bound protein 2); HGF (Hepatocyte growth factor (hepapoietin A; scatter factor)); HIF1A (Hypoxia-inducible factor 1, alpha subunit (basic helix-loop-helix transcription factor)); IGF1 (Insulin-like growth factor 1 (somatomedin C)); IGF2 (Insulin-like growth factor 2 (somatomedin A)); ITGBL1 (Integrin, beta-like 1 (with EGF-like repeat domains)); JAG1 (Jagged1 (Alagille syndrome)); JAG2 (Jagged 2); KAI1 (Kangai 1 (suppression of tumorigenicity 6, prostate); CD82 antigen (R2 leukocyte antigen, antigen detected by monoclonal and antibody IA4)); KDR (Kinase insert domain receptor (a type III receptor tyrosine kinase)); LAG3 (Lymphocyte-activation gene 3); LAMA1 (Laminin, alpha 1); LAMA2 (Laminin, alpha 2 (merosin, congenital muscular dystrophy)); LAMA4 (Laminin, alpha 4); LAMA5 (Laminin, alpha 5); LY64 (Lymphocyte antigen 64 (mouse) homolog, radioprotective, 105kD); LYZ (Lysozyme (renal amyloidosis)); MDU1 (Antigen identified by monoclonal antibodies 4F2, TRA1.10, TROP4, and T43); MET (Met proto-oncogene (hepatocyte growth factor receptor)); MIC2 (Antigen identified by monoclonal antibodies 12E7, F21 and 013); MICA (MHC class I polypeptide-related sequence A); MME (Membrane metallo-endopeptidase (neutral endopeptidase, enkephalinase, CALLA, CD10)); MMP1 (Matrix metalloproteinase 1 (interstitial collagenase)); MMP10 (Matrix metalloproteinase 10 (stromelysin 2)); MMP2 (Matrix metalloproteinase 2 (gelatinase A, 72kD gelatinase, 72kD type IV collagenase)); MMP9 (Matrix metalloproteinase 9 (gelatinase B, 92kD gelatinase, 92kD type IV collagenase)); NEO1 (Neogenin (chicken) homolog 1); NOS2A (Nitric oxide synthase 2A (inducible, hepatocytes)); NOS3 (Nitric oxide synthase 3 (endothelial cell)); NRP1 (Neuropilin 1); NRP2 (Neuropilin 2); PAI1 (Plasminogen activator inhibitor, type I); PAI2 (Plasminogen activator inhibitor, type II (arginine-serpin)); PDNP1 (Phosphodiesterase I/nucleotide pyrophosphatase 1 (homologous to mouse Ly-41 antigen)); PF4 (Platelet factor 4); PF4V1 (Platelet factor 4 variant 1); PLAU (Plasminogen activator, urokinase); PLAUR (Plasminogen activator, urokinase receptor); PLG (Plasminogen); PRKCA (Protein kinase C, alpha); PRKCB1 (Protein kinase C, beta 1); PRKM1 (Protein kinase, mitogen-activated 1 (MAP kinase 1; p40, p41)); PRKM10 (Protein kinase mitogen-activated 10 (MAP kinase)); PRKM3 (Protein kinase, mitogen-activated 3 (MAP kinase 3; p44)); PTAFR (Platelet-activating factor receptor); PTCH (Patched (Drosophila) homolog); PTK2 (PTK2 protein tyrosine kinase 2); PTK2B (Protein tyrosine kinase 2 beta); RHO (Rhodopsin (retinitis pigmentosa 4, autosomal dominant)); RTN1 (Reticulon 1); SDC1 (Syndecan 1); SDC2 (Syndecan 2 (heparan sulfate proteoglycan 1, cell surface-associated, fibroglycan)); SDC4 (Syndecan 4 (amphiglycan, ryudocan)); SELPLG (Selectin P ligand); SIAT1 (Sialyltransferase 1 (beta-galactoside alpha-2,6-sialytransferase)); SLAM (Signaling lymphocytic activation molecule); SOS1 (Son of sevenless (Drosophila) homolog 1); SST (Somatostatin); SSTR1 (Somatostatin receptor 1); SSTR2 (Somatostatin receptor 2); SSTR3 (Somatostatin receptor 3); SSTR4 (Somatostatin receptor 4); SSTR5 (Somatostatin receptor 5); ST2 (Suppression of tumorigenicity 2); STAT1 (Signal transducer and activator of transcription 1, 91kD); TEK (TEK tyrosine kinase, endothelial); TFPI (Tissue factor pathway inhibitor (lipoprotein-associated coagulation inhibitor)); TGFB 1 (Transforming growth factor, beta 1); TGFB1 (Transforming growth factor, beta 1); TGFB1 (Transforming growth factor, beta 1); TGFBR1 (Transforming growth factor, beta receptor I (activin A receptor type II-like kinase, 53kD)); TGFBR1 (Transforming growth factor, beta receptor I (activin A receptor type II-like kinase, 53kD)); TGFBR1 (Transforming growth factor, beta receptor I (activin A receptor type II-like kinase, 53kD)); TGFBR2 (Transforming growth factor, beta receptor II (70-80kD)); THBS1 (Thrombospondin 1); THBS1 (Thrombospondin 1); THBS2 (Thrombospondin 2); THBS4 (Thrombospondin 4); TIE (Tyrosine kinase with immunoglobulin and epidermal growth factor homology domains); TIEG (TGFB inducible early growth response); TIEG2 (TGFB inducible early growth response 2); TIMP1 (Tissue inhibitor of metalloproteinase 1 (erythroid potentiating activity, collagenase inhibitor) ); TIMP2 (Tissue inhibitor of metalloproteinase 2); TM4SF2 (Transmembrane 4 superfamily member 2); TM4SF3 (Transmembrane 4 superfamily member 3); TNF (Tumor necrosis factor (TNF superfamily, member 2)); TNFRSF1A (Tumor necrosis factor receptor superfamily, member 1A (); TNFSF12 (Tumor necrosis factor (ligand) superfamily, member 12); TNFSF4 (Tumor necrosis factor (ligand) superfamily, member 4 (tax-transcriptionally activated glycoprotein 1, 34kD)); TNFSF5 (Tumor necrosis factor (ligand) superfamily, member 5); TNFSF7 (Tumor necrosis factor (ligand) superfamily, member 7); VCAM1 (Vascular cell adhesion molecule 1); VEGF (Vascular endothelial growth factor); VEGFB (Vascular endothelial growth factor B); VEGFC (Vascular endothelial growth factor C); VTN (Vitronectin (serum spreading factor, somatomedin B, complement S-protein)); and VWF (Von Willebrand factor).

### Apoptosis

APAF1 (Apoptotic protease activating factor); API1 (Apoptosis inhibitor 1); API2 (Apoptosis inhibitor 2); API3 (Apoptosis inhibitor 3); API4 (Apoptosis inhibitor 4 (survivin)); BAD (BCL2-antagonist of cell death); BAD (BCL2-antagonist of cell death); BAG4 (BCL2-associated athanogene 4); BAK1 (BCL2-antagonist/killer 1); BAX (BCL2-associated X protein); BCL10 (B-cell CLL/lymphoma 10); BCL2 (B-cell CLL/lymphoma 2); BCL2A1 (BCL2-related protein A1); BCL2L1 (BCL2-like 1); BCL2L2 (BCL2-like 2); BNIP1 (BCL2/adenovirus E1B 19kD-interacting protein 1); BNIP2 (BCL2/adenovirus E1B 19kD-interacting protein 2); BNIP3 (BCL2/adenovirus E1B 19kD-interacting protein 3); CASP1 (Caspase 1, apoptosis-related cysteine protease (interleukin 1, beta, convertase) ); CASP10 (Caspase 10, apoptosis-related cysteine protease); CASP2 (Caspase 2, apoptosis-related cysteine protease (neural precursor cell expressed, developmentally down-regulated 2)); CASP3 (Caspase 3, apoptosis-related cysteine protease); CASP4 (Caspase 4, apoptosis-related cysteine protease); CASP5 (Caspase 5, apoptosis-related cysteine protease); CASP6 (Caspase 6, apoptosis-related cysteine protease); CASP7 (Caspase 7, apoptosis-related cysteine protease); CASP8 (Caspase 8, apoptosis-related cysteine protease); CASP9 (Caspase 9, apoptosis-related cysteine protease); DAP (Death-associated protein); DAP3 (Death associated protein 3); DAPK3 (Death-associated protein kinase 3); DAXX (Death-associated protein 6); DFFA (DNA fragmentation factor, 45 kD, alpha subunit); MCL1 (Myeloid cell leukemia sequence 1 (BCL2-related)); MDM4 (Mouse double minute 4, human homolog of; p53-binding protein); MYD88 (Myeloid differentiation primary response gene (88)); NAIP (Neuronal apoptosis inhibitory protein); PAWR (PRKC, apoptosis, WT1, regulator); PDCD1 (Programmed cell death 1); PDCD2 (Programmed cell death 2); PSEN2 (Presenilin 2 (Alzheimer disease 4)); REQ (Requiem, apoptosis response zinc finger gene); SFRP5 (Secreted frizzled-related protein 5); STK17A (Serine/threonine kinase 17a (apoptosis-inducing)); STK17B (Serine/threonine kinase 17b (apoptosis-inducing)); TNFAIP1 (Tumor necrosis factor, alpha-induced protein 1 (endothelial)); TNFRSF6 (Tumor necrosis factor receptor superfamily, member); and TP53BP2 (Tumor protein p53-binding protein).

### Behavior

ADRA1A (Adrenergic, alpha-1A-, receptor); ADRA1C (Adrenergic, alpha-1C-, receptor); ADRA2A (Adrenergic, alpha-2A-, receptor); ADRA2B (Adrenergic, alpha-2B-, receptor); ADRA2C (Adrenergic, alpha-2C-, receptor); ADRB1 (Adrenergic, beta-1-, receptor); ADRB2 (Adrenergic, beta-2-, receptor, surface); ADRB3 (Adrenergic, beta-3-, receptor); ADRBK1 (Adrenergic, beta, receptor kinase 1); ADRBK2 (Adrenergic, beta, receptor kinase 2); COMT (Catechol-O-methyltransferase); DBH (Dopamine beta-hydroxylase (dopamine beta-monooxygenase)); DDC (Dopa decarboxylase (aromatic L-amino acid decarboxylase)); DRD 1 (Dopamine receptor D1); DRD2 (Dopamine receptor D2); DRD3 (Dopamine receptor D3); DRD4 (Dopamine receptor D4); DRD5 (Dopamine receptor D5); MAOA (Monoamine oxidase A); MAOB (Monoamine oxidase B); OPRM1 (Opioid receptor, mu 1); PNMT (Phenylethanolamine N-methyltransferase); and TH (Tyrosine hydroxylase)

### Cell cycle

ABL2 (V-abl Abelson murine leukemia viral oncogene homolog 2 (arg, Abelson-related gene)); ACP1 (Acid phosphatase 1, soluble); ACP2 (Acid phosphatase 2, lysosomal); ACP5 (Acid phosphatase 5, tartrate resistant); ACPP (Acid phosphatase, prostate); ACVR1B (Activin A receptor, type IB); ADK (Adenosine kinase); ADRBK1 (Adrenergic, beta, receptor kinase 1); AK1 (Adenylate kinase 1); AK2 (Adenylate kinase 2); AK3 (Adenylate kinase 3); AKT1 (V-akt murine thymoma viral oncogene homolog 1); AKT2 (V-akt murine thymoma viral oncogene homolog 2); ALPI (Alkaline phosphatase, intestinal); ALPL (Alkaline phosphatase, liver/bone/kidney); ALPP (Alkaline phosphatase, placental (Regan isozyme)); ARAF1 (V-raf murine sarcoma 3611 viral oncogene homolog 1); BLK (B lymphoid tyrosine kinase); BMPR2 (Bone morphogenetic protein receptor, type II (serine/threonine kinase)); BMX (BMX non-receptor tyrosine kinase); BRAF (V-raf murine sarcoma viral oncogene homolog B1); BTK (Bruton agammaglobulinemia tyrosine kinase); CAK (Cell adhesion kinase); CALM1 (Calmodulin 1 (phosphorylase kinase, delta)); CAMK4 (Calcium/calmodulin-dependent protein kinase IV); CBFA2T1 (Core-binding factor, runt domain, alpha subunit 2; translocated to, 1; cyclin D-related); CBFA2T2 (Core-binding factor, runt domain, alpha subunit 2; translocated to, 2); CBFA2T3 (Core-binding factor, runt domain, alpha subunit 2; translocated to, 3); CCNA2 (Cyclin A2); CCNB1 (Cyclin B1); CCND1 (Cyclin D1 (PRAD1: parathyroid adenomatosis 1)); CCND2 (Cyclin D2); CCND3 (Cyclin D3); CCNE1 (Cyclin E1); CCNE2 (Cyclin E2); CCNF (Cyclin F); CCNH (Cyclin H); CCNK (Cyclin K); CD48 (CD48 antigen (B-cell membrane protein)); CD69 (CD69 antigen (p60, early T-cell activation antigen)); CDC10 (Cell division cycle 10 (homologous to CDC 10 of S. cerevisiae)); CDC18L (CDC18 (cell division cycle 18, S.pombe, homolog)-like); CDC2 (Cell division cycle 2, G1 to S and G2 to M); CDC25A (Cell division cycle 25A); CDC25A (Cell division cycle 25A); CDC25C (Cell division cycle 25C); CDC25C (Cell division cycle 25C); CDC27 (Cell division cycle 27); CDC2L1 (Cell division cycle 2-like 1 (PITSLRE proteins)); CDC42 (Cell division cycle 42 (GTP-binding protein, 25kD)); CDC7L1 (CDC7 (cell division cycle 7, S. cerevisiae, homolog)-like 1); CDK2 (Cyclin-dependent kinase 2); CDK2 (Cyclin-dependent kinase 2); CDK5 (Cyclin-dependent kinase 5); CDK6 (Cyclin-dependent kinase 6); CDK6 (Cyclin-dependent kinase 6); CDK7 (Cyclin-dependent kinase 7 (homolog of Xenopus MO15 cdk-activating kinase)); CDK7 (Cyclin-dependent kinase 7 (homolog of Xenopus MO15 cdk-activating kinase)); CDK8 (Cyclin-dependent kinase 8); CDK8 (Cyclin-dependent kinase 8); CDKN1B (Cyclin-dependent kinase inhibitor 1B (p27, Kip1)); CDKN1C (Cyclin-dependent kinase inhibitor 1C (p57, Kip2)); CDKN2B (Cyclin-dependent kinase inhibitor 2B (p15, inhibits CDK4)); CHC1 (Chromosome condensation 1); CHEK1 (CHK1 (checkpoint, S.pombe) homolog); CHES 1 (Checkpoint suppressor 1); CHUK (Conserved helix-loop-helix ubiquitous kinase); CKS1 (CDC28 protein kinase 1); CKS2 (CDC28 protein kinase 2); CLK2 (CDC-like kinase 2); CLK3 (CDC-like kinase 3); CNK (Cytokine-inducible kinase); COT (Cot (cancer Osaka thyroid) oncogene); CSNK1A1 (Casein kinase 1, alpha 1); CSNK1D (Casein kinase 1, delta); CSNK1E (Casein kinase 1, epsilon); CSNK2A2 (Casein kinase 2, alpha prime polypeptide); CSNK2B (Casein kinase 2, beta polypeptide); DENR (Density-regulated protein); DGKA (Diacylglycerol kinase, alpha (80kD)); DGKG (Diacylglycerol kinase, gamma (90kD)); DGKQ (Diacylglycerol kinase, theta (110kD)); DMPK (Dystrophia myotonica-protein kinase); DUSP3 (Dual specificity phosphatase 3 (vaccinia virus phosphatase VH1-related)); DUSP4 (Dual specificity phosphatase 4); DYRK1 (Dual-specificity tyrosine-(Y)-phosphorylation regulated kinase 1); E2F2 (E2F transcription factor 2); E2F5 (E2F transcription factor 5, p130-binding); EFNA1 (Ephrin-A1); EFNA5 (Ephrin-A5); EFNB1 (Ephrin-B1); EFNB2 (Ephrin-B2); EGR1 (Early growth response 1); EPHA2 (EphA2); EPHA4 (EphA4); EPHA5 (EphA5); EPHA7 (EphA7); EPHB3 (EphB3); EPHB4 (EphB4); EPHB6 (EphB6); ERBB3 (V-erb-b2 avian erythroblastic leukemia viral oncogene homolog 3); FAP (Fibroblast activation protein, alpha); FBL (Fibrillarin); FES (Feline sarcoma (Snyder-Theilen) viral (v-fes)/Fujinami avian sarcoma (PRCII) viral (v-fps) oncogene homolog); FGFR1 (Fibroblast growth factor receptor 1 (fms-related tyrosine kinase 2, Pfeiffer syndrome)); FGFR2 (Fibroblast growth factor receptor 2 (bacteria-expressed kinase, keratinocyte growth factor receptor, craniofacial dysostosis 1, Crouzon syndrome, Pfeiffer syndrome, Jackson-Weiss syndrome)); FGR (Gardner-Rasheed feline sarcoma viral (v-fgr) oncogene homolog); FLT1 (Fms-related tyrosine kinase 1 (vascular endothelial growth factor/vascular permeability factor receptor)); FLT3LG (Fms-related tyrosine kinase 3 ligand); FLT4 (Fms-related tyrosine kinase 4); FRK (Fyn-related kinase); GAS2 (Growth arrest-specific 2); GLA (Galactosidase, alpha); GPRK6 (G protein-coupled receptor kinase 6); GSPT1 (G1 to S phase transition 1); HMR (Hormone receptor (growth factor-inducible nuclear protein N10)); HUS 1 (HUS1 (S. pombe) checkpoint homolog); IFI56 (Interferon-induced protein 56); IGFBP1 (Insulin-like growth factor binding protein 1); ILK (Integrin-linked kinase); INP10 (Interferon (gamma)-induced cell line; protein 10 from); INPP5D (Inositol polyphosphate-5-phosphatase, 145kD); ISG20 (Interferon stimulated gene (20kD)); JAK1 (Janus kinase 1 (a protein tyrosine kinase)); JAK3 (Janus kinase 3 (a protein tyrosine kinase, leukocyte)); KDR (Kinase insert domain receptor (a type III receptor tyrosine kinase)); LCAT (Lecithin-cholesterol acyltransferase); LIMK1 (LIM domain kinase 1); LIMK2 (LIM domain kinase 2); LRP1 (Low density lipoprotein-related protein 1 (alpha-2-macroglobulin receptor)); LTK (Leukocyte tyrosine kinase); LY6E (Lymphocyte antigen 6 complex, locus E); MAD 1L 1 (MAD1 (mitotic arrest deficient, yeast, homolog)-like 1); MAD2L1 (MAD2 (mitotic arrest deficient, yeast, homolog)-like 1); MATK (Megakaryocyte-associated tyrosine kinase); MCL1 (Myeloid cell leukemia sequence 1 (BCL2-related)); MCM4 (Minichromosome maintenance deficient (S. cerevisiae) 4); MEKK3 (MAP/ERK kinase kinase 3); MEKK5 (MAP/ERK kinase kinase 5); MKI67 (Antigen identified by monoclonal antibody Ki-67); MST1R (Macrophage stimulating 1 receptor (c-met-related tyrosine kinase)); NCK1 (NCK adaptor protein 1); NEK3 (NIMA (never in mitosis gene a)-related kinase); NME1 (Non-metastatic cells 1, protein (NM23A) expressed in); NME2 (Non-metastatic cells 2, protein (NM23B) expressed in); NOS2A (Nitric oxide synthase 2A (inducible, hepatocytes)); NPM1 (Nucleophosmin (nucleolar phosphoprotein B23, numatrin)); NTRK3 (Neurotrophic tyrosine kinase, receptor, type 3); OAS1 (2',5'-oligoadenylate synthetase 1); PA2G4 (Proliferation-associated 2G4, 38kD); PCNA (Proliferating cell nuclear antigen); PCTK3 (PCTAIRE protein kinase 3); PDGFRB (Platelet-derived growth factor receptor, beta polypeptide); PDK2 (Pyruvate dehydrogenase kinase, isoenzyme 2); PDK4 (Pyruvate dehydrogenase kinase, isoenzyme 4); PDPK1 (3-phosphoinositide dependent protein kinase-1); PHKA2 (Phosphorylase kinase, alpha 2 (liver), glycogen storage disease IX); PHKG2 (Phosphorylase kinase, gamma 2 (testis)); PIK3CA (Phosphoinositide-3-kinase, catalytic, alpha polypeptide); PIK3CG (Phosphoinositide-3-kinase, catalytic, gamma polypeptide); PIM1 (Pim-1 oncogene); POLB (Polymerase (DNA directed), beta); POLD1 (Polymerase (DNA directed), delta 1, catalytic subunit (125kD)); PPP1CA (Protein phosphatase 1, catalytic subunit, alpha isoform); PPP1CB (Protein phosphatase 1, catalytic subunit, beta isoform); PPP1CC (Protein phosphatase 1, catalytic subunit, gamma isoform); PPP1R3 (Protein phosphatase 1, regulatory (inhibitor) subunit 3 (glycogen and sarcoplasmic reticulum binding subunit, skeletal muscle)); PPP2CA (Protein phosphatase 2 (formerly 2A), catalytic subunit, alpha isoform); PPP2R1B (Protein phosphatase 2 (formerly 2A), regulatory subunit A (PR 65), beta isoform); PPP2R2A (Protein phosphatase 2 (formerly 2A), regulatory subunit B (PR 52), alpha isoform); PPP2R3 (Protein phosphatase 2 (formerly 2A), regulatory subunit B" (PR 72), alpha isoform and (PR 130), beta isoform); PPP2R4 (Protein phosphatase 2A, regulatory subunit B' (PR 53)); PPP2R5A (Protein phosphatase 2, regulatory subunit B (B56), alpha isoform); PPP2R5B (Protein phosphatase 2, regulatory subunit B (B56), beta isoform); PPP2R5D (Protein phosphatase 2, regulatory subunit B (B56), delta isoform); PPP2R5E (Protein phosphatase 2, regulatory subunit B (B56), epsilon isoform); PPP3CA (Protein phosphatase 3 (formerly 2B), catalytic subunit, alpha isoform (calcineurin A alpha)); PPP3CB (Protein phosphatase 3 (formerly 2B), catalytic subunit, beta isoform (calcineurin A beta)); PPP4C (Protein phosphatase 4 (formerly X), catalytic subunit); PRKAA2 (Protein kinase, AMP-activated, alpha 2 catalytic subunit); PRKACA (Protein kinase, cAMP-dependent, catalytic, alpha); PRKACB (Protein kinase, cAMP-dependent, catalytic, beta); PRKACG (Protein kinase, cAMP-dependent, catalytic, gamma); PRKAG1 (Protein kinase, AMP-activated, gamma 1 non-catalytic subunit); PRKAR1A (Protein kinase, cAMP-dependent, regulatory, type I, alpha (tissue specific extinguisher 1)); PRKAR1B (Protein kinase, cAMP-dependent, regulatory, type I, beta); PRKAR2B (Protein kinase, cAMP-dependent, regulatory, type II, beta); PRKCA (Protein kinase C, alpha); PRKCB1 (Protein kinase C, beta 1); PRKCG (Protein kinase C, gamma); PRKCI (Protein kinase C, iota); PRKCL1 (Protein kinase C-like 1); PRKCL2 (Protein kinase C-like 2); PRKCM (Protein kinase C, mu); PRKCQ (Protein kinase C, theta); PRKCZ (Protein kinase C, zeta); PRKG1 (Protein kinase, cGMP-dependent, type I); PRKG2 (Protein kinase, cGMP-dependent, type II); PRKM1 (Protein kinase, mitogen-activated 1 (MAP kinase 1; p40, p41)); PRKM10 (Protein kinase mitogen-activated 10 (MAP kinase)); PRKM11 (Protein kinase mitogen- activated 11); PRKM13 (Protein kinase mitogen-activated 13); PRKM3 (Protein kinase, mitogen-activated 3 (MAP kinase 3; p44)); PRKM6 (Protein kinase, mitogen-activated 6 (extracellular signal-regulated kinase, p97)); PRKM7 (Protein kinase mitogen-activated 7 (MAP kinase)); PRKM8 (Protein kinase mitogen-activated 8 (MAP kinase)); PRKM9 (Protein kinase mitogen-activated 9 (MAP kinase)); PRKMK2 (Protein kinase, mitogen-activated, kinase 2, p45 (MAP kinase kinase 2)); PRKMK3 (Protein kinase, mitogen-activated, kinase 3 (MAP kinase kinase 3)); PRKMK5 (Protein kinase, mitogen-activated, kinase 5 (MAP kinase kinase 5)); PRKMK7 (Protein kinase, mitogen-activated, kinase 7 (MAP kinase kinase 7)); PRKR (Protein kinase, interferon-inducible double stranded RNA dependent); PTEN (Phosphatase and tensin homolog (mutated in multiple advanced cancers 1)); PTK7 (PTK7 protein tyrosine kinase 7); PTK9 (Protein tyrosine kinase 9); PTPN1 (Protein tyrosine phosphatase, non-receptor type 1); PTPN12 (Protein tyrosine phosphatase, non-receptor type 12); PTPN 13 (Protein tyrosine phosphatase, non-receptor type 13 (APO-1/CD95 (Fas)-associated phosphatase)); PTPN2 (Protein tyrosine phosphatase, non-receptor type 2); PTPN3 (Protein tyrosine phosphatase, non-receptor type 3); PTPN4 (Protein tyrosine phosphatase, non-receptor type 4 (megakaryocyte)); PTPN6 (Protein tyrosine phosphatase, non-receptor type 6); PTPN7 (Protein tyrosine phosphatase, non-receptor type 7); PTPN9 (Protein tyrosine phosphatase, non-receptor type 9); PTPRA (Protein tyrosine phosphatase, receptor type, alpha polypeptide); PTPRB (Protein tyrosine phosphatase, receptor type, beta polypeptide); PTPRC (Protein tyrosine phosphatase, receptor type, c polypeptide); PTPRD (Protein tyrosine phosphatase, receptor type, D); PTPRF (Protein tyrosine phosphatase, receptor type, F); PTPRG (Protein tyrosine phosphatase, receptor type, gamma polypeptide); PTPRH (Protein tyrosine phosphatase, receptor type, H); PTPRK (Protein tyrosine phosphatase, receptor type, K); PTPRM (Protein tyrosine phosphatase, receptor type, M); PTPRN (Protein tyrosine phosphatase, receptor type, N); PTPRN2 (Protein tyrosine phosphatase, receptor type, N polypeptide 2); PTX3 (Pentaxin-related gene, rapidly induced by IL-1 beta); RAB8IP (Rab8 interacting protein (GC kinase)); RAB8IP (Rab8 interacting protein (GC kinase)); RAD9 (RAD9 (S. pombe) homolog); RAD9 (RAD9 (S. pombe) homolog); RBL1 (Retinoblastoma-like 1 (p107)); RET (Ret proto-oncogene (multiple endocrine neoplasia MEN2A, MEN2B and medullary thyroid carcinoma 1, Hirschsprung disease)); RHOK (Rhodopsin kinase); ROCK1 (Rho-associated, coiled-coil containing protein kinase 1); RPS6KA2 (Ribosomal protein S6 kinase, 90kD, polypeptide 2); RPS6KB1 (Ribosomal protein S6 kinase, 70kD, polypeptide 1); RYK (RYK receptor-like tyrosine kinase); SAPK3 (Stress-activated protein kinase 3); SERK1 (SAPK/Erk kinase 1); SRC (V-src avian sarcoma (Schmidt-Ruppin A-2) viral oncogene homolog); SRF (Serum response factor (c-fos serum response element-binding transcription factor)); SRPK2 (SFRS protein kinase 2); STK11 (Serine/threonine kinase 11 (Peutz-Jeghers syndrome)); STK2 (Serine/threonine kinase 2); STK3 (Serine/threonine kinase 3 (Ste20, yeast homolog)); STK4 (Serine/threonine kinase 4); SYK (Spleen tyrosine kinase); TCEB1L (Transcription elongation factor B (SIII), polypeptide 1-like); TESK1 (Testis-specific kinase 1); TFDP2 (Transcription factor Dp-2 (E2F dimerization partner 2)); TGFBI (Transforming growth factor, beta-induced, 68kD); TIEG (TGFB inducible early growth response); TK2 (Thymidine kinase 2, mitochondrial); TNFAIP1 (Tumor necrosis factor, alpha-induced protein 1 (endothelial)); TNFSF5 (Tumor necrosis factor (ligand) superfamily, member 5); TS546 (Temperature sensitivity complementation, cell cycle specific,); TTK (TTK protein kinase); TXK (TXK tyrosine kinase); TYK2 (Tyrosine kinase 2); TYRO3 (TYRO3 protein tyrosine kinase); VRK2 (Vaccinia related kinase 2); ZAP70 (Zeta-chain (TCR) associated protein kinase (70 kD)); and ZPK (Zipper (leucine) protein kinase).

### Cell signaling

ABL1 (V-abl Abelson murine leukemia viral oncogene homolog 1); ABL2 (V-abl Abelson murine leukemia viral oncogene homolog 2 (arg, Abelson-related gene)); ABR (Active BCR-related gene); ACP1 (Acid phosphatase 1, soluble); ACP2 (Acid phosphatase 2, lysosomal); ACP5 (Acid phosphatase 5, tartrate resistant); ACPP (Acid phosphatase, prostate); ACTG1 (Actin, gamma 1); ACVR1B (Activin A receptor, type IB); ADD3 (Adducin 3 (gamma)); ADK (Adenosine kinase); ADRBK1 (Adrenergic, beta, receptor kinase 1); AIF1 (Allograft inflammatory factor 1); AK1 (Adenylate kinase 1); AK2 (Adenylate kinase 2); AK3 (Adenylate kinase 3); AKT1 (V-akt murine thymoma viral oncogene homolog 1); AKT2 (V-akt murine thymoma viral oncogene homolog 2); ALCAM (Activated leucocyte cell adhesion molecule); ALOX12 (Arachidonate 12-lipoxygenase); ALOX5 (Arachidonate 5-lipoxygenase); ALPI (Alkaline phosphatase, intestinal); ALPL (Alkaline phosphatase, liver/bone/kidney); ALPP (Alkaline phosphatase, placental (Regan isozyme)); ANK1 (Ankyrin 1, erythrocytic); ANT2 (Adenine nucleotide translocator 2 (fibroblast)); ARAF1 (V-raf murine sarcoma 3611 viral oncogene homolog 1); ARHA (Ras homolog gene family, member A); ARHC (Ras homolog gene family, member C); ARHGDIB (Rho GDP dissociation inhibitor (GDI) beta); BLK (B lymphoid tyrosine kinase); BMPR2 (Bone morphogenetic protein receptor, type II (serine/threonine kinase)); BMX (BMX non-receptor tyrosine kinase); BRAF (V-raf murine sarcoma viral oncogene homolog B1); BTK (Bruton agammaglobulinemia tyrosine kinase); CAK (Cell adhesion kinase); CALM1 (Calmodulin 1 (phosphorylase kinase, delta)); CAMK4 (Calcium/calmodulin-dependent protein kinase IV); CBLB (Cas-Br-M (murine) ectropic retroviral transforming sequence b); CD44 (CD44 antigen (homing function and Indian blood group system)); CD47 (CD47 antigen (Rh-related antigen, integrin-associated signal transducer)); CD48 (CD48 antigen (B-cell membrane protein)); CD58 (CD58 antigen, (lymphocyte function-associated antigen 3)); CD69 (CD69 antigen (p60, early T-cell activation antigen)); CDC25A (Cell division cycle 25A); CDC25C (Cell division cycle 25C); CDC2L1 (Cell division cycle 2-like 1 (PITSLRE proteins)); CDC42 (Cell division cycle 42 (GTP-binding protein, 25kD)); CDC42 (Cell division cycle 42 (GTP-binding protein, 25kD)); CDC7L1 (CDC7 (cell division cycle 7, S. cerevisiae, homolog)-like 1); CDH13 (Cadherin 13, H-cadherin (heart)); CDH17 (Cadherin 17, LI cadherin (liver-intestine)); CDH2 (Cadherin 2, N-cadherin (neuronal)); CDH4 (Cadherin 4, R-cadherin (retinal)); CDH5 (Cadherin 5, VE-cadherin (vascular epithelium)); CDK2 (Cyclin-dependent kinase 2); CDK5 (Cyclin-dependent kinase 5); CDK6 (Cyclin-dependent kinase 6); CDK7 (Cyclin-dependent kinase 7 (homolog of Xenopus MO15 cdk-activating kinase)); CDK8 (Cyclin-dependent kinase 8); CDKN2B (Cyclin-dependent kinase inhibitor 2B (p15, inhibits CDK4)); CHEK1 (CHUK1 (checkpoint, S.pombe) homolog); CHUK (Conserved helix-loop-helix ubiquitous kinase); CKS1 (CDC28 protein kinase 1); CKS2 (CDC28 protein kinase 2); CLGN (Calmegin); CLK2 (CDC-like kinase 2); CLK3 (CDC-like kinase 3); CLTB (Clathrin, light polypeptide (Lcb)); CNK (Cytokine-inducible kinase); COT (Cot (cancer Osaka thyroid) oncogene); CSNK1A1 (Casein kinase 1, alpha 1); CSNK1D (Casein kinase 1, delta); CSNK1E (Casein kinase 1, epsilon); CSNK2A2 (Casein kinase 2, alpha prime polypeptide); CSNK2B (Casein kinase 2, beta polypeptide); CTNNA1 (Catenin (cadherin-associated protein), alpha 1 (102kD)); CTNNB1 (Catenin (cadherin-associated protein), beta 1 (88kD)); CTNND2 (Catenin (cadherin-associated protein), delta 2 (neural plakophilin-related arm-repeat protein)); DAPK1 (Death-associated protein kinase 1); DGKA (Diacylglycerol kinase, alpha (80kD)); DGKG (Diacylglycerol kinase, gamma (90kD)); DGKQ (Diacylglycerol kinase, theta (110kD)); DMPK (Dystrophia myotonica-protein kinase); DUSP3 (Dual specificity phosphatase 3 (vaccinia virus phosphatase VH1-related)); DUSP4 (Dual specificity phosphatase 4); DVL3 (Dishevelled 3 (homologous to Drosophila dsh)); DYRK1 (Dual-specificity tyrosine-(Y)-phosphorylation regulated kinase 1); EFNA1 (Ephrin-A1); EFNA5 (Ephrin-A5); EFNB1 (Ephrin-B1); EFNB2 (Ephrin-B2); EGR1 (Early growth response 1); EGR3 (Early growth response 3); EGR4 (Early growth response 4); EPHA2 (EphA2); EPHA4 (EphA4); EPHA5 (EphA5); EPHA7 (EphA7); EPHB3 (EphB3); EPHB4 (EphB4); EPHB6 (EphB6); ERBB3 (V-erb-b2 avian erythroblastic leukemia viral oncogene homolog 3); FAP (Fibroblast activation protein, alpha); FAT (FAT tumor suppressor (Drosophila) homolog); FBL (Fibrillarin); FES (Feline sarcoma (Snyder-Theilen) viral (v-fes)/Fujinami avian sarcoma (PRCII) viral (v-fps) oncogene homolog); FGFR1 (Fibroblast growth factor receptor 1 (fms-related tyrosine kinase 2, Pfeiffer syndrome)); FGFR2 (Fibroblast growth factor receptor 2 (bacteria-expressed kinase, keratinocyte growth factor receptor, craniofacial dysostosis 1, Crouzon syndrome, Pfeiffer syndrome, Jackson-Weiss syndrome)); FGR (Gardner-Rasheed feline sarcoma viral (v-fgr) oncogene homolog); FLNA (Filamin A, alpha (actin-binding protein-280)); FLT1 (Fms-related tyrosine kinase 1 (vascular endothelial growth factor/vascular permeability factor receptor)); FLT3LG (Fms-related tyrosine kinase 3 ligand); FLT4 (Fms-related tyrosine kinase 4); FN1 (Fibronectin 1); FRK (Fyn-related kinase); FYB (FYN-binding protein (FYB-120/130)); G1P3 (Interferon, alpha-inducible protein (clone IFI-6-16)); GBP1 (Guanylate binding protein 1, interferon-inducible, 67kD); GBP2 (Guanylate binding protein 2, interferon-inducible); GJB1 (Gap junction protein, beta 1, 32kD (connexin 32, Charcot-Marie-Tooth neuropathy, X-linked)); GLA (Galactosidase, alpha); GNAI1 (Guanine nucleotide binding protein (G protein), alpha inhibiting activity polypeptide 1); GNG 10 (Guanine nucleotide binding protein 10); GPRK6 (G protein-coupled receptor kinase 6); GRB2 (Growth factor receptor-bound protein 2); HMMR (Hyaluronan-mediated motility receptor (RHAMM)); HMR (Hormone receptor (growth factor-inducible nuclear protein N10)); ICAM1 (Intercellular adhesion molecule 1 (CD54), human rhinovirus receptor); ICAM2 (Intercellular adhesion molecule 2); ICAM3 (Intercellular adhesion molecule 3); IFI16 (Interferon, gamma-inducible protein 16); IFI56 (Interferon-induced protein 56); IFIT4 (Interferon-induced protein with tetratricopeptide repeats 4); IGFBP1 (Insulin-like growth factor binding protein 1); ILK (Integrin-linked kinase); INP10 (Interferon (gamma)-induced cell line; protein 10 from); INPP5D (Inositol polyphosphate-5-phosphatase, 145kD); ISG20 (Interferon stimulated gene (20kD)); ITGA2 (Integrin, alpha 2 (CD49B, alpha 2 subunit of VLA-2 receptor)); ITGA3 (Integrin, alpha 3 (antigen CD49C, alpha 3 subunit of VLA-3 receptor)); ITGA4 (Integrin, alpha 4 (antigen CD49D, alpha 4 subunit of VLA-4 receptor)); ITGA5 (Integrin, alpha 5 (fibronectin receptor, alpha polypeptide)); ITGA6 (Integrin, alpha 6); ITGA7 (Integrin, alpha 7); ITGA9 (Integrin, alpha 9); ITGAE (Integrin, alpha E (antigen CD103, human mucosal lymphocyte antigen 1; alpha polypeptide)); ITGAL (Integrin, alpha L (antigen CD11A (p180), lymphocyte function-associated antigen 1; alpha polypeptide)); ITGAM (Integrin, alpha M (complement component receptor 3, alpha; also known as CD11b (p170), macrophage antigen alpha polypeptide)); ITGAV (Integrin, alpha V (vitronectin receptor, alpha polypeptide, antigen CD51)); ITGAX (Integrin, alpha X (antigen CD11C (p150), alpha polypeptide)); ITGB1 (Integrin, beta 1 (fibronectin receptor, beta polypeptide, antigen CD29 includes MDF2, MSK12)); ITGB2 (Integrin, beta 2 (antigen CD18 (p95), lymphocyte function-associated antigen 1; macrophage antigen I (mac-1) beta subunit)); ITGB3 (Integrin, beta 3 (platelet glycoprotein IIIa, antigen CD61)); ITGB4 (Integrin, beta 4); ITGB5 (Integrin, beta 5); ITGB7 (Integrin, beta 7); ITGB8 (Integrin, beta 8); ITPKB (Inositol 1,4,5-trisphosphate 3-kinase B); JAK1 (Janus kinase 1 (a protein tyrosine kinase)); JAK3 (Janus kinase 3 (a protein tyrosine kinase, leukocyte)); KCNN3 (Potassium intermediate/small conductance calcium-activated channel, subfamily N, member 3); KDR (Kinase insert domain receptor (a type III receptor tyrosine kinase)); KPNB1 (Karyopherin (importin) beta 1); LCAT (Lecithin-cholesterol acyltransferase); LIMK1 (LIM domain kinase 1); LIMK2 (LIM domain kinase 2); LRP1 (Low density lipoprotein-related protein 1 (alpha-2-macroglobulin receptor)); LTK (Leukocyte tyrosine kinase); LY6E (Lymphocyte antigen 6 complex, locus E); MADH1 (MAD (mothers against decapentaplegic, Drosophila) homolog 1); MADH2 (MAD (mothers against decapentaplegic, Drosophila) homolog 2); MADH3 (MAD (mothers against decapentaplegic, Drosophila) homolog 3); MAP1B (Microtubule-associated protein 1B); MATK (Megakaryocyte-associated tyrosine kinase); MCL 1 (Myeloid cell leukemia sequence 1 (BCL2-related)); MEKK3 (MAP/ERK kinase kinase 3); MEKK5 (MAP/ERK kinase kinase 5); MST1R (Macrophage stimulating 1 receptor (c-met-related tyrosine kinase)); MX1 (Myxovirus (influenza) resistance 1, homolog of murine (interferon-inducible protein p78)); MX2 (Myxovirus (influenza) resistance 2, homolog of murine); MYL2 (Myosin, light polypeptide 2, regulatory, cardiac, slow); MYL3 (Myosin, light polypeptide 3, alkali; ventricular, skeletal, slow); NCAM1 (Neural cell adhesion molecule 1); NCK1 (NCK adaptor protein 1); NEK3 (NIMA (never in mitosis gene a)-related kinase); NME1 (Non-metastatic cells 1, protein (NM23A) expressed in); NME2 (Non-metastatic cells 2, protein (NM23B) expressed in); NOS2A (Nitric oxide synthase 2A (inducible, hepatocytes)); NPM1 (Nucleophosmin (nucleolar phosphoprotein B23, numatrin)); NTRK3 (Neurotrophic tyrosine kinase, receptor, type 3); OAS1 (2',5'-oligoadenylate synthetase 1); OCLN (Occludin); PCDH1 (Protocadherin 1 (cadherin-like 1)); PCTK3 (PCTAIRE protein kinase 3); PDE4B (Phosphodiesterase 4B, cAMP-specific (dunce (Drosophila)-homolog phosphodiesterase E4)); PDGFRB (Platelet-derived growth factor receptor, beta polypeptide); PDK2 (Pyruvate dehydrogenase kinase, isoenzyme 2); PDK4 (Pyruvate dehydrogenase kinase, isoenzyme 4); PDPK1 (3-phosphoinositide dependent protein kinase-1); PECAM1 (Platelet/endothelial cell adhesion molecule (CD31 antigen)); PGY3 (P glycoprotein 3/multiple drug resistance 3); PHKA2 (Phosphorylase kinase, alpha 2 (liver), glycogen storage disease IX); PHKG2 (Phosphorylase kinase, gamma 2 (testis)); PIK3C3 (Phosphoinositide-3-kinase, class 3); PIK3CA (Phosphoinositide-3-kinase, catalytic, alpha polypeptide); PIK3CA (Phosphoinositide-3-kinase, catalytic, alpha polypeptide); PIK3CG (Phosphoinositide-3-kinase, catalytic, gamma polypeptide); PIM1 (Pim-1 oncogene); PKD1 (Polycystic kidney disease 1 (autosomal dominant)); PLA2G2A (Phospholipase A2, group IIA (platelets, synovial fluid)); PLCB4 (Phospholipase C, beta 4); PLCE (Phospholipase C, epsilon); PLCG2 (Phospholipase C, gamma 2 (phosphatidylinositol-specific)); PPP1CA (Protein phosphatase 1, catalytic subunit, alpha isoform); PPP1CB (Protein phosphatase 1, catalytic subunit, beta isoform); PPP1CC (Protein phosphatase 1, catalytic subunit, gamma isoform); PPP1R3 (Protein phosphatase 1, regulatory (inhibitor) subunit 3 (glycogen and sarcoplasmic reticulum binding subunit, skeletal muscle)); PPP2CA (Protein phosphatase 2 (formerly 2A), catalytic subunit, alpha isoform); PPP2R1B (Protein phosphatase 2 (formerly 2A), regulatory subunit A (PR 65), beta isoform); PPP2R2A (Protein phosphatase 2 (formerly 2A), regulatory subunit B (PR 52), alpha isoform); PPP2R3 (Protein phosphatase 2 (formerly 2A), regulatory subunit B" (PR 72), alpha isoform and (PR 130), beta isoform); PPP2R4 (Protein phosphatase 2A, regulatory subunit B' (PR 53)); PPP2R5A (Protein phosphatase 2, regulatory subunit B (B56), alpha isoform); PPP2R5B (Protein phosphatase 2, regulatory subunit B (B56), beta isoform); PPP2R5D (Protein phosphatase 2, regulatory subunit B (B56), delta isoform); PPP2R5E (Protein phosphatase 2, regulatory subunit B (B56), epsilon isoform); PPP3CA (Protein phosphatase 3 (formerly 2B), catalytic subunit, alpha isoform (calcineurin A alpha)); PPP3CB (Protein phosphatase 3 (formerly 2B), catalytic subunit, beta isoform (calcineurin A beta)); PPP4C (Protein phosphatase 4 (formerly X), catalytic subunit); PRKAA2 (Protein kinase, AMP-activated, alpha 2 catalytic subunit); PRKACA (Protein kinase, cAMP-dependent, catalytic, alpha); PRKACB (Protein kinase, cAMP-dependent, catalytic, beta); PRKACG (Protein kinase, cAMP-dependent, catalytic, gamma); PRKAG1 (Protein kinase, AMP-activated, gamma 1 non-catalytic subunit); PRKAR1A (Protein kinase, cAMP-dependent, regulatory, type I, alpha (tissue specific extinguisher 1)); PRKAR1B (Protein kinase, cAMP-dependent, regulatory, type I, beta); PRKAR2B (Protein kinase, cAMP-dependent, regulatory, type II, beta); PRKCA (Protein kinase C, alpha); PRKCB1 (Protein kinase C, beta 1); PRKCG (Protein kinase C, gamma); PRKCI (Protein kinase C, iota); PRKCL1 (Protein kinase C-like 1); PRKCL2 (Protein kinase C-like 2); PRKCM (Protein kinase C, mu); PRKCQ (Protein kinase C, theta); PRKCZ (Protein kinase C, zeta); PRKG1 (Protein kinase, cGMP-dependent, type I); PRKG2 (Protein kinase, cGMP-dependent, type II); PRKM1 (Protein kinase, mitogen-activated 1 (MAP kinase 1; p40, p41)); PRKM10 (Protein kinase mitogen-activated 10 (MAP kinase)); PRKM11 (Protein kinase mitogen- activated 11); PRKM 13 (Protein kinase mitogen-activated 13); PRKM3 (Protein kinase, mitogen-activated 3 (MAP kinase 3; p44)); PRKM6 (Protein kinase, mitogen-activated 6 (extracellular signal-regulated kinase, p97)); PRKM7 (Protein kinase mitogen-activated 7 (MAP kinase)); PRKM8 (Protein kinase mitogen-activated 8 (MAP kinase)); PRKM9 (Protein kinase mitogen-activated 9 (MAP kinase)); PRKMK2 (Protein kinase, mitogen-activated, kinase 2, p45 (MAP kinase kinase 2)); PRKMK3 (Protein kinase, mitogen-activated, kinase 3 (MAP kinase kinase 3)); PRKMK5 (Protein kinase, mitogen-activated, kinase 5 (MAP kinase kinase 5)); PRKMK7 (Protein kinase, mitogen-activated, kinase 7 (MAP kinase kinase 7)); PRKR (Protein kinase, interferon-inducible double stranded RNA dependent); PRKR (Protein kinase, interferon-inducible double stranded RNA dependent); PSME1 (Proteasome (prosome, macropain) activator subunit 1 (PA28 alpha)); PTEN (Phosphatase and tensin homolog (mutated in multiple advanced cancers 1)); PTK7 (PTK7 protein tyrosine kinase 7); PTK9 (Protein tyrosine kinase 9); PTPN1 (Protein tyrosine phosphatase, non-receptor type 1); PTPN12 (Protein tyrosine phosphatase, non-receptor type 12); PTPN13 (Protein tyrosine phosphatase, non-receptor type 13 (APO-1/CD95 (Fas)-associated phosphatase)); PTPN2 (Protein tyrosine phosphatase, non-receptor type 2); PTPN3 (Protein tyrosine phosphatase, non-receptor type 3); PTPN4 (Protein tyrosine phosphatase, non-receptor type 4 (megakaryocyte)); PTPN6 (Protein tyrosine phosphatase, non-receptor type 6); PTPN7 (Protein tyrosine phosphatase, non-receptor type 7); PTPN9 (Protein tyrosine phosphatase, non-receptor type 9); PTPRA (Protein tyrosine phosphatase, receptor type, alpha polypeptide); PTPRB (Protein tyrosine phosphatase, receptor type, beta polypeptide); PTPRC (Protein tyrosine phosphatase, receptor type, c polypeptide); PTPRD (Protein tyrosine phosphatase, receptor type, D); PTPRF (Protein tyrosine phosphatase, receptor type, F); PTPRG (Protein tyrosine phosphatase, receptor type, gamma polypeptide); PTPRH (Protein tyrosine phosphatase, receptor type, H); PTPRK (Protein tyrosine phosphatase, receptor type, K); PTPRM (Protein tyrosine phosphatase, receptor type, M); PTPRN (Protein tyrosine phosphatase, receptor type, N); PTPRN2 (Protein tyrosine phosphatase, receptor type, N polypeptide 2); PTX3 (Pentaxin-related gene, rapidly induced by IL-1 beta); RAB8IP (Rab8 interacting protein (GC kinase)); RAB8IP (Rab8 interacting protein (GC kinase)); RAD9 (RAD9 (S. pombe) homolog); RASA1 (RAS p21 protein activator (GTPase activating protein) 1); RET (Ret proto-oncogene (multiple endocrine neoplasia MEN2A, MEN2B and medullary thyroid carcinoma 1, Hirschsprung disease)); RGS1 (Regulator of G-protein signalling 1); RGS 16 (Regulator of G-protein signalling 16); RGS7 (Regulator of G-protein signalling 7); RHOK (Rhodopsin kinase); ROCK1 (Rho-associated, coiled-coil containing protein kinase 1); RPS6KA2 (Ribosomal protein S6 kinase, 90kD, polypeptide 2); RPS6KB 1 (Ribosomal protein S6 kinase, 70kD, polypeptide 1); RSN (Restin (Reed-Steinberg cell-expressed intermediate filament-associated protein)); RYK (RYK receptor-like tyrosine kinase); SAPK3 (Stress-activated protein kinase 3); SELE (Selectin E (endothelial adhesion molecule 1)); SELL (Selectin L (lymphocyte adhesion molecule 1)); SELP (Selectin P (granule membrane protein 140kD, antigen CD62)); SERK1 (SAPK/Erk kinase 1); SFN (Stratifin); SH3D1B (SH3 domain protein 1B); SLC2A3 (Solute carrier family 2 (facilitated glucose transporter), member 3); SLC2A5 (Solute carrier family 2 (facilitated glucose transporter), member 5); SPTA1 (Spectrin, alpha, erythrocytic 1 (elliptocytosis 2)); SPTB (Spectrin, beta, erythrocytic (includes sperocytosis, clinical type I)); SRC (V-src avian sarcoma (Schmidt-Ruppin A-2) viral oncogene homolog); SRF (Serum response factor (c-fos serum response element-binding transcription factor)); SRPK2 (SFRS protein kinase 2); STK11 (Serine/threonine kinase 11 (Peutz-Jeghers syndrome)); STK2 (Serine/threonine kinase 2); STK3 (Serine/threonine kinase 3 (Ste20, yeast homolog)); STK4 (Serine/threonine kinase 4); SYK (Spleen tyrosine kinase); TAP1 (Transporter 1, ABC (ATP binding cassette)); TAPBP (TAP binding protein (tapasin)); TBCC (Tubulin-specific chaperone c); TESK1 (Testis-specific kinase 1); TGFBI (Transforming growth factor, beta-induced, 68kD); THBS1 (Thrombospondin 1); TIAM1 (T-cell lymphoma invasion and metastasis 1); TIEG (TGFB inducible early growth response); TK2 (Thymidine kinase 2, mitochondrial); TNFAIP1 (Tumor necrosis factor, alpha-induced protein 1 (endothelial)); TNFAIP1 (Tumor necrosis factor, alpha-induced protein 1 (endothelial)); TNFSF5 (Tumor necrosis factor (ligand) superfamily, member 5); TTK (TTK protein kinase); TTN (Titin); TUBA1 (Tubulin, alpha I (testis specific)); TUBG (Tubulin, gamma polypeptide); TXK (TXK tyrosine kinase); TYK2 (Tyrosine kinase 2); TYRO3 (TYRO3 protein tyrosine kinase); UBE1L (Ubiquitin-activating enzyme E1, like); UBE2A (Ubiquitin-conjugating enzyme E2A (RAD6 homolog)); UBE2B (Ubiquitin-conjugating enzyme E2B (RAD6 homolog)); VASP (Vasodilator-stimulated phosphoprotein); VAV2 (Vav 2 oncogene); VCAM1 (Vascular cell adhesion molecule 1); VCL (Vinculin); VIM (Vimentin); VRK2 (Vaccinia related kinase 2); WAS (Wiskott-Aldrich syndrome (ecezema-thrombocytopenia)); ZAP70 (Zeta-chain (TCR) associated protein kinase (70 kD)); and ZPK (Zipper (leucine) protein kinase).

### Development

ACCPN (Agenesis of corpus callosum and peripheral neuropathy (Andermann); ACVR1 (Activin A receptor, type I); ACVR1B (Activin A receptor, type IB); ACVR2 (Activin A receptor, type II); ACVR2B (Activin A receptor, type IIB); ACVRL1 (Activin A receptor type II-like 1); ADFN (Albinism-deafness syndrome); AES (Amino-terminal enhancer of split); AFD1 (Acrofacial dysostosis 1, Nager type); AGC1 (Aggrecan 1 (chondroitin sulfate proteoglycan 1, large aggregating proteoglycan, antigen identified by monoclonal antibody A0122)); AHO2 (Albright hereditary osteodystrophy-2); AIH3 (Amelogenesis imperfecta 3, hypomaturation or hypoplastic type); ALX3 (Aristaless-like homeobox 3); AMCD1 (Arthrogryposis multiplex congenital, distal, type 1); AMCD2B (Arthrogryposis multiplex congenita, distal, type 2B); AMCN (Arthrogryposis multiplex congenita, neurogenic); AMCX1 (Arthrogryposis multiplex congenita, X-linked (spinal muscular atrophy,); AMDM (Acromesomelic dysplasia, Maroteaux type); AMH (Anti-Mullerian hormone); AMHR2 (Anti-Mullerian hormone receptor, type II); AMMECRI (Alport syndrome, mental retardation, midface hypoplasia and elliptocytosis chromosomal region, gene 1); ANOP1 (Anophthalmos 1 (with mental retardation, without limb anomalies or dental or urogenital abnormalities)); APC (Adenomatosis polyposis coli); ARIX (Aristaless (Drosophila) homeobox); ARVCF (Armadillo repeat gene deletes in velocardiofacial syndrome); ASCL1 (Achaete-scute complex (Drosophila) homolog-like 1); ASCL2 (Achaete-scute complex (Drosophila) homolog-like 2); ASH2L (Ash2 (absent, small, or homeotic, Drosophila, homolog)-like); ASMD (Anterior segment mesenchymal dysgenesis); ATD (Asphixiating thoracic dystrophy (chondroectodermal dysplasia-like syndrome)); ATF4 (Activating transcription factor 4 (tax-responsive enhancer element B67)); AXIN1 (Axin); AXIN2 (Axin 2 (conductin, axil)); BAD (BCL2-antagonist of cell death); BAPX1 (Bagpipe homeobox (Drosophila) homolog 1); BARD1 (BRCA1 associated RING domain 1); BARX2 (BarH-like homeobox 2); BAX (BCL2-associated X protein); BDB 1 (Brachydactyly type B1); BDC (); BDE (Brachydactyly type E); BDMR (Brachydactyly-mental retardation syndrome); BDNF (Brain-derived neurotrophic factor); BMP1 (Bone morphogenetic protein 1); BMP2 (Bone morphogenetic protein 2); BMP3 (Bone morphogenetic protein 3 (osteogenic)); BMP4 (Bone morphogenetic protein 4); BMP5 (Bone morphogenetic protein 5); BMP6 (Bone morphogenetic protein 6); BMP7 (Bone morphogenetic protein 7 (osteogenic protein 1)); BMPR1A (Bone morphogenetic protein receptor, type IA); BMPR1B (Bone morphogenetic protein receptor, type IB); BMPR2 (Bone morphogenetic protein receptor, type II (serine/threonine kinase)); CBFA1 (Core-binding factor, runt domain, alpha subunit 1); CBFA2 (Core-binding factor, runt domain, alpha subunit 2 (acute myeloid leukemia 1; aml1 oncogene)); CBFA3 (Core-binding factor, runt domain, alpha subunit 3); CER1 (Cerberus 1 (Xenopus laevis) homolog (cysteine knot superfamily)); CHH (Cartilage-hair hypoplasia); CHRD (Chordin); CHX10 (C elegans ceh-10 homeo domain-containing homolog); CREB2 (CAMP responsive element binding protein 2); CSH2 (Chorionic somatomammotropin hormone 2); DLK1 (Delta (Drosophila)-like 1); EBAF (Endometrial bleeding associated factor (left-right determination, factor A; transforming growth factor beta superfamily)); EDN1 (Endothelin 1); EDN2 (Endothelin 2); EDN3 (Endothelin 3); EDNRA (Endothelin receptor type A); EDNRB (Endothelin receptor type B); EDR2 (Early development regulator 2 (homolog of polyhomeotic 2)); EED (Embryonic ectoderm development protein); EFNA5 (Ephrin-A5); EN1 (Engrailed homolog 1); EN2 (Engrailed homolog 2); ENG (Endoglin (Osler-Rendu-Weber syndrome 1)); EOMES (Eomesodermin (Xenopus laevis) homology); EPHA2 (EphA2); EPHB2 (EphB2); FAST-1 (Human homolog of Xenopus forkhead activin signal transducer-1); FGD1 (Faciogenital dysplasia (Aarskog-Scott syndrome)); FGF1 (Fibroblast growth factor 1 (acidic)); FGF10 (Fibroblast growth factor 10); FGF13 (Fibroblast growth factor 13); FGF14 (Fibroblast growth factor 14); FGF16 (Fibroblast growth factor 16); FGF2 (Fibroblast growth factor 2 (basic)); FGF3 (Fibroblast growth factor 3 (murine mammary tumor virus integration site (v-int-2) oncogene homolog)); FGF4 (Fibroblast growth factor 4 (heparin secretory transforming protein 1, Kaposi sarcoma oncogene)); FGF7 (Fibroblast growth factor 7 (keratinocyte growth factor)); FGF8 (Fibroblast growth factor 8 (androgen-induced)); FGFR1 (Fibroblast growth factor receptor 1 (fms-related tyrosine kinase 2, Pfeiffer syndrome)); FGFR2 (Fibroblast growth factor receptor 2 (bacteria-expressed kinase, keratinocyte growth factor receptor, craniofacial dysostosis 1, Crouzon syndrome, Pfeiffer syndrome, Jackson-Weiss syndrome)); FGFR3 (Fibroblast growth factor receptor 3 (achondroplasia, thanatophoric dwarfism)); FGFR4 (Fibroblast growth factor receptor 4); FKHL16 (Forkhead (Drosophila)-like 16); FKHL7 (Forkhead (Drosophila)-like 7); FRZB (Frizzled-related protein); FZD 1 (Frizzled (Drosophila) homolog 1); FZD2 (Frizzled (Drosophila) homolog 2); FZD3 (Frizzled (Drosophila) homolog 3); FZD4 (Frizzled (Drosophila) homolog 4); FZD5 (Frizzled (Drosophila) homolog 5); FZD6 (Frizzled (Drosophila) homolog 6); FZD7 (Frizzled (Drosophila) homolog 7); FZD8 (Frizzled (Drosophila) homolog 8); FZD9 (Frizzled (Drosophila) homolog 9); GDF5 (Growth differentiation factor 5 (cartilage-derived morphogenetic protein-1)); GLI (Glioma-associated oncogene homolog (zinc finger protein)); GLI2 (GLI-Kruppel family member GLI2); GLI3 (GLI-Kruppel family member GLI3 (Greig cephalopolysyndactyly syndrome)); GLI4 (GLI-Kruppel family member GLI4); GSC (Goosecoid); GSCL (Goosecoid-like); HESX1 (Homeo box gene expressed in ES cells; Rathke pouch homeo box); HLXB9 (Homeo box HB9); HNF3A (Hepatocyte nuclear factor 3, alpha); HNF3B (Hepatocyte nuclear factor 3, beta); HNF3G (Hepatocyte nuclear factor 3, gamma); HNF4A (Hepatocyte nuclear factor 4, alpha); HNF4B (Hepatocyte nuclear factor 4, beta); HNF4G (Hepatocyte nuclear factor 4, gamma); HNF6A (Hepatocyte nuclear factor 6, alpha); HOX11 (Homeo box 11 (T-cell lymphoma 3-associated breakpoint)); HOXA1 (Homeo box A1); HOXA10 (Homeo box A10); HOXA11 (Homeo box A11); HOXA13 (Homeo box A 13); HOXA2 (Homeo box A2); HOXA3 (Homeo box A3); HOXA4 (Homeo box A4); HOXA5 (Homeo box A5); HOXA6 (Homeo box A6); HOXA7 (Homeo box A7); HOXA9 (Homeo box A9); HOXA@ (Homeo box A cluster); HOXB1 (Homeo box B1); HOXB2 (Homeo box B2); HOXB3 (Homeo box B3); HOXB4 (Homeo box B4); HOXB5 (Homeo box B5); HOXB6 (Homeo box B6); HOXB7 (Homeo box B7); HOXB8 (Homeo box B8); HOXB9 (Homeo box B9); HOXC10 (Homeo box C10); HOXC11 (Homeo box C11); HOXC12 (Homeo box C12); HOXC13 (Homeo box C13); HOXC4 (Homeo box C4); HOXC5 (Homeo box C5); HOXC6 (Homeo box C6); HOXC8 (Homeo box C8); HOXC9 (Homeo box C9); HOXD1 (Homeo box D1); HOXD10 (Homeo box D10); HOXD11 (Homeo box D11); HOXD12 (Homeo box D12); HOXD13 (Homeo box D13); HOXD3 (Homeo box D3); HOXD4 (Homeo box D4); HOXD8 (Homeo box D8); HOXD9 (Homeo box D9); IHH (Indian hedgehog (Drosophila) homolog); INHBA (Inhibin, beta A (activin A, activin AB alpha polypeptide)); INHBB (Inhibin, beta B (activin AB beta polypeptide)); ISL1 (ISL1 transcription factor, LIM/homeodomain, (islet-1)); JAG1 (Jagged1 (Alagille syndrome)); JAG2 (Jagged 2); LEF1 (Lymphoid enhancer-binding factor 1); LHX1 (LIM homeobox protein 1); LHX2 (LIM HOX gene 2); LHX3 (LIM/homeodomain protein LHX3); LMX1A (LIM homeobox transcription factor 1, alpha); LMX1B (LIM homeobox transcription factor 1, beta); MADH1 (MAD (mothers against decapentaplegic, Drosophila) homolog 1); MADH2 (MAD (mothers against decapentaplegic, Drosophila) homolog 2); MADH3 (MAD (mothers against decapentaplegic, Drosophila) homolog 3); MADH4 (MAD (mothers against decapentaplegic, Drosophila) homolog 4); MADH5 (MAD (mothers against decapentaplegic, Drosophila) homolog 5); MADH6 (MAD (mothers against decapentaplegic, Drosophila) homolog 6); MADH7 (MAD (mothers against decapentaplegic, Drosophila) homolog 7); MADH9 (MAD (mothers against decapentaplegic, Drosophila) homolog 9); MEIS1 (Meis1 (mouse) homolog); MEIS2 (Meis (mouse) homolog 2); MEIS3 (Meis (mouse) homolog 3); MEKK5 (MAP/ERK kinase kinase 5); MLLT1 (Myeloid/lymphoid or mixed-lineage leukemia (trithorax (Drosophila) homolog); translocated to, 1); MSX1 (Msh (Drosophila) homeo box homolog 1 (formerly homeo box 7)); MSX2 (Msh (Drosophila) homeo box homolog 2); NOG (Noggin); ORW2 (Osler-Rendu-Weber syndrome 2); PAX1 (Paired box gene 1); PAX1 (Paired box gene 1); PAX2 (Paired box gene 2); PAX3 (Paired box gene 3 (Waardenburg syndrome 1)); PAX4 (Paired box gene 4); PAX5 (Paired box gene 5 (B-cell lineage specific activator protein)); PAX6 (Paired box gene 6 (aniridia, keratitis)); PAX7 (Paired box gene 7); PAX8 (Paired box gene 8); PAX9 (Paired box gene 9); PBX1 (Pre-B-cell leukemia transcription factor 1); PBX2 (Pre-B-cell leukemia transcription factor 2); PBX3 (Pre-B-cell leukemia transcription factor 3); PITX2 (Paired-like homeodomain transcription factor 2); PKHD1 (Polycystic kidney and hepatic disease 1 (autosomal recessive)); PROP1 (Prophet of Pit1, paired-like homeodomain transcription factor); PTCH (Patched (Drosophila) homolog); PTCH2 (Patched (Drosophila) homolog 2); RARA (Retinoic acid receptor, alpha); RARB (Retinoic acid receptor, beta); RARG (Retinoic acid receptor, gamma); RET (Ret proto-oncogene (multiple endocrine neoplasia MEN2A, MEN2B and medullary thyroid carcinoma 1, Hirschsprung disease)); RIEG2 (Rieger syndrome 2); RXRA (Retinoid X receptor, alpha); RXRB (Retinoid X receptor, beta); RXRG (Retinoid X receptor, gamma); SFRP1 (Secreted frizzled-related protein 1); SFRP2 (Secreted frizzled-related protein 2); SFRP4 (Secreted frizzled-related protein 4); SFRP5 (Secreted frizzled-related protein 5); SHH (Sonic hedgehog (Drosophila) homolog); SMARCA2 (SWI/SNF related, matrix associated, actin dependent regulator of chromatin, subfamily a, member 2); SMARCA4 (SWI/SNF related, matrix associated, actin dependent regulator of chromatin, subfamily a, member 4); SMARCB1 (SWI/SNF related, matrix associated, actin dependent regulator of chromatin, subfamily b, member 1); SMARCC1 (SWI/SNF related, matrix associated, actin dependent regulator of chromatin, subfamily c, member 1); SMARCC2 (SWI/SNF related, matrix associated, actin dependent regulator of chromatin, subfamily c, member 2); SMARCD1 (SWI/SNF related, matrix associated, actin dependent regulator of chromatin, subfamily d, member 1); SMARCD2 (SWI/SNF related, matrix associated, actin dependent regulator of chromatin, subfamily d, member 2); SMARCD3 (SWI/SNF related, matrix associated, actin dependent regulator of chromatin, subfamily d, member 3); SMARCE1 (SWI/SNF related, matrix associated, actin dependent regulator of chromatin, subfamily e, member 1); SMOH (Smoothened (Drosophila) homolog); SOX1 (SRY (sex determining region Y)-box 1); SOX10 (SRY (sex-determining region Y)-box 10); SOX2 (SRY (sex determining region Y)-box 2); SOX9 (SRY (sex-determining region Y)-box 9 (campomelic dysplasia, autosomal sex-reversal)); SRF (Serum response factor (c-fos serum response element-binding transcription factor)); STAT3 (Signal transducer and activator of transcription 3 (acute-phase response factor)); T (T brachyury (mouse) homolog); TBX1 (T-box 1); TBX10 (T-box 10); TBX10 (T-box 10); TBX15 (T-box 15); TBX18 (T-box 18); TBX19 (T-box 19); TBX2 (T-box 2); TBX3 (T-box 3 (ulnar mammary syndrome)); TBX4 (T-box 4); TBX5 (T-box 5); TBX6 (T-box 6); TBX7 (T-box 7); TFAP2A (Transcription factor AP-2 alpha (activating enhancer-binding protein 2 alpha)); TFAP2B (Transcription factor AP-2 beta (activating enhancer-binding protein 2 beta)); TFCOUP2 (Transcription factor COUP 2 (chicken ovalbumin upstream promoter 2, apolipoprotein regulatory protein)); TGFBR1 (Transforming growth factor, beta receptor I (activin A receptor type II-like kinase, 53kD)); TWIST (Twist (Drosophila) homolog); WNT1 (Wingless-type MMTV integration site family, member 1); WNT10B (Wingless-type MMTV integration site family, member 10B); WNT11 (Wingless-type MMTV integration site family, member 11); WNT14 (Wingless-type MMTV integration site family, member 14); WNT15 (Wingless-type MMTV integration site family, member 15); WNT2 (Wingless-type MMTV integration site family member 2); WNT2B (Wingless-type MMTV integration site family, member 2B); WNT3 (Wingless-type MMTV integration site family, member 3); WNT5A (Wingless-type MMTV integration site family, member 5A); WNT6 (Wingless-type MMTV integration site family, member 6); WNT7A (Wingless-type MMTV integration site family, member 7A); WNT7B (Wingless-type MMTV integration site family, member 7B); WNT8A (Wingless-type MMTV integration site family, member 8A); WNT8B (Wingless-type MMTV integration site family, member 8B); ZIC3 (Zic family member 3 (odd-paired Drosophila homolog, heterotaxy 1)); and ZIC3 (Zic family member 3 (odd-paired Drosophila homolog, heterotaxy 1)).

### DNA adducts

DFFA (DNA fragmentation factor, 45 kD, alpha subunit); DNMT1 (DNA (cytosine-5-)-methyltransferase 1); DNMT2 (DNA (cytosine-5-)-methyltransferase 2); IGHMBP2 (Immunoglobulin mu binding protein 2); LIG1 (Ligase I, DNA, ATP-dependent); LIG3 (Ligase III, DNA, ATP-dependent); LIG4 (Ligase IV, DNA, ATP-dependent); MGMT (O-6-methylguanine-DNA methyltransferase); NTHL1 (Nth (E.coli endonuclease III)-like 1); PRIM1 (Primase, polypeptide 1 (49kD)); RAG1 (Recombination activating gene 1); RFC3 (Replication factor C (activator 1) 3 (38kD)); RFC4 (Replication factor C (activator 1) 4 (37kD)); TERT (Telomerase reverse transcriptase); TOP1 (Topoisomerase (DNA) I); TOP2A (Topoisomerase (DNA) II alpha (170kD)); TOP2B (Topoisomerase (DNA) II beta (180kD)); TOP3 (Topoisomerase (DNA) III); and TOP3B (Topoisomerase (DNA) III beta).

### DNA damage

ADPRT (ADP-ribosyltransferase (NAD+; poly (ADP-ribose) polymerase)); APEX (APEX nuclease (multifunctional DNA repair enzyme)); ATM (Ataxia telangiectasia mutated (includes complementation groups A, C and D)); BLM (Bloom syndrome); BRCA1 (Breast cancer 1, early onset); BRCA2 (Breast cancer 2, early onset); CKN1 (Cockayne syndrome 1 (classical)); DDB1 (Damage-specific DNA binding protein 1 (127kD)); DDB2 (Damage-specific DNA binding protein 2 (48kD)); DDIT1 (DNA-damage-inducible transcript 1); DDIT1L (DNA-damage-inducible transcript 1-like); DDIT3 (DNA-damage-inducible transcript 3); DFFA (DNA fragmentation factor, 45 kD, alpha subunit); DNMT1 (DNA (cytosine-5-)-methyltransferase 1); DNMT2 (DNA (cytosine-5-)-methyltransferase 2); ERCC1 (Excision repair cross-complementing rodent repair deficiency, complementation group 1 (includes overlapping antisense sequence)); ERCC2 (Excision repair cross-complementing rodent repair deficiency, complementation group 2 (xeroderma pigmentosum D)); ERCC3 (Excision repair cross-complementing rodent repair deficiency, complementation group 3 (xeroderma pigmentosum group B complementing)); ERCC5 (Excision repair cross-complementing rodent repair deficiency, complementation group 5 (xeroderma pigmentosum, complementation group G (Cockayne syndrome))); ERCC6 (Excision repair cross-complementing rodent repair deficiency, complementation group 6); FANCA (Fanconi anemia, complementation group A); FANCB (Fanconi anemia, complementation group B); FANCC (Fanconi anemia, complementation group C); FANCG (Fanconi anemia, complementation group G); G22P1 (Thyroid autoantigen 70kD (Ku antigen)); GTBP (G/T mismatch-binding protein); IGHMBP2 (Immunoglobulin mu binding protein 2); INPPL1 (Inositol polyphosphate phosphatase-like 1); LIG1 (Ligase I, DNA, ATP-dependent); LIG3 (Ligase III, DNA, ATP-dependent); LIG3 (Ligase III, DNA, ATP-dependent); LIG4 (Ligase IV, DNA, ATP-dependent); MGMT (O-6-methylguanine-DNA methyltransferase); MLH1 (MutL (E. coli) homolog 1 (colon cancer, nonpolyposis type 2)); MPG (N-methylpurine-DNA glycosylase); MSH2 (MutS (E. coli) homolog 2 (colon cancer, nonpolyposis type 1)); MSH3 (MutS (E. coli) homolog 3); MSH4 (MutS (E. coli) homolog 4); MSH5 (MutS (E. coli) homolog 5); MTH1 (MutT (E. coli) human homolog (8-oxo-7,8-dihydroguanosine triphosphatase)); NTHL1 (Nth (E.coli endonuclease III)-like 1); PMS1 (Postmeiotic segregation increased (S. cerevisiae) 1); PMS2 (Postmeiotic segregation increased (S. cerevisiae) 2); PMS2L1 (Postmeiotic segregation increased 2-like 1); PMS2L11 (Postmeiotic segregation increased 2-like 11); PMS2L12 (Postmeiotic segregation increased 2-like 12); PMS2L2 (Postmeiotic segregation increased 2-like 2); PMS2L3 (Postmeiotic segregation increased 2-like 3); PMS2L4 (Postmeiotic segregation increased 2-like 4); PMS2L5 (Postmeiotic segregation increased 2-like 5); PMS2L6 (Postmeiotic segregation increased 2-like 6); PMS2L8 (Postmeiotic segregation increased 2-like 8); PMS2L9 (Postmeiotic segregation increased 2-like 9); POLB (Polymerase (DNA directed), beta); PRIM1 (Primase, polypeptide 1 (49kD)); PRKDC (Protein kinase, DNA-activated, catalytic polypeptide); RAD1 (RAD1 (S. pombe) homolog); RAD17 (RAD17 (S. pombe) homolog); RAD21 (RAD21 (S. pombe) homolog); RAD23A (RAD23 (S. cerevisiae) homolog A); RAD23B (RAD23 (S. cerevisiae) homolog B); RAD51 (RAD51 (S. cerevisiae) homolog (E coli RecA homolog)); RAD51C (RAD51 (S. cerevisiae) homolog C); RAD51L1 (RAD51 (S. cerevisiae)-like 1); RAD51L3 (RAD51 (S. cerevisiae)-like 3); RAD52 (RAD52 (S. cerevisiae) homolog); RAD54L (RAD54 (S.cerevisiae)-like); RAG1 (Recombination activating gene 1); RFC3 (Replication factor C (activator 1) 3 (38kD)); RFC4 (Replication factor C (activator 1) 4 (37kD)); TDG (Thymine-DNA glycosylase); TERT (Telomerase reverse transcriptase); TOP1 (Topoisomerase (DNA) I); TOP2A (Topoisomerase (DNA) II alpha (170kD)); TOP2B (Topoisomerase (DNA) II beta (180kD)); TOP3 (Topoisomerase (DNA) III); TOP3B (Topoisomerase (DNA) III beta); TP53 (Tumor protein p53 (Li-Fraumeni syndrome)); TRLP1 (TRNA leucine (AAG) pseudogene 1); UNG (Uracil-DNA glycosylase); WRN (Werner syndrome); XPA (Xeroderma pigmentosum, complementation group A); XPC (Xeroderma pigmentosum, complementation group C); XRCC1 (X-ray repair complementing defective repair in Chinese hamster cells 1); XRCC2 (X-ray repair complementing defective repair in Chinese hamster cells 2); XRCC3 (X-ray repair complementing defective repair in Chinese hamster cells 3); XRCC4 (X-ray repair complementing defective repair in Chinese hamster cells 4); XRCC5 (X-ray repair complementing defective repair in Chinese hamster cells 5 (double-strand-break rejoining; Ku autoantigen, 80kD)); and XRCC8 (X-ray repair complementing defective repair in Chinese hamster cells 8).

### DNA replication

ADAR (Adenosine deaminase, RNA-specific); ADPRT (ADP-ribosyltransferase (NAD+; poly (ADP-ribose) polymerase)); ATM (Ataxia telangiectasia mutated (includes complementation groups A, C and D)); ATR (Ataxia telangiectasia and Rad3 related); ATRX (Alpha thalassemia/mental retardation syndrome X-linked); BLM (Bloom syndrome); CENPB (Centromere protein B (80kD)); CENPC1 (Centromere protein C 1); CHD4 (Chromodomain helicase DNA binding protein 4); CHDL (Chromodomain-helicase-DNA-binding protein); CKN1 (Cockayne syndrome 1 (classical)); DNA2L (DNA2 (DNA replication helicase, yeast, homolog)-like); DNASE1 (Deoxyribonuclease I); DNASE1L1 (Deoxyribonuclease I-like 1); DNASE1L2 (Deoxyribonuclease I-like 2); ERCC1 (Excision repair cross-complementing rodent repair deficiency, complementation group 1 (includes overlapping antisense sequence)); ERCC2 (Excision repair cross-complementing rodent repair deficiency, complementation group 2 (xeroderma pigmentosum D)); ERCC3 (Excision repair cross-complementing rodent repair deficiency, complementation group 3 (xeroderma pigmentosum group B complementing)); ERCC4 (Excision repair cross-complementing rodent repair deficiency, complementation group 4); ERCC5 (Excision repair cross-complementing rodent repair deficiency, complementation group 5 (xeroderma pigmentosum, complementation group G (Cockayne syndrome))); ERCC6 (Excision repair cross-complementing rodent repair deficiency, complementation group 6); EXO1 (Exonuclease 1); FEN1 (Flap structure-specific endonuclease 1); G22P1 (Thyroid autoantigen 70kD (Ku antigen)); HMGIY (High-mobility group (nonhistone chromosomal) protein isoforms I and Y); HUS1 (HUS1 (S. pombe) checkpoint homolog); LIG2 (Ligase II, DNA, ATP-dependent); MLH 1 (MutL (E. coli) homolog 1 (colon cancer, nonpolyposis type 2)); MSH5 (MutS (E. coli) homolog 5); POLA (Polymerase (DNA directed), alpha); POLB (Polymerase (DNA directed), beta); POLD1 (Polymerase (DNA directed), delta 1, catalytic subunit (125kD)); RAD1 (RAD1 (S. pombe) homolog); RAD50 (RAD50 (S. cerevisiae) homolog); RAD51 (RAD51 (S. cerevisiae) homolog (E coli RecA homolog)); RAD51C (RAD51 (S. cerevisiae) homolog C); RAD52 (RAD52 (S. cerevisiae) homolog); RPA1 (Replication protein A1 (70kD)); RPA2 (Replication protein A2 (32kD)); RPA3 (Replication protein A3 (14kD)); RPA40 (RNA polymerase I subunit); SNRPA (Small nuclear ribonucleoprotein polypeptide A); SNRPA1 (Small nuclear ribonucleoprotein polypeptide A'); TOP1 (Topoisomerase (DNA) I); TOP2A (Topoisomerase (DNA) II alpha (170kD)); TOP3 (Topoisomerase (DNA) III); TOP3B (Topoisomerase (DNA) III beta); TR4 (TR4 nuclear hormone receptor); WRN (Werner syndrome); XPA (Xeroderma pigmentosum, complementation group A); XRCC1 (X-ray repair complementing defective repair in Chinese hamster cells 1); XRCC2 (X-ray repair complementing defective repair in Chinese hamster cells 2); XRCC3 (X-ray repair complementing defective repair in Chinese hamster cells 3); XRCC4 (X-ray repair complementing defective repair in Chinese hamster cells 4); and XRCC5 (X-ray repair complementing defective repair in Chinese hamster cells 5 (double-strand-break rejoining; Ku autoantigen, 80kD)).

### Gene regulation

ADA (Adenosine deaminase); ADPRT (ADP-ribosyltransferase (NAD+; poly (ADP-ribose) polymerase)); ADSS (Adenylosuccinate synthase); AHR (Aryl hydrocarbon receptor); ATBF1 (AT-binding transcription factor 1); ATF3 (Activating transcription factor 3); BARD1 (BRCA1 associated RING domain 1); CBF2 (CCAAT-box-binding transcription factor); CBFA2 (Core-binding factor, runt domain, alpha subunit 2 (acute myeloid leukemia 1; aml1 oncogene)); CBFA3 (Core-binding factor, runt domain, alpha subunit 3); CBFB (Core-binding factor, beta subunit); CEBPA (CCAAT/enhancer binding protein (C/EBP), alpha); CEBPB (CCAAT/enhancer binding protein (C/EBP), beta); CEBPD (CCAAT/enhancer binding protein (C/EBP), delta); CEBPE (CCAAT/enhancer binding protein (C/EBP), epsilon); CEBPG (CCAAT/enhancer binding protein (C/EBP), gamma); CENPE (Centromere protein E (312kD)); CHD1 (Chromodomain helicase DNA binding protein 1); CHD2 (Chromodomain helicase DNA binding protein 2); CHD3 (Chromodomain helicase DNA binding protein 3); CHDL (Chromodomain-helicase-DNA-binding protein); CREB2 (CAMP responsive element binding protein 2); CREBBP (CREB binding protein (Rubinstein-Taybi syndrome)); CSE1L (Chromosome segregation 1 (yeast homolog)-like); CSTF3 (Cleavage stimulation factor, 3' pre-RNA, subunit 3, 77kD); CTPS (CTP synthase); DCK (Deoxycytidine kinase); DCTD (DCMP deaminase); DGUOK (Deoxyguanosine kinase); DNASE1 (Deoxyribonuclease I); DNASE1L3 (Deoxyribonuclease I-like 3); DUT (DUTP pyrophosphatase); DXS423E (Segregation of mitotic chromosomes 1 (SMC1, yeast human homolog of;); E2F2 (E2F transcription factor 2); E2F5 (E2F transcription factor 5, p130-binding); EEF1A1 (Eukaryotic translation elongation factor 1 alpha 1); EEF2 (Eukaryotic translation elongation factor 2); EGR1 (Early growth response 1); EIF2B1 (Eukaryotic translation initiation factor 2B, subunit 1 (alpha, 26kD)); EIF3S6 (Eukaryotic translation initiation factor 3, subunit 6 (48kD)); EIF4E (Eukaryotic translation initiation factor 4E); EIF4G2 (Eukaryotic translation initiation factor 4 gamma, 2); ELF1 (E74-like factor 1 (ets domain transcription factor)); ELF3 (E74-like factor 3 (ets domain transcription factor)); ELK4 (ELK4, ETS-domain protein (SRF accessory protein 1) NOTE: Symbol and name provisional); EP300 (E1A binding protein p300); ESR1 (Estrogen receptor 1); ETV3 (Ets variant gene 3); ETV4 (Ets variant gene 4 (E1A enhancer-binding protein, E1AF)); ETV5 (Ets variant gene 5 (ets-related molecule)); FKHR (Forkhead (Drosophila) homolog 1 (rhabdomyosarcoma)); FLI1 (Friend leukemia virus integration 1); GART (Phosphoribosylglycinamide formyltransferase, phosphoribosylglycinamide synthetase, phosphoribosylaminoimidazole synthetase); GATA1 (GATA-binding protein 1 (globin transcription factor 1)); GATA2 (GATA-binding protein 2); GATA3 (GATA-binding protein 3); GATA4 (GATA-binding protein 4); GLI (Glioma-associated oncogene homolog (zinc finger protein)); GRSF1 (G-rich RNA sequence binding factor 1); H4FI (H4 histone family, member I); HIF1A (Hypoxia-inducible factor 1, alpha subunit (basic helix-loop-helix transcription factor)); HIVEP1 (Human immunodeficiency virus type I enhancer-binding protein 1); HLF (Hepatic leukemia factor); HMG17 (High-mobility group (nonhistone chromosomal) protein 17); HMG2 (High-mobility group (nonhistone chromosomal) protein 2); HNRPK (Heterogeneous nuclear ribonucleoprotein K); ICSBP1 (Interferon consensus sequence binding protein 1); ID1 (Inhibitor of DNA binding 1, dominant negative helix-loop-helix protein); ID2 (Inhibitor of DNA binding 2, dominant negative helix-loop-helix protein); ID3 (Inhibitor of DNA binding 3, dominant negative helix-loop-helix protein); ID4 (Inhibitor of DNA binding 4, dominant negative helix-loop-helix protein); IRF1 (Interferon regulatory factor 1); IRF2 (Interferon regulatory factor 2); IRF4 (Interferon regulatory factor 4); IRF5 (Interferon regulatory factor 5); IRF7 (Interferon regulatory factor 7); JUN (V-jun avian sarcoma virus 17 oncogene homolog); JUND (Jun D proto-oncogene); LAF4 (Lymphoid nuclear protein related to AF4); LYL1 (Lymphoblastic leukemia derived sequence 1); MAFG (V-maf musculoaponeurotic fibrosarcoma (avian) oncogene family, protein G); MAX (MAX protein); MDM2 (Mouse double minute 2, human homolog of; p53-binding protein); MHC2TA (MHC class II transactivator); MKI67 (Antigen identified by monoclonal antibody Ki-67); MNDA (Myeloid cell nuclear differentiation antigen); MSX1 (Msh (Drosophila) homeo box homolog 1 (formerly homeo box 7)); MTHFD (5,10-methylenetetrahydrofolate dehydrogenase, 5,10-methylenetetrahydrofolate cyclohydrolase, 10-formyltetrahydrofolate synthetase); MYC (V-myc avian myelocytomatosis viral oncogene homolog); NCBP (Nuclear cap binding protein, 80kD); NCBP (Nuclear cap binding protein, 80kD); NDP52 (Nuclear domain 10 protein); NFE2 (Nuclear factor (erythroid-derived 2), 45kD); NFKB1 (Nuclear factor of kappa light polypeptide gene enhancer in B-cells 1 (p105)); NFKB2 (Nuclear factor of kappa light polypeptide gene enhancer in B-cells 2 (p49/p100)); NFYA (Nuclear transcription factor Y, alpha); NP (Nucleoside phosphorylase); NUMA1 (Nuclear mitotic apparatus protein 1); ODC 1 (Ornithine decarboxylase 1); PAX3 (Paired box gene 3 (Waardenburg syndrome 1)); PBX1 (Pre-B-cell leukemia transcription factor 1); PBX3 (Pre-B-cell leukemia transcription factor 3); PCNA (Proliferating cell nuclear antigen); PEX6 (Peroxisomal biogenesis factor 6); PML (Promyelocytic leukemia); POU2AF1 (POU domain, class 2, associating factor 1); POU2F1 (POU domain, class 2, transcription factor 1); POU2F2 (POU domain, class 2, transcription factor 2); PPAT (Phosphoribosyl pyrophosphate amidotransferase); PRPS1 (Phosphoribosyl pyrophosphate synthetase 1); PTB (Polypyrimidine tract binding protein (heterogeneous nuclear ribonucleoprotein I)); RANBP2 (RAN binding protein 2); RARB (Retinoic acid receptor, beta); RARG (Retinoic acid receptor, gamma); RECQL (RecQ protein-like (DNA helicase Q1-like)); RENBP (Renin-binding protein); RPL27 (Ribosomal protein L27); RPL32 (Ribosomal protein L32); RPL5 (Ribosomal protein L5); RPS16 (Ribosomal protein S16); RPS21 (Ribosomal protein S21); RPS5 (Ribosomal protein S5); RPS9 (Ribosomal protein S9); RXRA (Retinoid X receptor, alpha); SATB1 (Special AT-rich sequence binding protein 1 (binds to nuclear matrix/scaffold-associating DNA's)); SFRS7 (Splicing factor, arginine/serine-rich 7 (35kD)); SKIL (SKI-like); SMARCA2 (SWI/SNF related, matrix associated, actin dependent regulator of chromatin, subfamily a, member 2); SOX4 (SRY (sex determining region Y)-box 4); SP100 (Nuclear antigen Sp100); SPIB (Spi-B transcription factor (Spi-1/PU.1 related)); SRF (Serum response factor (c-fos serum response element-binding transcription factor)); STAT3 (Signal transducer and activator of transcription 3 (acute-phase response factor)); STAT4 (Signal transducer and activator of transcription 4); STAT5A (Signal transducer and activator of transcription 5A); TAF2C2 (TATA box binding protein (TBP)-associated factor, RNA polymerase II, C2, 105kD); TAL2 (T-cell acute lymphocytic leukemia 2); TCEB1L (Transcription elongation factor B (SIII), polypeptide 1-like); TCF12 (Transcription factor 12 (HTF4, helix-loop-helix transcription factors 4)); TCF3 (Transcription factor 3 (E2A immunoglobulin enhancer binding factors E12/E47)); TCF7 (Transcription factor 7 (T-cell specific, HMG-box)); TFAP2B (Transcription factor AP-2 beta (activating enhancer-binding protein 2 beta)); TFAP4 (Transcription factor AP-4 (activating enhancer-binding protein 4)); TFDP2 (Transcription factor Dp-2 (E2F dimerization partner 2)); THRA (Thyroid hormone receptor, alpha (avian erythroblastic leukemia viral (v-erb-a) oncogene homolog)); TIAL1 (TIA1 cytotoxic granule-associated RNA-binding protein-like 1); TK1 (Thymidine kinase 1, soluble); TOP1 (Topoisomerase (DNA) I); TOP2B (Topoisomerase (DNA) II beta (180kD)); TP53 (Tumor protein p53 (Li-Fraumeni syndrome)); UBL1 (Ubiquitin-like 1 (sentrin)); WT1 (Wilms tumor 1); XPO1 (Exportin 1 (CRM1, yeast, homolog)); ZFP36 (Zinc finger protein homologous to Zfp-36 in mouse); ZNF173 (Zinc finger protein 173); ZNF200 (Zinc finger protein 200); ZNF42 (Zinc finger protein 42 (myeloid-specific retinoic acid- responsive));

### Immunology

A1BG (Alpha-1-B glycoprotein); A2M (Alpha-2-macroglobulin); AAA (Achalasia-addisonianism-alacrimia syndrome (Allgrove syndrome); AABT (Beta-amino acids, renal transport of); AACT (Alpha-1-antichymotrypsin); AARS (Alanyl-tRNA synthetase); ABAT (4-aminobutyrate aminotransferase); ABC7 (ATP-binding cassette 7); ABO (ABO blood group (transferase A, alpha); 1-3-N-acetylgalactosaminyltransferase; transferase B, alpha 1-3-galactosyltransferase);ACADL (Acyl-Coenzyme A dehydrogenase, long chain); ACADM (Acyl-Coenzyme A dehydrogenase, C-4 to C-12 straight chain); ACADS (Acyl-Coenzyme A dehydrogenase, C-2 to C-3 short chain); ACAT2 (Acetyl-Coenzyme A acetyltransferase 2 (acetoacetyl Coenzyme A thiolase)); ACHE (Acetylcholinesterase (YT blood group)); ACLS (Acrocallosal syndrome); ACO1 (Aconitase 1, soluble); ACO2 (Aconitase 2, mitochondrial); ACP1 (Acid phosphatase 1, soluble); ACP5 (Acid phosphatase 5, tartrate resistant); ACTC (Actin, alpha, cardiac muscle); ACTG2 (Actin, gamma 2, smooth muscle, enteric); ACTN2 (Actinin, alpha 2); ACUG (Arthrocutaneouveal granulomatosis (Blau syndrome)); ACY1 (Aminoacylase 1); ADA (Adenosine deaminase); ADAM10 (A disintegrin and metalloprotease domain 10); ADAM8 (A disintegrin and metalloprotease domain 8); ADCYAP1 (Adenylate cyclase activating polypeptide 1 (pituitary)); ADD1 (Adducin 1 (alpha)); ADH1 (Alcohol dehydrogenase 1 (class I), alpha polypeptide); ADH2 (Alcohol dehydrogenase 2 (class I), beta polypeptide); ADH3 (Alcohol dehydrogenase 3 (class I), gamma polypeptide); ADH4 (Alcohol dehydrogenase 4 (class II), pi polypeptide); ADH5 (Alcohol dehydrogenase 5 (class III), chi polypeptide); ADH5 (Alcohol dehydrogenase 5 (class III), chi polypeptide); ADK (Adenosine kinase); ADORA1 (Adenosine A1 receptor); ADORA2A (Adenosine A2a receptor); ADORA2A (Adenosine A2a receptor); ADPRT (ADP-ribosyltransferase (NAD+; poly (ADP-ribose) polymerase)); ADRA2A (Adrenergic, alpha-2A-, receptor); ADRA2B (Adrenergic, alpha-2B-, receptor); ADRA2C (Adrenergic, alpha-2C-, receptor); ADRB 1 (Adrenergic, beta-1-, receptor); ADRB2 (Adrenergic, beta-2-, receptor, surface); ADRB3 (Adrenergic, beta-3-, receptor); ADSS (Adenylosuccinate synthase); AFP (Alpha-fetoprotein); AGA (Aspartylglucosaminidase); AGMX2 (Agammaglobulinemia, X-linked 2 (with growth hormone deficiency)); AGT (Angiotensinogen); AGTR1 (Angiotensin receptor 1); AGTR2 (Angiotensin receptor 2); AGXT (Alanine-glyoxylate aminotransferase (oxalosis I; hyperoxaluria I; glycolicaciduria; serine-pyruvate aminotransferase)); AHCY (S-adenosylhomocysteine hydrolase); AHR (Aryl hydrocarbon receptor); AHSG (Alpha-2-HS-glycoprotein); AIH2 (Amelogenesis imperfecta 2, hypocalcification (autosomal dominant)); AIRE (Autoimmune regulator (automimmune) polyendocrinopathy candidiasis ectodermal dystrophy)); AK1 (Adenylate kinase 1); AKT1 (V-akt murine thymoma viral oncogene homolog 1); ALAD (Aminolevulinate, delta-, dehydratase); ALAS2 (Aminolevulinate, delta-, synthase 2) (sideroblastic/hypochromic anemia)); ALB (Albumin); ALD (Adrenoleukodystrophy/adrenomyeloneuropathy); ALDH1 (Aldehyde dehydrogenase 1, soluble); ALDH2 (Aldehyde dehydrogenase 2, mitochondrial); ALDH6 (Aldehyde dehydrogenase 6); ALDH9 (Aldehyde dehydrogenase 9 (gamma-aminobutyraldehyde dehydrogenase, E3 isozyme)); ALDOA (Aldolase A, fructose-bisphosphate); ALDOB (Aldolase B, fructose-bisphosphate); ALOX12 (Arachidonate 12-lipoxygenase); ALOX5 (Arachidonate 5-lipoxygenase); ALPL (Alkaline phosphatase, liver/bone/kidney); ALPP (Alkaline phosphatase, placental (Regan isozyme)); ALPPL2 (Alkaline phosphatase, placental-like 2); AMBP (Alpha-1-microglobulin/bikunin precursor); AMCN (Arthrogryposis multiplex congenita, neurogenic); AMELX (Amelogenin (X chromosome, amelogenesis imperfecta 1)); AMH (Anti-Mullerian hormone); AMPH (Amphiphysin (Stiff-Mann syndrome with breast cancer 128kD autoantigen)); AMPHL (Amphiphysin-like); AMY1A (Amylase, alpha 1A; salivary); AMY2A (Amylase, alpha 2A; pancreatic); ANK1 (Ankyrin 1, erythrocytic); ANPEP (Alanyl (membrane) aminopeptidase (aminopeptidase N, aminopeptidase M, microsomal aminopeptidase, CD13, p150)); ANX11 (Annexin XI (56kD autoantigen)); ANX3 (Annexin III (lipocortin III, 1,2-cyclic-inositol-phosphate phosphodiesterase, placental anticoagulant protein III, calcimedin 35-alpha)); ANX4 (Annexin IV (placental anticoagulant protein II)); ANX5 (Annexin V (endonexin II)); ANX8 (Annexin VIII); APAF1 (Apoptotic protease activating factor); APBA2 (Amyloid beta (A4) precursor protein-binding, family A, member 2 (X11-like)); APBB1 (Amyloid beta (A4) precursor protein-binding, family B, member 1 (Fe65)); APC (Adenomatosis polyposis coli); APCS (Amyloid P component, serum); APEH (N-acylaminoacyl-peptide hydrolase); API1 (Apoptosis inhibitor 1); API2 (Apoptosis inhibitor 2); API3 (Apoptosis inhibitor 3); API4 (Apoptosis inhibitor 4 (survivin)); APOA1 (Apolipoprotein A-I); APOA2 (Apolipoprotein A-II); APOA4 (Apolipoprotein A-IV); APOB (Apolipoprotein B (including Ag(x) antigen)); APOC2 (Apolipoprotein C-II); APOC3 (Apolipoprotein C-III); APOD (Apolipoprotein D); APOE (Apolipoprotein E); APOH (Apolipoprotein H (beta-2-glycoprotein I)); APP (Amyloid beta (A4) precursor protein (protease nexin-II, Alzheimer disease)); APRT (Adenine phosphoribosyltransferase); AQP1 (Aquaporin 1 (channel-forming integral protein, 28kD)); AQP2 (Aquaporin 2 (collecting duct)); AR (Androgen receptor (dihydrotestosterone receptor; testicular feminization; spinal and bulbar muscular atrophy; Kennedy disease)); AREG (Amphiregulin (schwannoma-derived growth factor)); ARHE (Ras homolog gene family, member E); ARHGAP1 (Rho GTPase activating protein 1); ARHGAP5 (Rho GTPase activating protein 5); ARNT (Aryl hydrocarbon receptor nuclear translocator); ARNTL (Aryl hydrocarbon receptor nuclear translocator-like); ARSA (Arylsulfatase A); ART1 (ADP-ribosyltransferase 1); ARVD2 (Arrhythmogenic right ventricular dysplasia 2); ASL (Argininosuccinate lyase); ASNS (Asparagine synthetase); ASPH (Aspartate beta-hydroxylase); AT3 (Antithrombin III); ATCAY (Ataxia, cerebellar, Cayman type); ATM (Ataxia telangiectasia mutated (includes complementation) groups A, C and D)); ATOX1 (ATX1 (antioxidant protein 1, yeast) homolog 1); ATP1A1 (ATPase, Na+/K+ transporting, alpha 1 polypeptide); ATP2B2 (ATPase, Ca++ transporting, plasma membrane 2); ATP4B (ATPase, H+/K+ exchanging, beta polypeptide); ATP7B (ATPase, Cu++ transporting, beta polypeptide (Wilson disease)); ATR (Ataxia telangiectasia and Rad3 related); ATRN (Attractin (with dipeptidylpeptidase IV activity)); ATRX (Alpha thalassemia/mental retardation syndrome X-linked); ATSV (Axonal transport of synaptic vesicles); AUH (AU RNA-binding protein/enoyl-Coenzyme A hydratase); AVP (Arginine vasopressin (neurophysin II, antidiuretic hormone, diabetes insipidus, neurohypophyseal)); AVPR1A (Arginine vasopressin receptor 1A); AVPR2 (Arginine vasopressin receptor 2 (nephrogenic diabetes insipidus)); AXL (AXL receptor tyrosine kinase); AZGP1 (Alpha-2-glycoprotein 1, zinc); AZU1 (Azurocidin 1 (cationic antimicrobial protein 37)); B120 (Brain protein 120); B2M (Beta-2-microglobulin); BAG1 (BCL2-associated athanogene); BAK1 (BCL2-antagonist/killer 1); BAX (BCL2-associated X protein); BCHE (Butyrylcholinesterase); BCKDHA (Branched chain keto acid dehydrogenase E1, alpha polypeptide (maple syrup urine disease)); BCL1 (B-cell CLL/lymphoma 1); BCL2 (B-cell CLL/lymphoma 2); BCL2A1 (BCL2-related protein A1); BCL2L1; BCL2L2 (BCL2-like 2); BCL7 (B-cell CLL/lymphoma 7); BCPM (Benign chronic pemphigus (Hailey-Hailey disease)); BCPR (Breast cancer-related regulator of TP53); BCR (Breakpoint cluster region); BDKRB1 (Bradykinin receptor B1); BDKRB2 (Bradykinin receptor B2); BENE (BENE protein); BF (B-factor, properdin); BGLAP (Bone gamma-carboxyglutamate (gla) protein (osteocalcin)); BGP (Biliary glycoprotein); BHR1 (Bronchial hyperresponsiveness-1 (bronchial asthma)); BID (BH3 interacting domain death agonist); BLK (B lymphoid tyrosine kinase); BLM (Bloom syndrome); BLMH (Bleomycin hydrolase); BMI1 (Murine leukemia viral (bmi-1) oncogene homolog); BN51T (BN51 (BHK21) temperature sensitivity complementing); BPAG1 (Bullous pemphigoid antigen 1 (230/240kD)); BPI (Bactericidal/permeability-increasing protein); BRAF (V-raf murine sarcoma viral oncogene homolog B1); BRAK (CXC chemokine in breast and kidney); BRCA1 (Breast cancer 1, early onset); BRCA2 (Breast cancer 2, early onset); BSEP (Bile salt export pump (ABC member 16, MDR/TAP subfamily)); BST1 (Bone marrow stromal cell antigen 1); BTD (Biotinidase); BTK (Bruton agammaglobulinemia tyrosine kinase); BUB1 (Budding uninhibited by benzimidazoles 1 (yeast homolog)); C1NH (Complement component 1 inhibitor (angioedema, hereditary)); C1QA (Complement component 1, q subcomponent, alpha polypeptide); C1QB (Complement component 1, q subcomponent, beta Polypeptide); C1QBP (Complement component 1, q subcomponent binding protein); C1QG (Complement component 1, q subcomponent, gamma polypeptide); C1R (Complement component 1, r subcomponent); C1S (Complement component 1, s subcomponent); C2 (Complement component 2); C3 (Complement component 3); C3AR1 (Complement component 3a receptor 1); C4A (Complement component 4A); C4B (Complement component 4B); C4BPA (Complement component 4-binding protein, alpha); C4BPAL2 (Complement component 4-binding protein, alpha-like 2); C4BPB (Complement component 4-binding protein, beta); C5 (Complement component 5); C5R1 (Complement component 5 receptor 1 (C5a ligand)); C6 (Complement component 6); C7 (Complement component 7); C8A (Complement component 8, alpha polypeptide); C8B (Complement component 8, beta polypeptide); C8G (Complement component 8, gamma polypeptide); C9 (Complement component 9); CA1 (Carbonic anhydrase I); CA2 (Carbonic anhydrase II); CACNA1S (Calcium channel, voltage-dependent, L type, alpha 1S subunit); CACNB2 (Calcium channel, voltage-dependent, beta 2 subunit); CALCA (Calcitonin/calcitonin-related polypeptide, alpha); CALCR (Calcitonin receptor); CAMP (Cathelicidin antimicrobial peptide); CAPG (Capping protein (actin filament), gelsolin-like); CAPN3 (Calpain, large polypeptide L3); CASP1 (Caspase 1, apoptosis-related cysteine protease (interleukin 1, beta, convertase)); CASP10 (Caspase 10, apoptosis-related cysteine protease); CASP2 (Caspase 2, apoptosis-related cysteine protease (neural precursor cell expressed, developmentally down-regulated 2)); CASP3 (Caspase 3, apoptosis-related cysteine protease); CASP4 (Caspase 4, apoptosis-related cysteine protease); CASP5 (Caspase 5, apoptosis-related cysteine protease); CASP6 (Caspase 6, apoptosis-related cysteine protease); CASP7 (Caspase 7, apoptosis-related cysteine protease); CASP8 (Caspase 8, apoptosis-related cysteine protease); CASP9 (Caspase 9, apoptosis-related cysteine protease); CASR (Calcium-sensing receptor (hypocalciuric hypercalcemia 1, severe neonatal hyperparathyroidism)); CAT (Catalase); CAV3 (Caveolin 3); CBBM (Blue-monochromatic colorblindness (blue cone monochromacy)); CBFA2 (Core-binding factor, runt domain, alpha subunit 2 (acute myeloid leukemia 1; aml1 oncogene)); CBFA2T1 (Core-binding factor, runt domain, alpha subunit 2; translocated to, 1; cyclin D-related); CBFA2T2 (Core-binding factor, runt domain, alpha subunit 2; translocated to, 2); CBFA2T3 (Core-binding factor, runt domain, alpha subunit 2; translocated to, 3); CBFB (Core-binding factor, beta subunit); CBLN1 (Cerebellin 1 precursor); CBR1 (Carbonyl reductase 1); CBS (Cystathionine-beta-synthase); CBX4 (Chromobox homolog 4 (Drosophila Pc class)); CCAL1 (Chondrocalcinosis 1 (calcium pyrophosphate-deposition disease, early onset osteoarthritis)); CCR1 (Chemokine (C-C motif) receptor 1); CCR1 (Chemokine (C-C motif) receptor 1); CCR2 (Chemokine (C-C motif) receptor 2); CCR3 (Chemokine (C-C motif) receptor 3); CCR3 (Chemokine (C-C motif) receptor 3); CCR4 (Chemokine (C-C motif) receptor 4); CCR5 (Chemokine (C-C motif) receptor 5); CCR5 (Chemokine (C-C motif) receptor 5); CCR6 (Chemokine (C-C motif) receptor 6); CCR7 (Chemokine (C-C motif) receptor 7); CCR7 (Chemokine (C-C motif) receptor 7); CCR8 (Chemokine (C-C motif) receptor 8); CD 14 (CD 14 antigen); CD 151 (CD151 antigen); CD 19 (CD 19 antigen); CD1A (CD1A antigen, a polypeptide); CD1B (CD1B antigen, b polypeptide); CD2 (CD2 antigen (p50), sheep red blood cell receptor); CD20 (CD20 antigen); CD22 (CD22 antigen); CD36 (CD36 antigen (collagen type I receptor, thrombospondin receptor)); CD36L1 (CD36 antigen (collagen type I receptor, thrombospondin receptor)-like 1); CD39 (CD39 antigen); CD3E (CD3E antigen, epsilon polypeptide (TiT3 complex)); CD3G (CD3G antigen, gamma polypeptide (TiT3 complex)); CD3Z (CD3Z antigen, zeta polypeptide (TiT3 complex)); CD4 (CD4 antigen (p55)); CD44 (CD44 antigen (homing function and Indian blood group system)); CD47 (CD47 antigen (Rh-related antigen, integrin-associated signal transducer)); CD5 (CD5 antigen (p56-62)); CD53 (CD53 antigen); CD58 (CD58 antigen, (lymphocyte function-associated antigen 3)); CD59 (CD59 antigen p18-20 (antigen identified by monoclonal antibodies 16.3A5, EJ16, EJ30, EL32 and G344)); CD5L (CD5 antigen-like (scavenger receptor cysteine rich family)); CD6 (CD6 antigen); CD63 (CD63 antigen (melanoma 1 antigen)); CD68 (CD68 antigen); CD69 (CD69 antigen (p60, early T-cell activation antigen)); CD7 (CD7 antigen (p41)); CD74 (CD74 antigen (invariant polypeptide of major histocompatibility complex, class II antigen-associated)); CD79A (CD79A antigen (immunoglobulin-associated alpha)); CD79B (CD79B antigen (immunoglobulin-associated beta)); CD80 (CD80 antigen (CD28 antigen ligand 1, B7-1 antigen)); CD81 (CD81 antigen (target of antiproliferative antibody 1)); CD86 (CD86 antigen (CD28 antigen ligand 2, B7-2 antigen)); CD8A (CD8 antigen, alpha polypeptide (p32)); CDA (Cytidine deaminase); CDAN1 (Congenital dyserythropoietic anemia, type I); CDAN2 (Congenital dyserythropoietic anemia, type II); CDC2L1 (Cell division cycle 2-like 1 (PITSLRE proteins)); CDH12 (Cadherin 12 (N-cadherin 2)); CDK2 (Cyclin-dependent kinase 2); CDK4 (Cyclin-dependent kinase 4); CDKN1A (Cyclin-dependent kinase inhibitor 1A (p21, Cip1)); CDKN1B (Cyclin-dependent kinase inhibitor 1B (p27, Kip 1)); CDKN1C (Cyclin-dependent kinase inhibitor 1C (p57, Kip2)); CDKN2A (Cyclin-dependent kinase inhibitor 2A (melanoma, p16, inhibits CDK4)); CDKN2C (Cyclin-dependent kinase inhibitor 2C (p18, inhibits CDK4)); CDR1 (Cerebellar degeneration-related protein (34kD)); CDR2 (Cerebellar degeneration-related protein (62kD)); CDSN (Corneodesmosin); CDW52 (CDW52 antigen (CAMPATH-1 antigen)); CEBPB (CCAAT/enhancer binding protein (C/EBP), beta); CEBPE (CCAAT/enhancer binding protein (C/EBP), epsilon); CEL (Carboxyl ester lipase (bile salt-stimulated lipase)); CELL (Carboxyl ester lipase-like (bile salt-stimulated lipase-like)); CENPB (Centromere protein B (80kD)); CENPC1 (Centromere protein C 1); CES1 (Carboxylesterase 1 (monocyte/macrophage serine esterase 1)); CETP (Cholesteryl ester transfer protein, plasma); CFTR (Cystic fibrosis transmembrane conductance regulator); CGA (Glycoprotein hormones, alpha polypeptide); CHC1 (Chromosome condensation 1); CHE2 (Cholinesterase (serum) 2); CHGA (Chromogranin A (parathyroid secretory protein 1)); CHH (Cartilage-hair hypoplasia); CHIT1 (Chitinase 1); CHM (Choroideremia (Rab escort protein 1)); CHML (Choroideremia-like (Rab escort protein 2)); CHRM4 (Cholinergic receptor, muscarinic 4); CHRNA1 (Cholinergic receptor, nicotinic, alpha polypeptide 1 (muscle)); CHRNA7 (Cholinergic receptor, nicotinic, alpha polypeptide 7); CHS1 (Chediak-Higashi syndrome 1); CISH (Cytokine inducible SH2-containing protein); CKB (Creatine kinase, brain); CKM (Creatine kinase, muscle); CKN1 (Cockayne syndrome 1 (classical)); CLA1 (Cerebellar ataxia 1 (autosomal recessive)); CLCN1 (Chloride channel 1 , skeletal muscle); CLCN4 (Chloride channel 4); CLCNKB (Chloride channel Kb); CLDN3 (Claudin 3); CLN3 (Ceroid-lipofuscinosis, neuronal 3, juvenile (Batten, Spielmeyer-Vogt disease)); CLTA (Clathrin, light polypeptide (Lca)); CLU (Clusterin (complement lysis inhibitor, SP-40,40, sulfated glycoprotein 2, testosterone-repressed prostate message 2, apolipoprotein J)); CMA1 (Chymase 1, mast cell); CMAR (Cell matrix adhesion regulator); CMD1A (Cardiomyopathy, dilated 1A (autosomal dominant)); CMKBR9 (Chemokine (C-C motif) receptor 9); CMKBR9 (Chemokine (C-C motif) receptor 9); CMKLR1 (Chemokine-like receptor 1); CNC (Carney complex, multiple neoplasia and lentiginosis); CNGA1 (Cyclic nucleotide gated channel alpha 1); CNK (Cytokine-inducible kinase); CNP (2',3'-cyclic nucleotide 3' phosphodiesterase); CNTF (Ciliary neurotrophic factor); CNTFR (Ciliary neurotrophic factor receptor); COIL (Coilin p80); COL11A2 (Collagen, type XI, alpha 2); COL17A1 (Collagen, type XVII, alpha 1); COL1A1 (Collagen, type I, alpha 1); COL1A2 (Collagen, type I, alpha 2); COL2A1 (Collagen, type II, alpha 1 (primary osteoarthritis, spondyloepiphyseal dysplasia, congenital)); COL3A1 (Collagen, type III, alpha 1 (Ehlers-Danlos syndrome type IV, autosomal dominant)); COL4A1 (Collagen, type IV, alpha 1); COL4A3 (Collagen, type IV, alpha 3 (Goodpasture antigen)); COL5A1 (Collagen, type V, alpha 1); COL7A1 (Collagen, type VII, alpha 1 (epidermolysis bullosa, dystrophic, dominant and recessive)); COL9A2 (Collagen, type IX, alpha 2); COMT (Catechol-O-methyltransferase); COX4 (Cytochrome c oxidase subunit IV); CP (Ceruloplasmin (ferroxidase)); CP20 (Lymphocyte cytosolic protein, molecular weight 20kD); CPE (Carboxypeptidase E); CPM (Carboxypeptidase M); CPO (Coproporphyrinogen oxidase (coproporphyria, harderoporphyria)); CPS1 (Carbamoyl-phosphate synthetase 1, mitochondrial); CR1 (Complement component (3b/4b) receptor 1, including Knops blood group system); CR1L (Complement component (3b/4b) receptor 1-like); CR2 (Complement component (3d/Epstein Barr virus) receptor 2); CREB1 (CAMP responsive element binding protein 1); CREBL1 (CAMP responsive element binding protein-like 1); CREM (CAMP responsive element modulator); CRP (C-reactive protein, pentraxin-related); CRS1C (Cryptidin-related sequence-1C); CRX (Cone-rod homeobox); CRYAB (Crystallin, alpha B); CSBP1 (Cytokine suppressive antiinflammatory drug binding protein 1 (p38 MAP kinase)); CSE1L (Chromosome segregation 1 (yeast homolog)-like); CSF1 (Colony stimulating factor 1 (macrophage)); CSF1 (Colony stimulating factor 1 (macrophage)); CSF1R (Colony stimulating factor 1 receptor, formerly McDonough feline sarcoma viral (v-fms) oncogene homolog); CSF2 (Colony stimulating factor 2 (granulocyte-macrophage)); CSF2RA (Colony stimulating factor 2 receptor, alpha, low-affinity (granulocyte-macrophage)); CSF2RB (Colony stimulating factor 2 receptor, beta, low-affinity (granulocyte-macrophage)); CSF2RY (Granulocyte-macrophage colony-stimulating factor receptor, alpha); CSF3 (Colony stimulating factor 3 (granulocyte)); CSF3R (Colony stimulating factor 3 receptor (granulocyte)); CSF3R (Colony stimulating factor 3 receptor (granulocyte)); CSH1 (Chorionic somatomammotropin hormone 1 (placental lactogen)); CSMF (Chondrosarcoma, extraskeletal myxoid, fused to EWS in); CSN1 (Casein, alpha); CSN2 (Casein, beta); CSNU3 (Cystinuria type 3); CSPG2 (Chondroitin sulfate proteoglycan 2 (versican)); CSPG3 (Chondroitin sulfate proteoglycan 3 (neurocan)); CSRP1 (Cysteine and glycine-rich protein 1); CSRP2 (Cysteine and glycine-rich protein 2 (LIM domain only, smooth muscle)); CST3 (Cystatin C (amyloid angiopathy and cerebral hemorrhage)); CSTB (Cystatin B (stefin B)); CTF1 (Cardiotrophin 1); CTGF (Connective tissue growth factor); CTLA4 (Cytotoxic T-lymphocyte-associated protein 4); CTNS (Cystinosis, nephropathic); CTSB (Cathepsin B); CTSC (Cathepsin C); CTSF (Cathepsin F); CTSG (Cathepsin G); CTSK (Cathepsin K (pycnodysostosis)); CTSL (Cathepsin L); CTSS (Cathepsin S); CUBN (Cubilin (intrinsic factor-cobalamin receptor)); CX3CR1 (Chemokine (C-X3-C) receptor 1); CXCR4 (Chemokine (C-X-C motif), receptor 4 (fusin)); CYBA (Cytochrome b-245, alpha polypeptide); CYBB (Cytochrome b-245, beta polypeptide (chronic granulomatous disease)); CYP11A (Cytochrome P450, subfamily XIA (cholesterol side chain cleavage)); CYP11B2 (Cytochrome P450, subfamily XIB (steroid 11-beta-hydroxylase), polypeptide 2); CYP17 (Cytochrome P450, subfamily XVII (steroid 17-alpha-hydroxylase), adrenal hyperplasia); CYP19 (Cytochrome P450, subfamily XIX (aromatization of androgens)); CYP1A2 (Cytochrome P450, subfamily I (aromatic compound-inducible), polypeptide 2); CYP1B1 (Cytochrome P450, subfamily I (dioxin-inducible), polypeptide 1 (glaucoma 3, primary infantile)); CYP21 (Cytochrome P450, subfamily XXI (steroid 21-hydroxylase, congenital adrenal hyperplasia)); CYP27A1 (Cytochrome P450, subfamily XXVIIA (steroid 27-hydroxylase, cerebrotendinous xanthomatosis), polypeptide 1); CYP2A (Cytochrome P450, subfamily IIA (phenobarbital-inducible)); CYP2A6 (Cytochrome P450, subfamily IIA (phenobarbital-inducible), polypeptide 6); CYP2C9 (Cytochrome P450, subfamily IIC (mephenytoin 4-hydroxylase), polypeptide 9); CYP2D@ (Cytochrome P450, subfamily IID (debrisoquine, sparteine, etc., -metabolizing) cluster); CYP2E (Cytochrome P450, subfamily IIE (ethanol-inducible)); CYP7A1 (Cytochrome P450, subfamily VIIA (cholesterol 7 alpha-monooxygenase), polypeptide 1); D2S69E (T-lymphocyte activation gene 519); D6S207E (Minor histocompatibility antigen HA-2); D6S2244E (Ke4 gene, mouse, human homolog of); D6S231E (DEK gene); D6S51E (HLA-B associated transcript-2); D6S52E (HLA-B associated transcript-3); D6S54E (HLA-B associated transcript-4); D6S81E (HLA-B associated transcript-1); D6S82E (HLA-B associated transcript-5); DAD1 (Defender against cell death 1); DAF (Decay accelerating factor for complement (CD55, Cromer blood group system)); DAG1 (Dystroglycan I (dystrophin-associated glycoprotein 1)); DAO (D-amino-acid oxidase); DAP (Death-associated protein); DAPK1 (Death-associated protein kinase 1); DAPK3 (Death-associated protein kinase 3); DBH (Dopamine beta-hydroxylase (dopamine beta-monooxygenase)); DCC (Deleted in colorectal carcinoma); DCP1 (Dipeptidyl carboxypeptidase 1 (angiotensin I converting enzyme)); DCX (Doublecortex; lissencephaly, X-linked (doublecortin)); DDX10 (DEAD/H (Asp-Glu-Ala-Asp/His) box polypeptide 10 (RNA helicase)); DDX11 (DEAD/H (Asp-Glu-Ala-Asp/His) box polypeptide 11 (S.cerevisiae CHL1-like helicase) ); DEFA1 (Defensin, alpha 1, myeloid-related sequence); DEFA4 (Defensin, alpha 4, corticostatin); DEFA5 (Defensin, alpha 5, Paneth cell-specific); DEFA6 (Defensin, alpha 6, Paneth cell-specific); DEFB1 (Defensin, beta 1); DF (D component of complement (adipsin)); DFFA (DNA fragmentation factor, 45 kD, alpha subunit); DFFB (DNA fragmentation factor, 40 kD, beta subunit); DGCR (DiGeorge syndrome chromosome region); DGI1 (Dentinogenesis imperfecta 1); DHFR (Dihydrofolate reductase); DI (Diego blood group); DIA1 (Diaphorase (NADH) (cytochrome b-5 reductase)); DM (Dystrophia myotonica (includes dystrophia myotonia protein kinase)); DMD (Dystrophin (muscular dystrophy, Duchenne and Becker types), includes DXS142, DXS164, DXS206, DXS230, DXS239, DXS268, DXS269, DXS270, DXS272); DMP1 (Dentin matrix acidic phosphoprotein); DNASE1 (Deoxyribonuclease I); DNASE1L1 (Deoxyribonuclease I-like 1); DNASE1L2 (Deoxyribonuclease I-like 2); DNASE1L3 (Deoxyribonuclease I-like 3); DNASE2 (Deoxyribonuclease II, lysosomal); DNTT (Deoxynucleotidyltransferase, terminal); DOCK1 (Dedicator of cyto-kinesis 1); DPP4 (Dipeptidylpeptidase IV (CD26, adenosine deaminase complexing protein 2) ); DPYD (Dihydropyrimidine dehydrogenase); DRA (Down-regulated in adenoma); DRD2 (Dopamine receptor D2); DRD3 (Dopamine receptor D3); DRD4 (Dopamine receptor D4); DSG1 (Desmoglein 1); DSG3 (Desmoglein 3 (pemphigus vulgaris antigen)); DSP (Desmoplakin (DPI, DPII)); DTD (Diastrophic dysplasia); DTNA (Dystrobrevin, alpha); DTR (Diphtheria toxin receptor (heparin-binding epidermal growth factor-like growth factor)); DXS423E (Segregation of mitotic chromosomes 1 (SMC1, yeast human homolog of;); DXS435E (A-11 gene); DYS (Dysautonomia (Riley-Day syndrome, hereditary sensory autonomic neuropathy type III)); DYX2 (Dyslexia 2); E2F1 (E2F transcription factor 1); EBF (Early B-cell factor); ECB2); ECGF1 (Endothelial cell growth factor 1 (platelet-derived)); ED1 (Ectodermal dysplasia 1, anhidrotic); EDG1 (Endothelial differentiation, sphingolipid G-protein-coupled receptor, 1); EDN1 (Endothelin 1); EEC2 (Ectrodactyly, ectodermal dysplasia and cleft lip/palate syndrome 2); EGR1 (Early growth response 1); EGR2 (Early growth response 2 (Krox-20 (Drosophila) homolog)); EIF3S6 (Eukaryotic translation initiation factor 3, subunit 6 (48kD)); EIF4G1 (Eukaryotic translation initiation factor 4 gamma, 1); EIF4G2 (Eukaryotic translation initiation factor 4 gamma, 2); EIF5A (Eukaryotic translation initiation factor 5A); EJM1 (Epilepsy, juvenile myoclonic 1); ELA2 (Elastase 2, neutrophil); ELANH2 (Protease inhibitor 2 (anti-elastase), monocyte/neutrophil); ELAVL2 (ELAV (embryonic lethal, abnormal vision, Drosophila)-like 2); ELAVL4 (ELAV (embryonic lethal, abnormal vision, Drosophila)-like 4 (Hu antigen D)); ELN (Elastin (supravalvular aortic stenosis, Williams-Beuren syndrome)); ENG (Endoglin (Osler-Rendu-Weber syndrome 1)); ENPEP (Glutamyl aminopeptidase (aminopeptidase A)); EPB41 (Erythrocyte membrane protein band 4.1 (elliptocytosis 1, RH-linked)); EPB42 (Erythrocyte membrane protein band 4.2); EPHA3 (EphA3); EPHX1 (Epoxide hydrolase 1, microsomal (xenobiotic)); EPHX2 (Epoxide hydrolase 2, cytoplasmic); EPO (Erythropoietin); EPOR (Erythropoietin receptor); EPOR (Erythropoietin receptor); EPOR (Erythropoietin receptor); EPS 15 (Epidermal growth factor receptor pathway substrate 15); EPT (Epilepsy, partial); ERBB2 (V-erb-b2 avian erythroblastic leukemia viral oncogene homolog 2 (neuro/glioblastoma derived oncogene homolog)); ERCC1 (Excision repair cross-complementing rodent repair deficiency, complementation group 1 (includes overlapping antisense sequence)); ERCC2 (Excision repair cross-complementing rodent repair deficiency, complementation group 2 (xeroderma pigmentosum D)); ERCC3 (Excision repair cross-complementing rodent repair deficiency, complementation group 3 (xeroderma pigmentosum group B complementing)); ERCC4 (Excision repair cross-complementing rodent repair deficiency, complementation group 4); ERCC5 (Excision repair cross-complementing rodent repair deficiency, complementation group 5 (xeroderma pigmentosum, complementation group G (Cockayne syndrome))); ES1 (ES1 (zebrafish) protein, human homolog of); ESB3 (Esterase B3); ESD (Esterase D/formylglutathione hydrolase); ESR1 (Estrogen receptor 1); ESR2 (Estrogen receptor 2 (ER beta)); ETV4 (Ets variant gene 4 (E1A enhancer-binding protein, E1AF)); ETV6 (Ets variant gene 6 (TEL oncogene)); EYCL3 (Eye color 3 (brown)); F10 (Coagulation factor X); F11 (Coagulation factor XI (plasma thromboplastin antecedent)); F12 (Coagulation factor XII (Hageman factor)); F13A1 (Coagulation factor XIII, A1 polypeptide); F13B (Coagulation factor XIII, B polypeptide); F2 (Coagulation factor II (thrombin)); F2R (Coagulation factor II (thrombin) receptor); F2RL2 (Coagulation factor II (thrombin) receptor-like 2); F3 (Coagulation factor III (thromboplastin, tissue factor)); F5 (Coagulation factor V (proaccelerin, labile factor)); F7 (Coagulation factor VII (serum prothrombin conversion accelerator)); F7R (Coagulation factor VII regulator); F8A (Factor VIII associated gene); F8C (Coagulation factor VIIIc, procoagulant component (hemophilia A)); F9 (Coagulation factor IX (plasma thromboplastic component, Christmas disease, hemophilia B)); FABP1 (Fatty acid binding protein 1, liver); FABP2 (Fatty acid binding protein 2, intestinal); FABP6 (Fatty acid binding protein 6, ileal (gastrotropin)); FADD (Fas (TNFRSF6)-associated via death domain); FAH (Fumarylacetoacetate); FANCA (Fanconi anemia, complementation group A); FANCB (Fanconi anemia, complementation group B); FANCC (Fanconi anemia, complementation group C); FANCE (Fanconi anemia, complementation group E); FANCG (Fanconi anemia, complementation group G); FAT (FAT tumor suppressor (Drosophila) homolog); FBLN1 (Fibulin 1); FBN1 (Fibrillin 1 (Marfan syndrome)); FBP1 (Fructose-bisphosphatase 1); FCAR (Fc fragment of IgA, receptor for); FCER1A (Fc fragment of IgE, high affinity I, receptor for; alpha polypeptide); FCER1B (Fc fragment of IgE, high affinity I, receptor for; beta polypeptide); FCER2 (Fc fragment of IgE, low affinity II, receptor for (CD23A)); FCGR1A (Fc fragment of IgG, high affinity Ia, receptor for (CD64)); FCGR1B (Fc fragment of IgG, high affinity Ib, receptor for (CD64)); FCGR1B (Fc fragment of IgG, high affinity Ib, receptor for (CD64)); FCGR2A (Fc fragment of IgG, low affinity IIa, receptor for (CD32)); FCGR2B (Fc fragment of IgG, low affinity IIb, receptor for (CD32)); FCGR2C (Fc fragment of IgG, low affinity IIb, receptor for (CD32)); FCGR3A (Fc fragment of IgG, low affinity IIIa, receptor for (CD16)); FCGR3B (Fc fragment of IgG, low affinity IIIb, receptor for (CD 16)); FCN1 (Ficolin (collagen/fibrinogen domain-containing) 1); FDH (Formaldehyde dehydrogenase); FEA (F9 embryonic antigen); FEB1 (Febrile convulsions 1febrile convulsions 1); FECH (Ferrochelatase (protoporphyria)); FES (Feline sarcoma (Snyder-Theilen) viral (v-fes)/Fujinami avian sarcoma (PRCII) viral (v-fps) oncogene homolog); FGA (Fibrinogen, A alpha polypeptide); FGB (Fibrinogen, B beta polypeptide); FGF1 (Fibroblast growth factor 1 (acidic)); FGF9 (Fibroblast growth factor 9 (glia-activating factor)); FGFR1 (Fibroblast growth factor receptor 1 (fms-related tyrosine kinase 2, Pfeiffer syndrome)); FGFR3 (Fibroblast growth factor receptor 3 (achondroplasia, thanatophoric dwarfism)); FGG (Fibrinogen, gamma polypeptide); FGR (Gardner-Rasheed feline sarcoma viral (v-fgr) oncogene homolog); FHR2 (Factor H-related gene 2); FKHR (Forkhead (Drosophila) homolog 1 (rhabdomyosarcoma)); FLG (Filaggrin); FLNB (Filamin B, beta (actin-binding protein-278)); FLT3 (Fms-related tyrosine kinase 3); FLT3LG (Fms-related tyrosine kinase 3 ligand); FMO1 (Flavin containing monooxygenase 1); FMO3 (Flavin containing monooxygenase 3); FN1 (Fibronectin 1); FOLR1 (Folate receptor 1 (adult)); FOS (V-fos FBJ murine osteosarcoma viral oncogene homolog); FOSB (FBJ murine osteosarcoma viral oncogene homolog B); FPDMM); FPR1 (Formyl peptide receptor 1); FPRL1 (Formyl peptide receptor-like 1); FR (Froese blood group); FRAP1 (FRK506 binding protein 12-rapamycin associated protein 1); FRDA (Friedreich ataxia); FRG1 (FSHD region gene 1); FSHMD1A (Facioscapulohumeral muscular dystrophy 1A); FTL (Ferritin, light polypeptide); FTNB (Fertilin beta (a disintegrin and metalloproteinase domain 2)); FUCA1 (Fucosidase, alpha-L- 1, tissue); FUCA2 (Fucosidase, alpha-L- 2, plasma); FUT2 (Fucosyltransferase 2 (secretor status included)); FUT3 (Fucosyltransferase 3 (galactoside 3(4)-L-fucosyltransferase, Lewis blood group included)); FXR1 (Fragile X mental retardation, autosomal homolog); DAD1 (Defender against cell death 1); DAF (Decay accelerating factor for complement (CD55, Cromer blood group system)); DAG1 (Dystroglycan 1 (dystrophin-associated glycoprotein 1)); DAO (D-amino-acid oxidase); DAP (Death-associated protein); DAPK1 (Death-associated protein kinase 1); DAPK3 (Death-associated protein kinase 3); DBH (Dopamine beta-hydroxylase (dopamine beta-monooxygenase)); DCC (Deleted in colorectal carcinoma); DCP1 (Dipeptidyl carboxypeptidase 1 (angiotensin I converting enzyme)); DCX (Doublecortex; lissencephaly, X-linked (doublecortin)); DDX10 (DEAD/H (Asp-Glu-Ala-Asp/His) box polypeptide 10 (RNA helicase)); DDX11 (DEAD/H (Asp-Glu-Ala-Asp/His) box polypeptide 11 (S.cerevisiae CHL1-like helicase)); DEFA1 (Defensin, alpha 1, myeloid-related sequence); DEFA4 (Defensin, alpha 4, corticostatin); DEFA5 (Defensin, alpha 5, Paneth cell-specific); DEFA6 (Defensin, alpha 6, Paneth cell-specific); DEFB1 (Defensin, beta 1); DF (D component of complement (adipsin)); DFFA (DNA fragmentation factor, 45 kD, alpha subunit); DFFB (DNA fragmentation factor, 40 kD, beta subunit); DGCR (DiGeorge syndrome chromosome region); DGI1 (Dentinogenesis imperfecta 1); DHFR (Dihydrofolate reductase); DI (Diego blood group); DIA1 (Diaphorase (NADH) (cytochrome b-5 reductase)); DM (Dystrophia myotonica (includes dystrophia myotonia protein kinase)); DMD (Dystrophin (muscular dystrophy, Duchenne and Becker types), includes DXS142, DXS164, DXS206, DXS230, DXS239, DXS268, DXS269, DXS270, DXS272); DMP1 (Dentin matrix acidic phosphoprotein); DNASE1 (Deoxyribonuclease I); DNASE1L1 (Deoxyribonuclease I-like 1); DNASE1L2 (Deoxyribonuclease I-like 2); DNASE1L3 (Deoxyribonuclease I-like 3); DNASE2 (Deoxyribonuclease II, lysosomal); DNTT (Deoxynucleotidyltransferase, terminal); DOCK1 (Dedicator of cyto-kinesis 1); DPP4 (Dipeptidylpeptidase IV (CD26, adenosine deaminase complexing protein 2)); DPYD (Dihydropyrimidine dehydrogenase); DRA (Down-regulated in adenoma); DRD2 (Dopamine receptor D2); DRD3 (Dopamine receptor D3); DRD4 (Dopamine receptor D4); DSG1 (Desmoglein 1); DSG3 (Desmoglein 3 (pemphigus vulgaris antigen)); DSP (Desmoplakin (DPI, DPII)); DTD (Diastrophic dysplasia); DTNA (Dystrobrevin, alpha); DTR (Diphtheria toxin receptor (heparin-binding epidermal growth factor-like growth factor)); DXS423E (Segregation of mitotic chromosomes 1 (SMC1, yeast human homolog of;); DXS435E (A-11 gene); DYS (Dysautonomia (Riley-Day syndrome, hereditary sensory autonomic neuropathy type III)); DYX2 (Dyslexia 2); E2F1 (E2F transcription factor 1); EBF (Early B-cell factor ECB2); ECGF1 Endothelial cell growth factor 1 (platelet-derived)); ED1 (Ectodermal dysplasia 1, anhidrotic); EDG1 (Endothelial differentiation, sphingolipid); G-protein-coupled receptor, 1); EDN1 (Endothelin 1); EEC2 (Ectrodactyly, ectodermal dysplasia and cleft lip/palate syndrome 2); EGR1 (Early growth response 1); EGR2 (Early growth response 2 (Krox-20 (Drosophila) homolog)); EIF3S6 (Eukaryotic translation initiation factor 3, subunit 6 (48kD)); EIF4G1 (Eukaryotic translation initiation factor 4 gamma, 1); EIF4G2 (Eukaryotic translation initiation factor 4 gamma, 2); EIF5A (Eukaryotic translation initiation factor 5A); EJM1 (Epilepsy, juvenile myoclonic 1); ELA2 (Elastase 2, neutrophil); ELANH2 (Protease inhibitor 2 (anti-elastase), monocyte/neutrophil); ELAVL2 (ELAV (embryonic lethal, abnormal vision, Drosophila)-like 2); ELAVL4 (ELAV (embryonic lethal, abnormal vision, Drosophila)-like 4 (Hu antigen D)); ELN (Elastin (supravalvular aortic stenosis, Williams-Beuren syndrome) ); ENG (Endoglin (Osler-Rendu-Weber syndrome 1)); ENPEP (Glutamyl aminopeptidase (aminopeptidase A)); EPB41 (Erythrocyte membrane protein band 4.1 (elliptocytosis 1, RH-linked)); EPB42 (Erythrocyte membrane protein band 4.2); EPHA3 (EphA3); EPHX1 (Epoxide hydrolase 1, microsomal (xenobiotic)); EPHX2 (Epoxide hydrolase 2, cytoplasmic); EPO (Erythropoietin); EPOR (Erythropoietin receptor); EPOR (Erythropoietin receptor); EPOR (Erythropoietin receptor); EPS 15 (Epidermal growth factor receptor pathway substrate 15); EPT (Epilepsy, partial); ERBB2 (V-erb-b2 avian erythroblastic leukemia viral oncogene homolog 2 (neuro/glioblastoma derived oncogene homolog)); ERCC1 (Excision repair cross-complementing rodent repair deficiency, complementation group 1 (includes overlapping antisense sequence)); ERCC2 (Excision repair cross-complementing rodent repair deficiency, complementation group 2 (xeroderma pigmentosum D)); ERCC3 (Excision repair cross-complementing rodent repair deficiency, complementation group 3 (xeroderma pigmentosum group B complementing)); ERCC4 (Excision repair cross-complementing rodent repair deficiency, complementation group 4); ERCC5 (Excision repair cross-complementing rodent repair deficiency, complementation group 5 (xeroderma pigmentosum, complementation group G (Cockayne syndrome))); ES1 (ES1 (zebrafish) protein, human homolog of); ESB3 (Esterase B3); ESD (Esterase D/formylglutathione hydrolase); ESR1 (Estrogen receptor 1); ESR2 (Estrogen receptor 2 (ER beta)); ETV4 (Ets variant gene 4 (E1A enhancer-binding protein, E1AF) ); ETV6 (Ets variant gene 6 (TEL oncogene)); EYCL3 (Eye color 3 (brown)); F10 (Coagulation factor X); F11 (Coagulation factor XI (plasma thromboplastin antecedent)); F12 (Coagulation factor XII (Hageman factor)); F13A1 (Coagulation factor XIII, A1 polypeptide); F13B (Coagulation factor XIII, B polypeptide); F2 (Coagulation factor II (thrombin)); F2R (Coagulation factor II (thrombin) receptor); F2RL2 (Coagulation factor II (thrombin) receptor-like 2); F3 (Coagulation factor III (thromboplastin, tissue factor)); F5 (Coagulation factor V (proaccelerin, labile factor)); F7 (Coagulation factor VII (serum prothrombin conversion accelerator)); F7R (Coagulation factor VII regulator); F8A (Factor VIII associated gene); F8C (Coagulation factor VIIIc, procoagulant component (hemophilia A)); F9 (Coagulation factor IX (plasma thromboplastic component, Christmas disease, hemophilia B)); FABP1 (Fatty acid binding protein 1, liver); FABP2 (Fatty acid binding protein 2, intestinal); FABP6 (Fatty acid binding protein 6, ileal (gastrotropin)); FADD (Fas (TNFRSF6)-associated via death domain); FAH (Fumarylacetoacetate); FANCA (Fanconi anemia, complementation group A); FANCB (Fanconi anemia, complementation group B); FANCC (Fanconi anemia, complementation group C); FANCE (Fanconi anemia, complementation group E); FANCG (Fanconi anemia, complementation group G); FAT (FAT tumor suppressor (Drosophila) homolog); FBLN1 (Fibulin 1); FBN1 (Fibrillin 1 (Marfan syndrome)); FBP1 (Fructose-bisphosphatase 1); FCAR (Fc fragment of IgA, receptor for); FCER1A (Fc fragment of IgE, high affinity I, receptor for; alpha polypeptide); FCER1B (Fc fragment of IgE, high affinity I, receptor for; beta polypeptide); FCER2 (Fc fragment of IgE, low affinity II, receptor for (CD23A)); FCGR1A (Fc fragment of IgG, high affinity Ia, receptor for (CD64)); FCGR1B (Fc fragment of IgG, high affinity Ib, receptor for (CD64)); FCGR1B (Fc fragment of IgG, high affinity Ib, receptor for (CD64)); FCGR2A (Fc fragment of IgG, low affinity IIa, receptor for (CD32)); FCGR2B (Fc fragment of IgG, low affinity IIb, receptor for (CD32)); FCGR2C (Fc fragment of IgG, low affinity IIb, receptor for (CD32)); FCGR3A (Fc fragment of IgG, low affinity IIIa, receptor for (CD16)); FCGR3B (Fc fragment of IgG, low affinity IIIb, receptor for (CD16)); FCN1 (Ficolin (collagen/fibrinogen domain-containing) 1); FDH (Formaldehyde dehydrogenase); FEA (F9 embryonic antigen); FEB1 (Febrile convulsions 1febrile convulsions 1); FECH (Ferrochelatase (protoporphyria)); FES (Feline sarcoma (Snyder-Theilen) viral (v-fes)/Fujinami avian sarcoma (PRCII) viral (v-fps) oncogene homolog); FGA (Fibrinogen, A alpha polypeptide); FGB (Fibrinogen, B beta polypeptide); FGF1 (Fibroblast growth factor 1 (acidic)); FGF9 (Fibroblast growth factor 9 (glia-activating factor)); FGFR1 (Fibroblast growth factor receptor 1 (fms-related tyrosine kinase 2, Pfeiffer syndrome)); FGFR3 (Fibroblast growth factor receptor 3 (achondroplasia, thanatophoric dwarfism)); FGG (Fibrinogen, gamma polypeptide); FGR (Gardner-Rasheed feline sarcoma viral (v-fgr) oncogene homolog); FHR2 (Factor H-related gene 2); FKHR (Forkhead (Drosophila) homolog 1 (rhabdomyosarcoma)); FLG (Filaggrin); FLNB (Filamin B, beta (actin-binding protein-278)); FLT3 (Fms-related tyrosine kinase 3); FLT3LG (Fms-related tyrosine kinase 3 ligand); FMO1 (Flavin containing monooxygenase 1); FMO3 (Flavin containing monooxygenase 3); FN1 (Fibronectin 1); FOLR1 (Folate receptor 1 (adult)); FOS (V-fos FBJ murine osteosarcoma viral oncogene homolog); FOSB (FBJ murine osteosarcoma viral oncogene homolog B); FPDMM); FPR1 (Formyl peptide receptor 1); FPRL1 (Formyl peptide receptor-like 1); FR (Froese blood group); FRAP1 (FK506 binding protein 12-rapamycin associated protein); FRDA (Friedreich ataxia); FRG1); FSHD region gene 1); FSHMD1A (Facioscapulohumeral muscular dystrophy 1A); FTL (Ferritin, light polypeptide); FTNB (Fertilin beta (a disintegrin and metalloproteinase domain 2)); FUCA1 (Fucosidase, alpha-L- 1, tissue); FUCA2 (Fucosidase, alpha-L- 2, plasma); FUT2 (Fucosyltransferase 2 (secretor status included)); FUT3 (Fucosyltransferase 3 (galactoside); 3(4)-L-fucosyltransferase, Lewis blood group included)); FXR1 (Fragile X mental retardation, autosomal homolog); FY (Duffy blood group); FYB (FYN-binding protein (FYB-120/130)); G22P1 (Thyroid autoantigen 70kD (Ku antigen)); G6PD (Glucose-6-phosphate dehydrogenase); GAA (Glucosidase, alpha; acid (Pompe disease, glycogen storage disease type II)); GABRA5 (Gamma-aminobutyric acid (GABA) A receptor, alpha 5); GABRA6 (Gamma-aminobutyric acid (GABA) A receptor, alpha 6); GABRB1 (Gamma-aminobutyric acid (GABA) A receptor, beta 1); GAD2 (Glutamate decarboxylase 2 (pancreatic islets and brain, 65kD)); GALC (Galactosylceramidase (Krabbe disease)); GALK1 (Galactokinase 1); GALT (Galactose-1-phosphate uridylyltransferase); GANC (Glucosidase, alpha; neutral C); GAS (Gastrin); GAS6 (Growth arrest-specific 6); GATA3 (GATA-binding protein 3); GBA (Glucosidase, beta; acid (includes glucosyl-ceramidase)); GC (Group-specific component (vitamin D binding protein)); GCDH (Glutaryl-Coenzyme A dehydrogenase); GCGR (Glucagon receptor); GCK (Glucokinase (hexokinase 4, maturity onset diabetes of the young 2)); GCP (Green cone pigment (color blindness, deutan)); GDH (Glucose dehydrogenase); GEM (GTP-binding protein overexpressed in skeletal muscle); GFI1 (Growth factor independent 1); GGCX (Gamma-glutamyl carboxylase); GGT1 (Gamma-glutamyltransferase 1); GGTA1 (Glycoprotein, alpha-galactosyltransferase 1); GH1 (Growth hormone 1); GH2 (Growth hormone 2); GHR (Growth hormone receptor); GIF (Gastric intrinsic factor (vitamin B synthesis)); GLA (Galactosidase, alpha); GLC1B (Glaucoma 1, open angle, B (adult-onset)); GLCLC (Glutamate-cysteine ligase (gamma-glutamylcysteine synthetase), catalytic (72.8kD)); GLO1 (Glyoxalase I); GLP1R (Glucagon-like peptide 1 receptor); GLRB (Glycine receptor, beta); GLS (Glutaminase); GLUD1 (Glutamate dehydrogenase 1); GLYB (Glycine B complementing); GLYS1 (Glycosuria 1, renal); GNAQ (Guanine nucleotide binding protein (G protein), q polypeptide); GNAS1 (Guanine nucleotide binding protein (G protein), alpha stimulating activity polypeptide 1); GNB3 (Guanine nucleotide binding protein (G protein), beta polypeptide 3); GNL1 (Guanine nucleotide binding protein-like 1); GOT2 (Glutamic-oxaloacetic transaminase 2, mitochondrial (aspartate aminotransferase 2)); GP1BA (Glycoprotein Ib (platelet), alpha polypeptide); GP1BB (Glycoprotein Ib (platelet), beta polypeptide); GP9 (Glycoprotein IX (platelet)); GPD2 (Glycerol-3-phosphate dehydrogenase 2 (mitochondrial)); GPR10 (G protein-coupled receptor 10); GPR13 (G protein-coupled receptor 13); GPR15 (G protein-coupled receptor 15); GPR2 (G protein-coupled receptor 2); GPR30 (G protein-coupled receptor 30); GPR4 (G protein-coupled receptor 4); GPR5 (G protein-coupled receptor 5); GPR9 (G protein-coupled receptor 9); GPT (Glutamic-pyruvate transaminase (alanine aminotransferase)); GPX1 (Glutathione peroxidase 1); GPX2 (Glutathione peroxidase 2 (gastrointestinal)); GRB10 (Growth factor receptor-bound protein 10); GRB14 (Growth factor receptor-bound protein 14); GRIK1 (Glutamate receptor, ionotropic, kainate 1); GRIK2 (Glutamate receptor, ionotropic, kainate 2); GRL (Glucocorticoid receptor); GRO1 (GRO1 oncogene (melanoma growth stimulating activity, alpha)); GRO2 (GRO2 oncogene); GRO3 (GRO3 oncogene); GRPR (Gastrin-releasing peptide receptor); GSN (Gelsolin (amyloidosis, Finnish type)); GSPT1 (G1 to S phase transition 1); GSR (Glutathione reductase); GSS (Glutathione synthetase); GSTA1 (Glutathione S-transferase A1); GSTA2 (Glutathione S-transferase A2); GSTA4 (Glutathione S-transferase A4); GSTM1 (Glutathione S-transferase M1); GSTM2 (Glutathione S-transferase M2 (muscle)); GSTM3 (Glutathione S-transferase M3 (brain)); GSTP1 (Glutathione S-transferase pi); GSTT1 (Glutathione S-transferase theta 1); GTF2H2 (General transcription factor IIH, polypeptide 2 (44kD subunit)); GTS (Gilles de la Tourette syndrome); GUCY1A3 (Guanylate cyclase 1, soluble, alpha 3); GUCY2D (Guanylate cyclase 2D, membrane (retina-specific)); GUSB (Glucuronidase, beta); GYPA (Glycophorin A (includes MN blood group)); GYPB (Glycophorin B (includes Ss blood group)); GYS1 (Glycogen synthase I (muscle)); GZMA (Granzyme A (granzyme 1, cytotoxic T-lymphocyte-associated serine esterase 3)); GZMB (Granzyme B (granzyme 2, cytotoxic T-lymphocyte-associated serine esterase 1)); GZMM (Granzyme M (lymphocyte met-ase 1)); H142T (Temperature sensitivity complementation, H142); HADHA (Hydroxyacyl-Coenzyme A dehydrogenase/3-ketoacyl-Coenzyme A thiolase/enoyl-Coenzyme A hydratase (trifunctional protein), alpha subunit); HADHSC (L-3-hydroxyacyl-Coenzyme A dehydrogenase, short chain); HAGH (Hydroxyacyl glutathione hydrolase; glyoxalase 2); HAL (Histidine ammonia-lyase); HBA1 (Hemoglobin, alpha 1); HBB (Hemoglobin, beta); HBG1 (Hemoglobin, gamma A); HBZ (Hemoglobin, zeta); HCF2 (Heparin cofactor II); HCFC1 (Host cell factor C1 (VP16-accessory protein)); HCR (Chemokine receptor); HD (Huntingtin (Huntington disease)); HDAC1 (Histone deacetylase 1); HEXA (Hexosaminidase A (alpha polypeptide)); HEXB (Hexosaminidase B (beta polypeptide)); HF1 (H factor 1 (complement)); HFE (Hemochromatosis); HFL1 (H factor (complement)-like 1); HGF (Hepatocyte growth factor (hepapoietin A; scatter factor)); HGL (Heregulin, alpha (45kD, ERBB2 p185-activator)); HIP1 (Huntingtin interacting protein 1); HIVEP1 (Human immunodeficiency virus type I enhancer-binding protein 1); HK1 (Hexokinase 1); HK2 (Hexokinase 2); HK3 (Hexokinase 3 (white cell)); HLA-A (Major histocompatibility complex, class I, A); HLA-B (Major histocompatibility complex, class I, B); HLA-C (Major histocompatibility complex, class I, C); HLA-DMA (Major histocompatibility complex, class II, DM alpha); HLA-DMB (Major histocompatibility complex, class II, DM beta); HLA-DNA (Major histocompatibility complex, class II, DN alpha); HLA-DOB (Major histocompatibility complex, class II, DO beta); HLA-DPA1 (Major histocompatibility complex, class II, DP alpha 1); HLA-DPB1 (Major histocompatibility complex, class II, DP beta 1); HLA-DQA1 (Major histocompatibility complex, class II, DQ alpha 1); HLA-DQB1 (Major histocompatibility complex, class II, DQ beta 1); HLA-DRA (Major histocompatibility complex, class II, DR alpha); HLA-DRB1 (Major histocompatibility complex, class II, DR beta 1); HLA-E (Major histocompatibility complex, class I, E); HLA-F (Major histocompatibility complex, class I, F); HLA-G (HLA-G histocompatibility antigen, class I, G); HLALS (Major histocompatibility complex, class I-like sequence (NOTE: symbol provisional)); HLCS (Holocarboxylase synthetase (biotin-[proprionyl-Coenzyme A-carboxylase (ATP-hydrolysing)] ligase)); HLX1 (H2.0 (Drosophila)-like homeo box 1); HM74 (Putative chemokine receptor; GTP-binding protein); HMAB (Monocyte antigen B); HMBS (Hydroxymethylbilane synthase); HMGCL (3-hydroxymethyl-3-methylglutaryl-Coenzyme A lyase ((hydroxymethylglutaricaciduria)); HMGIC (High-mobility group (nonhistone chromosomal) protein isoform I-C); HMSNL ); HMX1 (Homeo box (H6 family) 1); HNRPD (Heterogeneous nuclear ribonucleoprotein D); HOXB5 (Homeo box B5); HOXD13 (Homeo box D13); HOXD8 (Homeo box D8); HP (Haptoglobin); HPE1 (Holoprosencephaly 1, alobar); HPN (Hepsin (transmembrane protease, serine 1)); HPR (Haptoglobin-related protein); HPRT1 (Hypoxanthine phosphoribosyltransferase 1 (Lesch-Nyhan syndrome)); HPS (Hermansky-Pudlak syndrome); HPX (Hemopexin); HR (Hairless (mouse) homolog); HRAS (V-Ha-ras Harvey rat sarcoma viral oncogene homolog); HRG (Histidine-rich glycoprotein); HRMT1L1 (HMT1 (hnRNP methyltransferase, S. cerevisiae)-like 1); HRMT1L2 (HMT1 (hnRNP methyltransferase, S. cerevisiae)-like 2); HRY (Hairy (Drosophila)-homolog); HSD17B3 (Hydroxysteroid (17-beta) dehydrogenase 3); HSD3B1 (Hydroxy-delta-5-steroid dehydrogenase, 3 beta- and steroid delta-isomerase 1); HSD3B2 (Hydroxy-delta-5-steroid dehydrogenase, 3 beta- and steroid delta-isomerase 2); HSPA1A (Heat shock 70kD protein 1); HSPA1L (Heat shock 70kD protein-like 1); HSPA2 (Heat shock 70kD protein 2); HSPA6 (Heat shock 70kD protein 6 (HSP70B')); HSPG2 (Heparan sulfate proteoglycan 2 (perlecan)); HTLVR (Human T-cell leukemia virus (I and II) receptor); HTN1 (Histatin 1); HTN3 (Histatin 3); HTR2A (5-hydroxytryptamine (serotonin) receptor 2A); HTR2C (5-hydroxytryptamine (serotonin) receptor 2C); HTR6 (5-hydroxytryptamine (serotonin) receptor 6); HTR7 (5-hydroxytryptamine (serotonin) receptor 7 (adenylate cyclase-coupled)); HVBS6 (Hepatitis B virus integration site 6); HY (Histocompatibility Y antigen); IARS (Isoleucine-tRNA synthetase); IBD1 (Inflammatory bowel disease 1); IBSP (Integrin-binding sialoprotein (bone sialoprotein, bone sialoprotein II)); ICAM1 (Intercellular adhesion molecule 1 (CD54), human rhinovirus receptor); ICAM2 (Intercellular adhesion molecule 2); ICAM3 (Intercellular adhesion molecule 3); ICAM4 (Intercellular adhesion molecule 4, Landsteiner-Wiener blood group); ICAM5 (Intercellular adhesion molecule 5, telencephalin); ICR5 (Ichthyosis congenita V, Sjogren-Larsson-like); ICS1 (Immotile cilia syndrome 1); ICTI (Immature colon carcinoma transcript 1); IDDM10 (Insulin-dependent diabetes mellitus 10); IDDM11 (Insulin-dependent diabetes mellitus 11); IDDM15 (Insulin-dependent diabetes mellitus 15); IDDM17 (Insulin-dependent diabetes mellitus 17); IDDM4 (Insulin-dependent diabetes mellitus 4); IDDM6 (Insulin-dependent diabetes mellitus 6); IDDM7 (Insulin-dependent diabetes mellitus 7); IDDMX (Diabetes mellitus, insulin-dependent, X-linked, susceptibility to); IDH1 (Isocitrate dehydrogenase 1 (NADP+), soluble); IDS (Iduronate 2-sulfatase (Hunter syndrome)); IDUA (Iduronidase, alpha-L-); IF (I factor (complement)); IFNA1 (Interferon, alpha 1); IFNA10 (Interferon, alpha 10); IFNA13 (Interferon, alpha 13); IFNA2 (Interferon, alpha 2); IFNAR1 (Interferon (alpha, beta and omega) receptor 1); IFNAR2 (Interferon (alpha, beta and omega) receptor 2); IFNG (Interferon, gamma); IFNGR1 (Interferon gamma receptor 1); IFNGR2 (Interferon gamma receptor 2 (interferon gamma transducer 1)); IFNR (Interferon production regulator); IGAT (Immune response to synthetic polypeptide--IRGAT); IGER (IgE responsiveness (atopic)); IGES (Immunoglobulin E concentration, serum); IGF1 (Insulin-like growth factor 1 (somatomedin C)); IGF1R (Insulin-like growth factor 1 receptor); IGF2 (Insulin-like growth factor 2 (somatomedin A)); IGF2R (Insulin-like growth factor 2 receptor); IGFBP1 (Insulin-like growth factor binding protein 1); IGFBP10 (Insulin-like growth factor binding protein 10); IGFBP2 (Insulin-like growth factor binding protein 2 (36kD)); IGFBP3 (Insulin-like growth factor binding protein 3); IGHA1 (Immunoglobulin alpha 1); IGHA2 (Immunoglobulin alpha 2 (A2M marker)); IGHE (Immunoglobulin epsilon); IGHG1 (Immunoglobulin gamma 1 (Gm marker)); IGHG2 (Immunoglobulin gamma 2 (Gm marker)); IGHV@ (Immunoglobulin heavy polypeptide, variable region (cluster)); IGJ (Immunoglobulin J polypeptide, linker protein for immunoglobulin alpha and mu polypeptides); IGKC (Immunoglobulin kappa constant region); IGKV (Immunoglobulin kappa variable region); IGLP1 (Immune response to synthetic polypeptides-1); IGLP2 (Immune response to synthetic polypeptides-2); IL10 (Interleukin 10); IL10RA (Interleukin 10 receptor, alpha); IL10RA (Interleukin 10 receptor, alpha); IL10RB (Interleukin 10 receptor, beta); IL10RB (Interleukin 10 receptor, beta); IL11 (Interleukin 11); IL11RA (Interleukin 11 receptor, alpha); IL11RA (Interleukin 11 receptor, alpha); IL11RB (Interleukin 11 receptor, beta); IL12A (Interleukin 12A (natural killer cell stimulatory factor 1, cytotoxic lymphocyte maturation factor 1, p35)); IL12B (Interleukin 12B (natural killer cell stimulatory factor 2, cytotoxic lymphocyte maturation factor 2, p40)); IL12RB1 (Interleukin 12 receptor, beta 1); IL12RB2 (Interleukin 12 receptor, beta 2); IL12RB2 (Interleukin 12 receptor, beta 2); IL13 (Interleukin 13); IL13RA1 (Interleukin 13 receptor, alpha 1); IL13RA2 (Interleukin 13 receptor, alpha 2); IL13RA2 (Interleukin 13 receptor, alpha 2); IL15 (Interleukin 15); IL15RA (Interleukin 15 receptor, alpha); IL15RA (Interleukin 15 receptor, alpha); IL15RB (Interleukin 15 receptor, beta); IL16 (Interleukin 16 (lymphocyte chemoattractant factor)); IL17 (Interleukin 17 (cytotoxic T-lymphocyte-associated serine esterase 8)); IL18 (Interleukin 18 (interferon-gamma-inducing factor)); IL18BP (Interleukin 18 binding protein); IL18R1 (Interleukin 18 receptor 1); IL18RAP (Interleukin 18 receptor accessory protein); IL1A (Interleukin 1, alpha); IL1B (Interleukin 1, beta); IL1R1 (Interleukin 1 receptor, type I); IL1R1 (Interleukin 1 receptor, type I); IL1R2 (Interleukin 1 receptor, type II); IL1RAP (Interleukin 1 receptor accessory protein); IL1RL2 (Interleukin 1 receptor-like 2); IL1RN (Interleukin 1 receptor antagonist); IL2 (Interleukin 2); IL2RA (Interleukin 2 receptor, alpha); IL2RA (Interleukin 2 receptor, alpha); IL2RB (Interleukin 2 receptor, beta); IL2RB (Interleukin 2 receptor, beta); IL2RG (Interleukin 2 receptor, gamma (severe combined immunodeficiency) ); IL2RG (Interleukin 2 receptor, gamma (severe combined immunodeficiency)); IL3 (Interleukin 3 (colony-stimulating factor, multiple)); IL3RA (Interleukin 3 receptor, alpha (low affinity)); IL4 (Interleukin 4); IL4R (Interleukin 4 receptor); IL4R (Interleukin 4 receptor); IL5 (Interleukin 5 (colony-stimulating factor, eosinophil)); IL5RA (Interleukin 5 receptor, alpha); IL5RA (Interleukin 5 receptor, alpha); IL6 (Interleukin 6 (interferon, beta 2)); IL6R (Interleukin 6 receptor); IL6ST (Interleukin 6 signal transducer (gp130, oncostatin M receptor)); IL6ST (Interleukin 6 signal transducer (gp130, oncostatin M receptor)); IL7 (Interleukin 7); IL7R (Interleukin 7 receptor); IL8 (Interleukin 8); IL8RA (Interleukin 8 receptor, alpha); IL8RB (Interleukin 8 receptor, beta); IL8RB (Interleukin 8 receptor, beta); IL9 (Interleukin 9); IL9R (Interleukin 9 receptor); IL9R (Interleukin 9 receptor); ILF1 (Interleukin enhancer binding factor 1); ILF2 (Interleukin enhancer binding factor 2, 45kD); ILF3 (Interleukin enhancer binding factor 3, 90kD); IMPA1 (Inositol(myo)-1(or 4)-monophosphatase 1); IMPT1 (Imprinted polyspecific membrane transporter 1); INLU (Lutheran inhibitor, dominant (monoclonal antibody A3D8) ); INP10 (Interferon (gamma)-induced cell line; protein 10 from INPP5D Inositol polyphosphate-5-phosphatase, 145kD); INS (Insulin); INSR (Insulin receptor); IPF1 (Insulin promoter factor 1, homeodomain transcription factor); IPOX (Intestinal pseudoobstruction, neuronal, primary idiopathic); IRAK1 (Interleukin-1 receptor-associated kinase 1); IRAK2 (Interleukin-1 receptor-associated kinase 2); IRF4 (Interferon regulatory factor 4); IRS1 (Insulin receptor substrate 1); ITGA1 (Integrin, alpha 1); ITGA2 (Integrin, alpha 2 (CD49B, alpha 2 subunit of VLA-2 receptor)); ITGA2B (Integrin, alpha 2b (platelet glycoprotein IIb of IIb/IIIa complex, antigen CD41B)); ITGA4 (Integrin, alpha 4 (antigen CD49D, alpha 4 subunit of VLA-4 receptor) ); ITGA5 (Integrin, alpha 5 (fibronectin receptor, alpha polypeptide)); ITGA6 (Integrin, alpha 6); ITGA7 (Integrin, alpha 7); ITGAD (Integrin, alpha D); ITGAL (Integrin, alpha L (antigen CD11A (p180), lymphocyte function-associated antigen 1; alpha polypeptide)); ITGAM (Integrin, alpha M (complement component receptor 3, alpha; also known as CD11b (p170), macrophage antigen alpha polypeptide)); ITGAV (Integrin, alpha V (vitronectin receptor, alpha polypeptide, antigen CD51)); ITGAX (Integrin, alpha X (antigen CD11C (p150), alpha polypeptide)); ITGB1 (Integrin, beta 1 (fibronectin receptor, beta polypeptide, antigen CD29 includes MDF2, MSK12)); ITGB2 (Integrin, beta 2 (antigen CD18 (p95), lymphocyte function-associated antigen 1; macrophage antigen 1 (mac-1) beta subunit)); ITGB3 (Integrin, beta 3 (platelet glycoprotein IIIa, antigen CD61)); ITGB4 (Integrin, beta 4); ITGB4BP (Integrin beta 4 binding protein); ITGB5 (Integrin, beta 5); ITGB6 (Integrin, beta 6); ITGB7 (Integrin, beta 7); ITIH1 (Inter-alpha (globulin) inhibitor, H1 polypeptide); ITIH4 (Inter-alpha (globulin) inhibitor H4 (plasma Kallikrein-sensitive glycoprotein)); ITK (IL2-inducible T-cell kinase); IVL (Involucrin); JAK1 (Janus kinase 1 (a protein tyrosine kinase)); JAK2 (Janus kinase 2 (a protein tyrosine kinase)); JAK3 (Janus kinase 3 (a protein tyrosine kinase, leukocyte)); JPD (Juvenile periodontitis); JUP (Junction plakoglobin); KAL1 (Kallmann syndrome 1 sequence); KARS (Lysyl-tRNA synthetase); KCNA1 (Potassium voltage-gated channel, shaker-related subfamily, member 1 (episodic ataxia with myokymia)); KCNA2 (Potassium voltage-gated channel, shaker-related subfamily, member 2); KCNA3 (Potassium voltage-gated channel, shaker-related subfamily, member 3); KCNA5 (Potassium voltage-gated channel, shaker-related subfamily, member 5); KCNE1 (Potassium voltage-gated channel, Isk-related family, member 1); KCNJ12 (Potassium inwardly-rectifying channel, subfamily J, member 12); KCNJ3 (Potassium inwardly-rectifying channel, subfamily J, member 3); KCNQ1 (Potassium voltage-gated channel, KQT-like subfamily, member 1); KCNQ2 (Potassium voltage-gated channel, KQT-like subfamily, member 2); KDR (Kinase insert domain receptor (a type III receptor tyrosine kinase)); KEL (Kell blood group); KHK (Ketohexokinase (fructokinase)); KIR2DL4 (Killer cell immunoglobulin-like receptor, two domains, long cytoplasmic tail, 4); KIT (V-kit Hardy-Zuckerman 4 feline sarcoma viral oncogene homolog); KLKB1 (Kallikrein B plasma, (Fletcher factor) 1); KLRC2 (Killer cell lectin-like receptor subfamily C, member 2); KLRC3 (Killer cell lectin-like receptor subfamily C, member 3); KLRC4 (Killer cell lectin-like receptor subfamily C, member 4); KLRD1 (Killer cell lectin-like receptor subfamily D, member 1); KNG (Kininogen); KPNA1 (Karyopherin alpha 1 (importin alpha 5)); KRAS2 (V-Ki-ras2 Kirsten rat sarcoma 2 viral oncogene homolog); KRT4 (Keratin 4); KRT9 (Keratin 9 (epidermolytic palmoplantar keratoderma)); KRTHA4 (Keratin, hair, acidic, 4); KRTHB4 (Keratin, hair, basic, 4); KSR (Kinase suppressor of ras); L1CAM (L1 cell adhesion molecule (hydrocephalus, stenosis of queduct of Sylvius 1, MASA (mental retardation, aphasia, shuffling gait and adducted thumbs) syndrome, spastic paraplegia 1)); LAG3 (Lymphocyte-activation gene 3); LAG5 (Leukocyte antigen group 5); LAKL (Lymphokine-activated killer cell ligand); LALBA (Lactalbumin, alpha-); LAMA3 (Laminin, alpha 3 (nicein (150kD), kalinin (165kD), BM600 (150kD), epilegrin)); LAMC1 (Laminin, gamma 1 (formerly LAMB2)); LAMP2 (Lysosomal-associated membrane protein 2); LAMR1 (Laminin receptor 1 (67kD); Ribosomal protein SA); LBP (Lipopolysaccharide-binding protein); LCK (Lymphocyte-specific protein tyrosine kinase); LCN1 (Lipocalin 1 (protein migrating faster than albumin, tear prealbumin)); LCN2 (Lipocalin 2 (oncogene 24p3)); LCP1 (Lymphocyte cytosolic protein 1 (L-plastin)); LCP2 (Lymphocyte cytosolic protein 2 (SH2 domain-containing leukocyte protein of 76kD)); LDHA (Lactate dehydrogenase A); LDLR (Low density lipoprotein receptor (familia X02152); LDLR (Low density lipoprotein receptor (familial hypercholesterolemia)); LECT2 (Leukocyte cell-derived chemotaxin 2); LEP (Leptin (murine obesity homolog)); LEPR (Leptin receptor); LGALS1 (Lectin, galactoside-binding, soluble, 1 (galectin 1)); LGALS3 (Lectin, galactoside-binding, soluble, 3 (galectin 3)); LGALS3BP (Lectin, galactoside-binding, soluble, 3 binding protein (galectin 6 binding protein)); LGALS9 (Lectin, galactoside-binding, soluble, 9 (galectin 9)); LHB (Luteinizing hormone beta polypeptide); LHCGR (Luteinizing hormone/choriogonadotropin receptor); LIF (Leukemia inhibitory factor (cholinergic differentiation factor)); LIFR (Leukemia inhibitory factor receptor); LIFR (Leukemia inhibitory factor receptor); LIG1 (Ligase I, DNA, ATP-dependent); LKN-1 (Chemokine CC-2,); LMAN1 (Lectin, mannose-binding, 1); LMO4 (LIM domain only 4); LNPEP (Leucyl/cystinyl aminopeptidase); LOX (Lysyl oxidase); LPA (Lipoprotein, Lp(a)); LPL (Lipoprotein lipase); LQT2 (Long (electrocardiographic) QT syndrome 2); LRP1 (Low density lipoprotein-related protein 1 (alpha-2-macroglobulin receptor)); LRP2 (Low density lipoprotein-related protein 2); LSL (Leptin, serum levels of); LSP1 (Lymphocyte-specific protein 1); LTA (Lymphotoxin alpha (TNF superfamily, member 1)); LTB4R (Leukotriene b4 receptor (chemokine receptor-like 1)); LTB4R (Leukotriene b4 receptor (chemokine receptor-like 1)); LTC4S (Leukotriene C4 synthase); LTF (Lactotransferrin); LTK (Leukocyte tyrosine kinase); LU (Lutheran blood group (Auberger b antigen included)); LY64 (Lymphocyte antigen 64 (mouse) homolog, radioprotective, 105kD); LY9 (Lymphocyte antigen 9); LYN (V-yes-1 Yamaguchi sarcoma viral related oncogene homolog); LYZ (Lysozyme (renal amyloidosis)); M1S1 (Membrane component, chromosome 1, surface marker 1 (40kD glycoprotein, identified by monoclonal antibody GA733)); MAB21L1 (Mab-21 (C. elegans)-like 1); MACAM1 (Mucosal addressin cell adhesion molecule-1); MADH1 (MAD (mothers against decapentaplegic, Drosophila) homolog 1); MADH2 (MAD (mothers against decapentaplegic, Drosophila) homolog 2); MADH4 (MAD (mothers against decapentaplegic, Drosophila) homolog 4); MAGEA1 (Melanoma antigen, family A, 1 (directs expression of antigen MZ2-E)); MAGEB1 (Melanoma antigen, family B, 1); MAL (Mal, T-cell differentiation protein); MALL (Mal, T-cell differentiation protein-like); MANB (Mannosidase, alpha B, lysosomal); MAP1B (Microtubule-associated protein 1B); MAPKAPK3 (Mitogen-activated protein kinase-activated protein kinase 3); MASP1 (Mannan-binding lectin serine protease 1 (C4/C2 activating component of Ra-reactive factor)); MAT2A (Methionine adenosyltransferase II, alpha); MATN1 (Matrilin 1, cartilage matrix protein); MATN3 (Matrilin 3); MBL2 (Mannose-binding lectin (protein C) 2, soluble (opsonic defect)); MC1R (Melanocortin 1 receptor (alpha melanocyte stimulating hormone receptor)); MC2R (Melanocortin 2 receptor (adrenocorticotropic hormone)); MCC (Mutated in colorectal cancers); MCF2 (MCF.2 cell line derived transforming sequence); MCP (Membrane cofactor protein (CD46, trophoblast-lymphocyte cross-reactive antigen)); MDF1 (Antigen identified by monoclonal antibody A-3A4); MDH2 (Malate dehydrogenase 2, NAD (mitochondrial)); MDU1 (Antigen identified by monoclonal antibodies 4F2, TRA1.10, TROP4, and T43); ME1 (Malic enzyme 1, soluble); ME2 (Malic enzyme 2, mitochondrial); MEKK1 (MAP/ERK kinase kinase 1); MEKK3 (MAP/ERK kinase kinase 3); MEMO1 (Methylation modifier for class I HLA); MEN1 (Multiple endocrine neoplasia I); MEP1A (Meprin A, alpha (PABA peptide hydrolase)); MER2 (Antigen identified by monoclonal antibodies 1D12, 2F7); MFAP2 (Microfibrillar-associated protein 2); MFAP4 (Microfibrillar-associated protein 4); MFTS (Migraine, familial typical, susceptibility to); MGCT ( MGI); MGP (Matrix Gla protein); MHC2TA (MHC class II transactivator); MIC2 (Antigen identified by monoclonal antibodies 12E7, F21 and O13); MIC5 (Antigen identified by monoclonal antibody R1); MIC7 (Antigen identified by monoclonal antibody 28.3.7); MICA (MHC class I polypeptide-related sequence A); MIF (Macrophage migration inhibitory factor (glycosylation-inhibiting factor)); MIG (Monokine induced by gamma interferon); MIR-10 (Leukocyte immunoglobulin-like receptor); MITF (Microphthalmia-associated transcription factor); MLLT2 (Myeloid/lymphoid or mixed-lineage leukemia (trithorax (Drosophila) homolog); translocated to, 2); MLN (Motilin); MLR (Mineralocorticoid receptor (aldosterone receptor)); MMP12 (Matrix metalloproteinase 12 (macrophage elastase)); MMP13 (Matrix metalloproteinase 13 (collagenase 3)); MMP14 (Matrix metalloproteinase 14 (membrane-inserted)); MMP15 (Matrix metalloproteinase 15 (membrane-inserted)); MMP16 (Matrix metalloproteinase 16 (membrane-inserted)); MMP17 (Matrix metalloproteinase 17 (membrane-inserted)); MMP18 (Matrix metalloproteinase 18); MMP 19 (Matrix metalloproteinase 19); MMP2 (Matrix metalloproteinase 2 (gelatinase A, 72kD gelatinase, 72kD type IV collagenase)); MMP20 (Matrix metalloproteinase 20); MMP21 (Matrix metalloproteinase 21); MMP3 (Matrix metalloproteinase 3 (stromelysin 1, progelatinase)); MMP7 (Matrix metalloproteinase 7 (matrilysin, uterine)); MMP8 (Matrix metalloproteinase 8 (neutrophil collagenase)); MMP9 (Matrix metalloproteinase 9 (gelatinase B, 92kD gelatinase, 92kD type IV collagenase)); MNG1 (Multinodular goitre 1); MOG (Myelin oligodendrocyte glycoprotein); MPL (Myeloproliferative leukemia virus oncogene); MPO (Myeloperoxidase); MRBC (Monkey RBC receptor); MRC1 (Mannose receptor, C type 1); MRX20 (Mental retardation, X-linked 20); MSH3 (MutS (E. coli) homolog 3); MSLR1 (Macrophage scavenger receptor-like 1); MSR1 (Macrophage scavenger receptor 1); MSS (Marinesco-Sjogren syndrome); MSSE (Multiple self-healing squamous epithelioma); MST1 (Macrophage stimulating 1 (hepatocyte growth factor-like)); MST1R (Macrophage stimulating 1 receptor (c-met-related tyrosine kinase)); MSX2 (Msh (Drosophila) homeo box homolog 2); MTCO1 (Cytochrome c oxidase I); MTHFD (5,10-methylenetetrahydrofolate dehydrogenase, 5,10-methylenetetrahydrofolate cyclohydrolase, 10-formyltetrahydrofolate synthetase); MTHFR (5,10-methylenetetrahydrofolate reductase (NADPH)); MTND2 (NADH dehydrogenase 2); MTP (Microsomal triglyceride transfer protein (large polypeptide, 88kD)); MTR (5-methyltetrahydrofolate-homocysteine methyltransferase); MTRR (5-methyltetrahydrofolate-homocysteine methyltransferase reductase); MUC1 (Mucin 1, transmembrane); MUC2 (Mucin 2, intestinal/tracheal); MUC4 (Mucin 4, tracheobronchial); MUL (Mulibrey nanism); MUT (Methylmalonyl Coenzyme A mutase); MX1 (Myxovirus (influenza) resistance 1, homolog of murine (interferon-inducible protein p78)); MXI1 (MAX-interacting protein 1); MYB (V-myb avian myeloblastosis viral oncogene homolog); MYBPC3 (Myosin-binding protein C, cardiac); MYC (V-myc avian myelocytomatosis viral oncogene homolog); MYCL1 (V-myc avian myelocytomatosis viral oncogene homolog 1, lung carcinoma derived); MYD88 (Myeloid differentiation primary response gene (88)); MYF5 (Myogenic factor 5); MYF6 (Myogenic factor 6 (herculin)); MYO5A (Myosin VA (heavy polypeptide 12, myoxin)); MYO9B (Myosin IXB); NAB1 (NGFI-A binding protein 1 (ERG1 binding protein 1)); NAGA (N-acetylgalactosaminidase, alpha-); NAIP (Neuronal apoptosis inhibitory protein); NAPA (N-ethylmaleimide-sensitive factor attachment protein, alpha); NAPB ); NAPG (N-ethylmaleimide-sensitive factor attachment protein, gamma); NAT1 (N-acetyltransferase 1 (arylamine N-acetyltransferase)); NAT2 (N-acetyltransferase 2 (arylamine N-acetyltransferase)); NB (Neuroblastoma (neuroblastoma suppressor)); NCAM1 (Neural cell adhesion molecule 1); NCF1 (Neutrophil cytosolic factor 1 (47kD, chronic granulomatous disease, autosomal 1)); NCF2 (Neutrophil cytosolic factor 2 (65kD, chronic granulomatous disease, autosomal 2)); NCF4 (Neutrophil cytosolic factor 4 (40kD)); NDP (Norrie disease (pseudoglioma)); NDUFS2 (NADH dehydrogenase (ubiquinone) Fe-S protein 2 (49kD) (NADH-coenzyme Q reductase)); NEB (Nebulin); NEU (Neuraminidase); NF1 (Neurofibromin 1 (neurofibromatosis, von Recklinghausen disease, Watson disease)); NF2 (Neurofibromin 2 (bilateral acoustic neuroma)); NFATC1 (Nuclear factor of activated T-cells, cytoplasmic 1); NFATC3 (Nuclear factor of activated T-cells, cytoplasmic 3); NFATC4 (Nuclear factor of activated T-cells, cytoplasmic 4); NFE2 (Nuclear factor (erythroid-derived 2), 45kD); NFE2L2 (Nuclear factor (erythroid-derived 2)-like 2); NFIL3 (Nuclear factor, interleukin 3 regulated); NFKB1 (Nuclear factor of kappa light polypeptide gene enhancer in B-cells 1 (p105)); NFKB2 (Nuclear factor of kappa light polypeptide gene enhancer in B-cells 2 (p49/p100)); NFKBIA (Nuclear factor of kappa light polypeptide gene enhancer in B-cells inhibitor, alpha); NFRKB (Nuclear factor related to kappa B binding protein); NFYA (Nuclear transcription factor Y, alpha); NGFB (Nerve growth factor, beta polypeptide); NKS1 (Natural killer cell susceptibility 1); NM (Neutrophil migration); NME1 (Non-metastatic cells 1, protein (NM23A) expressed in); NMOR2 (NAD(P)H menadione oxidoreductase 2, dioxin-inducible); NNMT (Nicotinamide N-methyltransferase); NOS1 (Nitric oxide synthase 1 (neuronal)); NOS2A (Nitric oxide synthase 2A (inducible, hepatocytes)); NOS2B (Nitric oxide synthase 2B); NOS2C (Nitric oxide synthase 2C); NOS3 (Nitric oxide synthase 3 (endothelial cell)); NOTCH1 (Notch (Drosophila) homolog 1 (translocation-associated)); NOTCH4 (Notch (Drosophila) homolog 4); NP (Nucleoside phosphorylase); NPC1 (Niemann-Pick disease, type C1); NPHP1 (Nephronophthisis 1 (juvenile)); NPY1R (Neuropeptide Y receptor Y1); NRAMP1 (Natural resistance-associated macrophage protein 1 (might include Leishmaniasis)); NRAMP2 (Natural resistance-associated macrophage protein 2); NRAS (Neuroblastoma RAS viral (v-ras) oncogene homolog); NRL (Neural retina leucine zipper); NT5 (5' nucleotidase (CD73)); NTF3 (Neurotrophin 3); NUCB1 (Nucleobindin 1); NUMA1 (Nuclear mitotic apparatus protein 1); NURR1 (Nuclear receptor related 1 (transcriptionally inducible)); OA1 (Ocular albinism 1 (Nettleship-Falls)); OAS1 (2',5'-oligoadenylate synthetase 1); OAS2 (2'-5'oligoadenylate synthetase 2); OAT (Ornithine aminotransferase (gyrate atrophy)); OCRL (Oculocerebrorenal syndrome of Lowe); ODC1 (Ornithine decarboxylase 1); OGG1 (8-oxoguanine DNA glycosylase); OLFR2 (Olfactory receptor 2); OMG (Oligodendrocyte myelin glycoprotein); OPA1 (Optic atrophy 1 (autosomal dominant)); OPA3 (Iraqi-Jewish optic atrophy plus (3-methylglutaconicaciduria type 3)); OPLL ); OPRM1 (Opioid receptor, mu 1); OPTA2 (Osteopetrosis, autosomal dominant, type II); OPTB1 (Osteopetrosis, autosomal recessive); OR1D2 (Olfactory receptor, family 1, subfamily D, member 2); ORCTL2 (Beckwith-Wiedemann syndrome chromosome region 1, candidate A; Organic cation transporter-like 2; Imprinted polyspecific membrane transporter 1); ORM1 (Orosomucoid 1); ORM2 (Orosomucoid 2); OSM (Oncostatin M); OTC (Ornithine carbamoyltransferase); OXT (Oxytocin, prepro- (neurophysin I)); P (P blood group globoside); P1 (P blood group (P one antigen)); P2RX1 (Purinergic receptor P2X, ligand-gated ion channel, 1); P2RY1 (Purinergic receptor P2Y, G-protein coupled, 1); PA2G4 (Proliferation-associated 2G4, 38kD); PAC1 (Prostate adenocarcinoma-1); PACE (Paired basic amino acid cleaving enzyme (furin, membrane associated receptor protein)); PAEP (Progestagen-associated endometrial protein (placental protein 14, pregnancy-associated endometrial alpha-2-globulin, alpha uterine protein)); PAFAH (Platelet-activating factor acetylhydrolase); PAFAH1B1 (Platelet-activating factor acetylhydrolase, isoform Ib, alpha subunit (45kD)); PAFAH1B2 (Platelet-activating factor acetylhydrolase, isoform Ib, beta subunit (30kD)); PAFAH2 (Platelet-activating factor acetylhydrolase 2 (40kD)); PAH (Phenylalanine hydroxylase); PAI1 (Plasminogen activator inhibitor, type I); PAPPA (Pregnancy-associated plasma protein A); PAR4 (Protease-activated receptor-4 PAWR); PRKC, apoptosis, WT1, regulator); PAX2 (Paired box gene 2); PAX3 (Paired box gene 3 (Waardenburg syndrome 1)); PAX8 (Paired box gene 8); PCBD (6-pyruvoyl-tetrahydropterin synthase/dimerization cofactor of hepatocyte nuclear factor 1 alpha (TCF1)); PCCA (Propionyl Coenzyme A carboxylase, alpha polypeptide); PCCB (Propionyl Coenzyme A carboxylase, beta polypeptide); PCI (Protein C inhibitor (plasminogen activator inhibitor III)); PCK1 (Phosphoenolpyruvate carboxykinase 1 (soluble)); PCM1 (Pericentriolar material 1); PCNT (Pericentrin); PCTK1 (PCTAIRE protein kinase 1); PCYT2 (Phosphate cytidylyltransferase 2, ethanolamine); PDB2); PDCD2 (Programmed cell death 2); PDE3B (Phosphodiesterase 3B, cGMP-inhibited); PDE4A (Phosphodiesterase 4A, cAMP-specific (dunce (Drosophila)-homolog phosphodiesterase E2)); PDE4B (Phosphodiesterase 4B, cAMP-specific (dunce (Drosophila)-homolog phosphodiesterase E4)); PDE7A (Phosphodiesterase 7A); PDES 1 B (Phosphodiesterase IB); PDGFA (Platelet-derived growth factor alpha polypeptide); PDGFB (Platelet-derived growth factor beta polypeptide (simian sarcoma viral (v-sis) oncogene homolog)); PDGFRA (Platelet-derived growth factor receptor, alpha polypeptide); PDGFRB (Platelet-derived growth factor receptor, beta polypeptide); PDHA1 (Pyruvate dehydrogenase (lipoamide) alpha 1); PDX1 (Pyruvate dehydrogenase complex, lipoyl-containing component X; E3-binding protein); PECAM1 (Platelet/endothelial cell adhesion molecule (CD31 antigen)); PEPC (Peptidase C); PEPD (Peptidase D); PEX10 (Peroxisome biogenesis factor 10); PF4 (Platelet factor 4); PF4V1 (Platelet factor 4 variant 1); PFBI (Plasmodium falciparum blood infection levels); PFC (Properdin P factor, complement); PFKL (Phosphofructokinase, liver); PFKM (Phosphofructokinase, muscle); PFKP (Phosphofructokinase, platelet); PFN1 (Profilin 1); PGA3 (Pepsinogen 3, group I (pepsinogen A)); PGC (Progastricsin (pepsinogen C)); PGD (Phosphogluconate dehydrogenase); PGF (Placental growth factor, vascular endothelial growth factor-related protein); PGK1 (Phosphoglycerate kinase 1); PGK2 (Phosphoglycerate kinase 2); PGL1 (Paraganglioma or familial glomus tumors 1); PGM1 (Phosphoglucomutase 1); PGM3 (Phosphoglucomutase 3); PGP (Phosphoglycolate phosphatase); PGY1 (P glycoprotein 1/multiple drug resistance 1); PHAP1 (Putative human HLA class II associated protein I); PHB (Prohibitin); PI (Protease inhibitor 1 (anti-elastase), alpha-1-antitrypsin); PI3 (Protease inhibitor 3, skin-derived (SKALP)); PI6 (Protease inhibitor 6 (placental thrombin inhibitor)); PI8 (Protease inhibitor 8 (ovalbumin type)); PI9 (Protease inhibitor 9 (ovalbumin type)); PIGA (Phosphatidylinositol glycan, class A (paroxysmal nocturnal hemoglobinuria)); PIGF (Phosphatidylinositol glycan, class F); PIGR (Polymeric immunoglobulin receptor); PIK3CA (Phosphoinositide-3-kinase, catalytic, alpha polypeptide); PIK3CB (Phosphoinositide-3-kinase, catalytic, beta polypeptide); PIK3R1 (Phosphoinositide-3-kinase, regulatory subunit, polypeptide 1 (p85 alpha)); PIL (Protease inhibitor 1 (alpha-1-antitrypsin)-like); PIN (Dynein, cytoplasmic, light polypeptide); PKLR (Pyruvate kinase, liver and RBC); PLAGL1 (Pleomorphic adenoma gene-like 1); PLAT (Plasminogen activator, tissue); PLCD1 (Phospholipase C, delta 1); PLCG1 (Phospholipase C, gamma 1 (formerly subtype 148)); PLEK (Pleckstrin); PLG (Plasminogen); PLOD (Procollagen-lysine, 2-oxoglutarate 5-dioxygenase (lysine hydroxylase, Ehlers-Danlos syndrome type VI)); PLP (Proteolipid protein (Pelizaeus-Merzbacher disease, spastic paraplegia 2, uncomplicated)); PLT1 (Primed lymphocyte test-1); PML (Promyelocytic leukemia); PMP22 (Peripheral myelin protein 22); PMS2 (Postmeiotic segregation increased (S. cerevisiae) 2); PNMT (Phenylethanolamine N-methyltransferase); PNUTL1 (Peanut (Drosophila)-like 1); POLB (Polymerase (DNA directed), beta); POMC (Proopiomelanocortin (adrenocorticotropin/beta-lipotropin/ alpha-melanocyte stimulating hormone/beta-melanocyte stimulating hormone/ beta-endorphin)); PON1 (Paraoxonase 1); PON2 (Paraoxonase 2); PORC (Porphyria, acute; Chester type); POU2AF1 (POU domain, class 2, associating factor 1); POU5F1 (POU domain, class 5, transcription factor 1); PPBP (Pro-platelet basic protein (includes platelet basic protein, beta-thromboglobulin, connective tissue-activating peptide III, neutrophil-activating peptide-2)); PPCD (Posterior polymorphous corneal dystrophy); PPH1 (Primary pulmonary hypertension 1); PPIB (Peptidylprolyl isomerase B (cyclophilin B)); PPOX (Protoporphyrinogen oxidase); PPP1R8 (Protein phosphatase 1, regulatory (inhibitor) subunit 8); PRB1 (Proline-rich protein BstNI subfamily 1); PRB2 (Proline-rich protein BstNI subfamily 2); PRB3 (Proline-rich protein BstNI subfamily 3); PRB4 (Proline-rich protein BstNI subfamily 4); PREP (Prolyl endopeptidase); PRF1 (Perforin 1 (preforming protein)); PRG1 (Proteoglycan 1, secretory granule); PRH1 (Proline-rich protein HaeIII subfamily 1); PRH2 (Proline-rich protein HaeIII subfamily 2); PRKCQ (Protein kinase C, theta); PRKDC (Protein kinase, DNA-activated, catalytic polypeptide); PRKG1 (Protein kinase, cGMP-dependent, type I); PRKM10 (Protein kinase mitogen-activated 10 (MAP kinase)); PRKM9 (Protein kinase mitogen-activated 9 (MAP kinase)); PRL (Prolactin); PRLR (Prolactin receptor); PRNP (Prion protein (p27-30) (Creutzfeld-Jakob disease, Gerstmann-Strausler-Scheinker syndrome, fatal familial insomnia)); PROC (Protein C (inactivator of coagulation factors Va and VIIIa) ); PROP1 (Prophet of Pit1, paired-like homeodomain transcription factor); PROS1 (Protein S (alpha)); PRPH (Peripherin); PRSS1 (Protease, serine, 1 (trypsin 1)); PRSS2 (Protease, serine, 2 (trypsin 2)); PRSS7 (Protease, serine, 7 (enterokinase)); PRSS8 (Protease, serine, 8 (prostasin)); PRTN3 (Proteinase 3 (serine proteinase, neutrophil, Wegener granulomatosis autoantigen)); PSAP (Prosaposin (variant Gaucher disease and variant metachromatic leukodystrophy)); PSD (Pleckstrin and Sec7 domain protein); PSMB8 (Proteasome (prosome, macropain) subunit, beta type, 8 (large multifunctional protease 7)); PSORS1 (Psoriasis susceptibility 1); PSORS2 (Psoriasis susceptibility 2); PSORS3 (Psoriasis susceptibility 3); PTAFR (Platelet-activating factor receptor); PTC (Phenylthiocarbamide tasting); PTCH (Patched (Drosophila) homolog); PTEN (Phosphatase and tensin homolog (mutated in multiple advanced cancers 1)); PTGDS (Prostaglandin D2 synthase (21 kD, brain)); PTGER3 (Prostaglandin E receptor 3 (subtype EP3)); PTGIR (Prostaglandin I2 (prostacyclin) receptor (IP)); PTGS1 (Prostaglandin-endoperoxide synthase 1 (prostaglandin G/H synthase and cyclooxygenase)); PTGS2 (Prostaglandin-endoperoxide synthase 2 (prostaglandin G/H synthase and cyclooxygenase)); PTK2B (Protein tyrosine kinase 2 beta); PTN (Pleiotrophin (heparin binding growth factor 8, neurite growth-promoting factor 1)); PTPN 13 (Protein tyrosine phosphatase, non-receptor type 13 (APO-1/CD95 (Fas)-associated phosphatase)); PTPN6 (Protein tyrosine phosphatase, non-receptor type 6); PTPRC (Protein tyrosine phosphatase, receptor type, c polypeptide); PTPRCAP (Protein tyrosine phosphatase, receptor type, c polypeptide-associated protein); PTPRD (Protein tyrosine phosphatase, receptor type, D); PTPRF (Protein tyrosine phosphatase, receptor type, F); PTPRG (Protein tyrosine phosphatase, receptor type, gamma polypeptide); PTX3 (Pentaxin-related gene, rapidly induced by IL-1 beta PUJO ); PVR (Poliovirus receptor); PVRL1 (Poliovirus receptor-like 1); PVRL2 (Poliovirus receptor-like 2); PXE (Pseudoxanthoma elasticum); PXMP1 (Peroxisomal membrane protein 1 (70kD, Zellweger syndrome)); PXN (Paxillin); PYCR1 (Pyrroline-5-carboxylate reductase 1); PYGM (Phosphorylase, glycogen; muscle (McArdle syndrome, glycogen storage disease type V)); QDPR (Quinoid dihydropteridine reductase); RAC2 (Ras-related C3 botulinum toxin substrate 2 (rho family, small GTP binding protein Rac2)); RAC3 (Ras-related C3 botulinum toxin substrate 3 (rho family, small GTP binding protein Rac3)); RAD17 (RAD17 (S. pombe) homolog); RAD51L3 (RAD51 (S. cerevisiae)-like 3); RAF1 (V-raf-1 murine leukemia viral oncogene homolog 1); RAG1 (Recombination activating gene 1); RAG2 (Recombination activating gene 2); RANBP3 (RAN binding protein 3); RAP1A (RAP1A, member of RAS oncogene family); RB1 (Retinoblastoma 1 (including osteosarcoma)); RBP4 (Retinol-binding protein 4, interstitial); RBS (Roberts syndrome); RCN2 (Reticulocalbin 2, EF-hand calcium binding domain); RCP (Red cone pigment (color blindness, protan)); RCV1 (Recoverin); RDBP (RD RNA-binding protein); RDS (Retinal degeneration, slow (retinitis pigmentosa 7)); RELA (V-rel avian reticuloendotheliosis viral oncogene homolog A (nuclear factor of kappa light polypeptide gene enhancer in B-cells 3 (p65))); REN (Renin); RENBP (Renin-binding protein); REQ (Requiem, apoptosis response zinc finger gene); RFX1 (Regulatory factor X, 1 (influences HLA class II expression)); RFX2 (Regulatory factor X, 2 (influences HLA class II expression)); RFX3 (Regulatory factor X, 3 (influences HLA class II expression)); RFX4 (Regulatory factor X, 4 (influences HLA class II expression)); RFX5 (Regulatory factor X, 5 (influences HLA class II expression)); RFXAP (Regulatory factor X-associated protein); RGS2 (Regulator of G-protein signalling 2, 24kD); RHCE (Rhesus blood group, CcEe antigens); RHD (Rhesus blood group, D antigen); RHO (Rhodopsin (retinitis pigmentosa 4, autosomal dominant)); RMSAI (Regulator of mitotic spindle assembly 1); RN5S1@ (RNA, 5S cluster 1); RNR1 (RNA, ribosomal 1); RNU1A (RNA, U1A small nuclear); RNU2 (RNA, U2 small nuclear); ROM1 (Retinal outer segment membrane protein 1); RP2 (Retinitis pigmentosa 2 (X-linked recessive)); RPE65 (Retinal pigment epithelium-specific protein (65kD)); RPL7A (Ribosomal protein L7a); RPS4X (Ribosomal protein S4, X-linked); RRAD (Ras-related associated with diabetes); RRM1 (Ribonucleotide reductase M1 polypeptide); RSN (Restin (Reed-Steinberg cell-expressed intermediate filament-associated protein)); RTS (Rothmund-Thomson syndrome); RXRB (Retinoid X receptor, beta); RYR1 (Ryanodine receptor 1 (skeletal)); S100A4 (S 100 calcium-binding protein A4 (calcium protein, calvasculin, metastasin, murine placental homolog)); S100A7 (S100 calcium-binding protein A7 (psoriasin 1)); S100A8 (S100 calcium-binding protein A8 (calgranulin A)); SAA1 (Serum amyloid A1); SAG (S-antigen; retina and pineal gland (arrestin)); SAR1 (RasGAP-like with IQ motifs); SCLC1); SCN1B (Sodium channel, voltage-gated, type I, beta polypeptide); SCN4A (Sodium channel, voltage-gated, type IV, alpha polypeptide); SCN5A (Sodium channel, voltage-gated, type V, alpha polypeptide (long (electrocardiographic) QT syndrome 3)); SCNN1G (Sodium channel, nonvoltage-gated 1, gamma); SCYA1 (Small inducible cytokine A1 (I-309, homologous to mouse Tca-3)); SCYA11 (Small inducible cytokine subfamily A (Cys-Cys), member 11 (eotaxin)); SCYA13 (Small inducible cytokine subfamily A (Cys-Cys), member 13); SCYA14 (Small inducible cytokine subfamily A (Cys-Cys), member 14); SCYA15 (Small inducible cytokine subfamily A (Cys-Cys), member 15); SCYA16 (Small inducible cytokine subfamily A (Cys-Cys), member 16); SCYA17 (Small inducible cytokine subfamily A (Cys-Cys), member 17); SCYA18 (Small inducible cytokine subfamily A (Cys-Cys), member 18, pulmonary and activation-regulated); SCYA19 (Small inducible cytokine subfamily A (Cys-Cys), member 19); SCYA2 (Small inducible cytokine A2 (monocyte chemotactic protein 1, homologous to mouse Sig-je)); SCYA20 (Small inducible cytokine subfamily A (Cys-Cys), member 20); SCYA21 (Small inducible cytokine subfamily A (Cys-Cys), member 21); SCYA22 (Small inducible cytokine subfamily A (Cys-Cys), member 22); SCYA23 (Small inducible cytokine subfamily A (Cys-Cys), member 23); SCYA24 (Small inducible cytokine subfamily A (Cys-Cys), member 24); SCYA25 (Small inducible cytokine subfamily A (Cys-Cys), member 25); SCYA3 (Small inducible cytokine A3 (homologous to mouse Mip-1a)); SCYA3L1 (Small inducible cytokine A3-like 1); SCYA4 (Small inducible cytokine A4 (homologous to mouse Mip-1b)) SCYA5 (Small inducible cytokine A5 (RANTES)); SCYA7 (Small inducible cytokine A7 (monocyte chemotactic protein 3)); SCYA8 (Small inducible cytokine subfamily A (Cys-Cys), member 8 (monocyte chemotactic protein 2)); SCYB5 (Small inducible cytokine subfamily B (Cys-X-Cys), member 5 (epithelial-derived neutrophil-activating peptide 78)); SCYB6 (Small inducible cytokine subfamily B (Cys-X-Cys), member 6 (granulocyte chemotactic protein 2)); SCYC1 (Small inducible cytokine subfamily C, member 1 (lymphotactin)); SCYD1 (Small inducible cytokine subfamily D (Cys-X3-Cys), member 1 (fractalkine, neurotactin)); SDF1 (Stromal cell-derived factor 1); SDHC (Succinate dehydrogenase complex, subunit C, integral membrane protein, 15kD); SELE (Selectin E (endothelial adhesion molecule 1)); SELL (Selectin L (lymphocyte adhesion molecule 1)); SELP (Selectin P (granule membrane protein 140kD, antigen CD62)); SELPLG (Selectin P ligand); SERK1 (SAPK/Erk kinase 1); SF (Stoltzfus blood group); SFTPA1 (Surfactant, pulmonary-associated protein A1); SFTPA2 (Surfactant, pulmonary-associated protein A2); SFTPB (Surfactant, pulmonary-associated protein B); SFTPD (Surfactant, pulmonary-associated protein D); SGCB (Sarcoglycan, beta (43kD dystrophin-associated glycoprotein)); SGCD (Sarcoglycan, delta (35kD dystrophin-associated glycoprotein)); SGSH (N-sulfoglucosamine sulfohydrolase (sulfamidase)); SH2D1A (SH2 domain protein 1A, Duncan's disease (lymphoproliferative syndrome)); SHH (Sonic hedgehog (Drosophila) homolog); SHMT2 (Serine hydroxymethyltransferase 2 (mitochondrial)); SHOX (Short stature homeobox); SIAH1 (Seven in absentia (Drosophila) homolog 1); SIPA1 (Signal-induced proliferation-associated gene 1); SKIV2L (Superkiller viralicidic activity 2 (S. cerevisiae homolog)-like); SLC12A1 (Solute carrier family 12 (sodium/potassium/chloride transporters), member 1); SLC14A1 (Solute carrier family 14 (urea transporter), member 1 (Kidd blood group)); SLC18A2 (Solute carrier family 18 (vesicular monoamine), member 2); SLC1A5 (Solute carrier family 1 (neutral amino acid transporter), member 5); SLC20A1 (Solute carrier family 20 (phosphate transporter), member 1); SLC20A2 (Solute carrier family 20 (phosphate transporter), member 2); SLC2A1 (Solute carrier family 2 (facilitated glucose transporter), member 1); SLC2A2 (Solute carrier family 2 (facilitated glucose transporter), member 2); SLC2A4 (Solute carrier family 2 (facilitated glucose transporter), member 4); SLC3A1 (Solute carrier family 3 (cystine, dibasic and neutral amino acid transporters, activator of cystine, dibasic and neutral amino acid transport), member 1); SLC4A1 (Solute carrier family 4, anion exchanger, member 1 (erythrocyte membrane protein band 3, Diego blood group) ); SLC5A5 (Solute carrier family 5 (sodium iodide symporter), member 5); SLC6A2 (Solute carrier family 6 (neurotransmitter transporter, noradrenalin), member 2); SLC6A3 (Solute carrier family 6 (neurotransmitter transporter, dopamine), member 3); SLC6A4 (Solute carrier family 6 (neurotransmitter transporter, serotonin), member 4); SLC7A7 (Solute carrier family 7 (cationic amino acid transporter, y+ system), member 7); SLC9A1 (Solute carrier family 9 (sodium/hydrogen exchanger), isoform 1 (antiporter, Na+/H+, amiloride sensitive)); SLEB1 (Systemic lupus erythematosus susceptibility 1); SLPI (Secretory leukocyte protease inhibitor (antileukoproteinase)); SM1 (Schistosoma mansoni, susceptibility/resistance to); SMN1 (Survival of motor neuron 1, telomeric); SNAP23 (Synaptosomal-associated protein, 23kD); SNCG (Synuclein, gamma (breast cancer-specific protein 1)); SNRP70 (Small nuclear ribonucleoprotein 70kD polypeptide (RNP antigen)); SNRPB (Small nuclear ribonucleoprotein polypeptides B and B1); SNRPN (Small nuclear ribonucleoprotein polypeptide N); SOAT1 (Sterol O-acyltransferase (acyl-Coenzyme A: cholesterol acyltransferase) 1); SOD1 (Superoxide dismutase 1, soluble (amyotrophic lateral sclerosis 1 (adult))); SOD2 (Superoxide dismutase 2, mitochondrial); SORL1 (Sortilin-related receptor, L(DLR class) A repeats-containing); SOX10 (SRY (sex-determining region Y)-box 10); SOX4 (SRY (sex determining region Y)-box 4); SPG3A (Spastic paraplegia 3A (autosomal dominant)); SPN (Sialophorin (gpL115, leukosialin, CD43)); SPN (Sialophorin (gpL115, leukosialin, CD43)); SPP1 (Secreted phosphoprotein 1 (osteopontin, bone sialoprotein I, early T-lymphocyte activation 1)); SPTA1 (Spectrin, alpha, erythrocytic 1 (elliptocytosis 2)); SRD5A2 (Steroid-5-alpha-reductase, alpha polypeptide 2 (3-oxo-5 alpha-steroid delta 4-dehydrogenase alpha 2)); SSA2 (Sjogren syndrome antigen A2 (60kD, ribonucleoprotein autoantigen SS-A/Ro)); SSB (Sjogren syndrome antigen B (autoantigen La)); SSTR1 (Somatostatin receptor 1); ST3 (Suppression of tumorigenicity 3); STAM (Signal transducing adaptor molecule (SH3 domain and ITAM motif) 1); STAT1 (Signal transducer and activator of transcription 1, 91kD); STAT2 (Signal transducer and activator of transcription 2, 113kD); STAT3 (Signal transducer and activator of transcription 3 (acute-phase response factor)); STAT4 (Signal transducer and activator of transcription 4); STAT5A (Signal transducer and activator of transcription 5A); STAT6 (Signal transducer and activator of transcription 6, interleukin-4 induced); STATH (Statherin); STATI2 (STAT induced STAT inhibitor-2); STX1B (Syntaxin 1B); SULT1A1 (Sulfotransferase family 1A, phenol-preferring, member 1); SULT1A3 (Sulfotransferase family 1A, phenol-preferring, member 3); SULT2A1 (Sulfotransferase family 2A, dehydroepiandrosterone (DHEA) -preferring, member 1); SUOX (Sulfite oxidase); SUR (Sulfonylurea receptor (hyperinsulinemia)); SURF1 (Surfeit 1); SW (Swann blood group); T (T brachyury (mouse) homolog); TAP1 (Transporter 1, ABC (ATP binding cassette)); TAP2 (Transporter 2, ABC (ATP binding cassette)); TAT (Tyrosine aminotransferase); TAZ (Tafazzin (cardiomyopathy, dilated 3A (X-linked), endocardial fibroelastosis 2; Barth syndrome)); TBG (Thyroxin-binding globulin); TBP (TATA box binding protein); TBX2 (T-box 2); TBXA2R (Thromboxane A2 receptor); TBXAS1 (Thromboxane A synthase 1 (platelet, cytochrome P450, subfamily V)); TCF1 (Transcription factor 1, hepatic; LF-B1, hepatic nuclear factor (HNF1), albumin proximal factor); TCF19 (Transcription factor 19 (SC1)); TCF3 (Transcription factor 3 (E2A immunoglobulin enhancer binding factors E12/E47)); TCF7 (Transcription factor 7 (T-cell specific, HMG-box)); TCF8 (Transcription factor 8 (represses interleukin 2 expression)); TCL1A (T-cell leukemia/lymphoma 1A); TCL4 (T-cell leukemia/lymphoma 4); TCN1 (Transcobalamin I (vitamin B12 binding protein, R binder family)); TCN2 (Transcobalamin II; macrocytic anemia); TCP1 (T-complex 1); TCRA (T-cell receptor, alpha (V,D,J,C)); TCRB (T-cell receptor, beta cluster); TCRB (T-cell receptor, beta cluster); TCRG (T-cell receptor, gamma cluster); TCTE1 (T-complex-associated-testis-expressed 1); TDO2 (Tryptophan 2,3-dioxygenase); TECTA (Tectorin alpha); TERF2 (Telomeric repeat binding factor 2); TF (Transferrin); TFF2 (Trefoil factor 2 (spasmolytic protein 1)); TFPI (Tissue factor pathway inhibitor (lipoprotein-associated coagulation inhibitor)); TFPI2 (Tissue factor pathway inhibitor-2); TFRC (Transferrin receptor (p90, CD71)); TG (Thyroglobulin); TGFB1 (Transforming growth factor, beta 1); TGFB2 (Transforming growth factor, beta 2); TGFB3 (Transforming growth factor, beta 3); TGFBI (Transforming growth factor, beta-induced, 68kD); TGFBR3 (Transforming growth factor, beta receptor III (betaglycan, 300kD)); TGM1 (Transglutaminase 1 (K polypeptide epidermal type I, protein-glutamine-gamma-glutamyltransferase)); TGM2 (Transglutaminase 2 (C polypeptide, protein-glutamine-gamma-glutamyltransferase)); TH (Tyrosine hydroxylase); THBS1 (Thrombospondin 1); THBS2 (Thrombospondin 2); THPO (Thrombopoietin (myeloproliferative leukemia virus oncogene ligand, megakaryocyte growth and development factor)); THRA (Thyroid hormone receptor, alpha (avian erythroblastic leukemia viral (v-erb-a) oncogene homolog)); THRB (Thyroid hormone receptor, beta (avian erythroblastic leukemia viral (v-erb-a) oncogene homolog 2)); THY1 (Thy-1 cell surface antigen); TIEG (TGFB inducible early growth response); TIMP3 (Tissue inhibitor of metalloproteinase 3 (Sorsby fundus dystrophy, pseudoinflammatory)); TK1 (Thymidine kinase 1, soluble); TKT (Transketolase (Wemicke-Korsakoff syndrome)); TLR1 (Toll-like receptor 1); TLR2 (Toll-like receptor 2); TLR3 (Toll-like receptor 3); TLR4 (Toll-like receptor 4); TLR5 (Toll-like receptor 5); TM4SF7 (Transmembrane 4 superfamily member 7); TMEM1 (Transmembrane protein 1); TNF (Tumor necrosis factor (TNF superfamily, member 2)); TNFAIP2 (Tumor necrosis factor, alpha-induced protein 2); TNFAIP6 (Tumor necrosis factor, alpha-induced protein 6); TNFRSF11B (Tumor necrosis factor receptor superfamily, member 11b (osteoprotegerin)); TNFRSF12 (Tumor necrosis factor receptor superfamily, member 12 (translocating chain-association membrane protein)); TNFRSF14 (Tumor necrosis factor receptor superfamily, member 14 (herpesvirus entry mediator)); TNFRSF17 (Tumor necrosis factor receptor superfamily, member 17); TNFRSF1A (Tumor necrosis factor receptor superfamily, member 1A); TNFRSF 1 B (Tumor necrosis factor receptor superfamily, member 1B); TNFRSF5 (Tumor necrosis factor receptor superfamily, member 5); TNFRSF6 (Tumor necrosis factor receptor superfamily, member 6); TNFRSF6B (Tumor necrosis factor receptor superfamily, member 6b, decoy); TNFRSF7 (Tumor necrosis factor receptor superfamily, member 7); TNFRSF9 (Tumor necrosis factor receptor superfamily, member 9); TNFSF11 (Tumor necrosis factor (ligand) superfamily, member 11); TNFSF12 (Tumor necrosis factor (ligand) superfamily, member 12); TNFSF14 (Tumor necrosis factor (ligand) superfamily, member 14); TNFSF5 (Tumor necrosis factor (ligand) superfamily, member 5); TNFSF6 (Tumor necrosis factor (ligand) superfamily, member 6); TNNT2 (Troponin T2, cardiac); TP53 (Tumor protein p53 (Li-Fraumeni syndrome)); TP73 (Tumor protein p73); TPH (Tryptophan hydroxylase (tryptophan 5-monooxygenase)); TPI1 (Triosephosphate isomerase 1); TPM1 (Tropomyosin 1 (alpha)); TPMT (Thiopurine S-methyltransferase); TPO (Thyroid peroxidase); TPT1 (Tumor protein, translationally-controlled 1); TRAF1 (TNF receptor-associated factor 1); TRAF1 (TNF receptor-associated factor 1); TRAF2 (TNF receptor-associated factor 2); TRAF3 (TNF receptor-associated factor 3); TRAF4 (TNF receptor-associated factor 4); TRAF5 (TNF receptor-associated factor 5); TRAF6 (TNF receptor-associated factor 6); TRP1 (TRNA proline 1); TSHB (Thyroid stimulating hormone, beta); TSHR (Thyroid stimulating hormone receptor); TSSC3 (Tumor suppressing subtransferable candidate 3); TST (Thiosulfate sulfurtransferase (rhodanese)); TSTA3 (Tissue specific transplantation antigen P35B); TTIM1 (T-cell tumor invasion and metastasis 1); TTR (Transthyretin (prealbumin, amyloidosis type I)); TUB (Tubby (mouse) homolog); TUBA3 (Tubulin, alpha, brain-specific); TUBAL1 (Tubulin, alpha-like 1); TUBB (Tubulin, beta polypeptide); TWIST (Twist (Drosophila) homolog); TXN (Thioredoxin); U2AF1 (U2(RNU2) small nuclear RNA auxillary factor 1 (non-standard symbol)); UBC (Ubiquitin C); UBE1 (Ubiquitin-activating enzyme E1 (A1S9T and BN75 temperature sensitivity complementing)); UBE2B (Ubiquitin-conjugating enzyme E2B (RAD6 homolog)); UBE2I (Ubiquitin-conjugating enzyme E2I (homologous to yeast UBC9)); UBE2V2 (Ubiquitin-conjugating enzyme E2 variant 2); UBE3A (Ubiquitin protein ligase E3A (human papilloma virus E6-associated protein, Angelman syndrome)); UBL1 (Ubiquitin-like 1 (sentrin)); UCP2 (Uncoupling protein 2 (mitochondrial, proton carrier)); UCP3 (Uncoupling protein 3 (mitochondrial, proton carrier)); UFS (Urofacial syndrome); UGB (Uteroglobin); UGDH (UDP-glucose dehydrogenase); UGT1 (UDP glycosyltransferase 1); UMPK (Uridine monophosphate kinase); UMPS (Uridine monophosphate synthetase (orotate phosphoribosyl transferase and); orotidine-5'-decarboxylase)); UP (Uridine phosphorylase); UPK1B (Uroplakin 1B); UROD (Uroporphyrinogen decarboxylase); UROS (Uroporphyrinogen III synthase (congenital erythropoietic porphyria)); USH2A (Usher syndrome 2A (autosomal recessive, mild)); USP7 (Ubiquitin specific protease 7 (herpes virus-associated)); VASP (Vasodilator-stimulated phosphoprotein); VCAM1 (Vascular cell adhesion molecule 1); VDAC1 (Voltage-dependent anion channel 1); VDR (Vitamin D (1,25- dihydroxyvitamin D3) receptor); VHL (Von Hippel-Lindau syndrome); VMD2 (Vitelliform macular dystrophy (Best disease, bestrophin)); VPREB1 (Pre-B lymphocyte gene 1 (non-standard provisional symbol)); VPREB2 (Pre-B lymphocyte-specific protein-2); VSD1 (Ventricular septal defect 1); VTN (Vitronectin (serum spreading factor, somatomedin B, complement S-protein)); VWF (Von Willebrand factor); WAS (Wiskott-Aldrich syndrome (ecezema-thrombocytopenia)); WEE1 (Wee1+ (S. pombe) homolog); WFS (Wolfram syndrome); WT1 (Wilms tumor 1); WT3 (Wilms tumor-3); WWS (Wieacker-Wolff syndrome); XBP1 (X-box binding protein 1); XG (Xg blood group (pseudoautosomal boundary-divided on the X chromosome)); XGR (Expression of XG and MIC2 on erythrocytes); XK (Kell blood group precursor (McLeod phenotype)); XPA (Xeroderma pigmentosum, complementation group A); XPC (Xeroderma pigmentosum, complementation group C); XPNPEPL (X-prolyl aminopeptidase (aminopeptidase P)-like); XRCC1 (X-ray repair complementing defective repair in Chinese hamster cells 1); XRCC2 (X-ray repair complementing defective repair in Chinese hamster cells 2); XRCC3 (X-ray repair complementing defective repair in Chinese hamster cells 3); YB1 (Major histocompatibility complex, class II, Y box-binding protein I; DNA-binding protein B); ZFP161 (Zinc finger protein homologous to Zfp161 in mouse); ZFP36 (Zinc finger protein homologous to Zfp-36 in mouse); ZFY (Zinc finger protein, Y-linked); ZNF121 (Zinc finger protein 121 (clone ZHC32)); ZRK (Zona pellucida receptor tyrosine kinase, 95kD);

### Metabolism

ACAT1 (Acetyl-Coenzyme A acetyltransferase 1 (acetoacetyl Coenzyme A thiolase); ACAT2 (Acetyl-Coenzyme A acetyltransferase 2 (acetoacetyl Coenzyme A thiolase); ACATN (Acetyl-Coenzyme A transporter; BCAT1 (Branched chain aminotransferase 1, cytosolic; CRAT (Carnitine acetyltransferase; DIA4 (Diaphorase (NADH/NADPH) (cytochrome b-5 reductase); DUSP2 (Dual specificity phosphatase 2; EPHX1 (Epoxide hydrolase 1, microsomal (xenobiotic); EPHX2 (Epoxide hydrolase 2, cytoplasmic; GATM (Glycine amidinotransferase (L-arginine:glycine amidinotransferase); GJA4 (Gap junction protein, alpha 4, 37kD (connexin 37); HADHSC (L-3-hydroxyacyl-Coenzyme A dehydrogenase, short chain; HK1 (Hexokinase 1; HK3 (Hexokinase 3 (white cell); HMBS (Hydroxymethylbilane synthase; IARS (Isoleucine-tRNA synthetase; NAT1 (N-acetyltransferase 1 (arylamine N-acetyltransferase); OAT (Ornithine aminotransferase (gyrate atrophy); OTC (Ornithine carbamoyltransferase; PCCA (Propionyl Coenzyme A carboxylase, alpha polypeptide; PCCB (Propionyl Coenzyme A carboxylase, beta polypeptide; PDHA1 (Pyruvate dehydrogenase (lipoamide) alpha 1; QDPR (Quinoid dihydropteridine reductase; SGSH (N-sulfoglucosamine sulfohydrolase (sulfamidase); SHBG (Sex hormone-binding globulin; SHMT2 (Serine hydroxymethyltransferase 2 (mitochondrial); SLC7A1 (Solute carrier family 7 (cationic amino acid transporter, y+ system), member 1; SLC7A2 (Solute carrier family 7 (cationic amino acid transporter, y+ system), member 2; SLC7A4 (Solute carrier family 7 (cationic amino acid transporter, y+ system), member 4; SOAT1 (Sterol O-acyltransferase (acyl-Coenzyme A: cholesterol acyltransferase) 1; SOAT2 (Sterol O-acyltransferase 2; SORD (Sorbitol dehydrogenase; TPI1 (Triosephosphate isomerase 1; TYMS (Thymidylate synthetase; TYR (Tyrosinase (oculocutaneous albinism IA); TYRP1 (Tyrosinase-related protein 1; UGT2B 17 (UDP glycosyltransferase 2 family, polypeptide B17; UGT2B7 (UDP glycosyltransferase 2 family, polypeptide B7; UGTREL1 (UDP-galactose transporter related;

### Metastasis

ACTG1 (Actin, gamma 1); ADD3 (Adducin 3 (gamma)); ALCAM (Activated leucocyte cell adhesion molecule); ANK1 (Ankyrin 1, erythrocytic); ANPEP (Alanyl (membrane) aminopeptidase (aminopeptidase N, aminopeptidase M, microsomal aminopeptidase, CD13, p150)); BTN (Butyrophilin); CD14 (CD14 antigen); CD 19 (CD19 antigen); CD1D (CD1D antigen, d polypeptide); CD2 (CD2 antigen (p50), sheep red blood cell receptor); CD20 (CD20 antigen); CD22 (CD22 antigen); CD33 (CD33 antigen (gp67)); CD34 (CD34 antigen); CD37 (CD37 antigen); CD38 (CD38 antigen (p45)); CD39 (CD39 antigen); CD4 (CD4 antigen (p55)); CD44 (CD44 antigen (homing function and Indian blood group system)); CD47 (CD47 antigen (Rh-related antigen, integrin-associated signal transducer)); CD48 (CD48 antigen (B-cell membrane protein)); CD53 (CD53 antigen); CD58 (CD58 antigen, (lymphocyte function-associated antigen 3)); CD59 (CD59 antigen p18-20 (antigen identified by monoclonal antibodies 16.3A5, EJ16, EJ30, EL32 and G344)); CD63 (CD63 antigen (melanoma 1 antigen)); CD68 (CD68 antigen); CD7 (CD7 antigen (p41)); CD72 (CD72 antigen); CD8A (CD8 antigen, alpha polypeptide (p32)); CD9 (CD9 antigen (p24)); CD97 (CD97 antigen); CDH13 (Cadherin 13, H-cadherin (heart)); CDH17 (Cadherin 17, LI cadherin (liver-intestine)); CDH2 (Cadherin 2, N-cadherin (neuronal)); CDH4 (Cadherin 4, R-cadherin (retinal)); CDH5 (Cadherin 5, VE-cadherin (vascular epithelium)); CDW52 (CDW52 antigen (CAMPATH-1 antigen)); CLTB (Clathrin, light polypeptide (Lcb)); DAF (Decay accelerating factor for complement (CD55, Cromer blood group system)); DPP4 (Dipeptidylpeptidase IV (CD26, adenosine deaminase complexing protein 2)); ENG (Endoglin (Osler-Rendu-Weber syndrome 1)); F3 (Coagulation factor III (thromboplastin, tissue factor)); FAP (Fibroblast activation protein, alpha); FAT (FAT tumor suppressor (Drosophila) homolog); FLNA (Filamin A, alpha (actin-binding protein-280)); FN1 (Fibronectin 1); GJA4 (Gap junction protein, alpha 4, 37kD (connexin 37)); HMMR (Hyaluronan-mediated motility receptor (RHAMM)); ICAM1 (Intercellular adhesion molecule 1 (CD54), human rhinovirus receptor); ICAM2 (Intercellular adhesion molecule 2); ICAM3 (Intercellular adhesion molecule 3); ITGA2 (Integrin, alpha 2 (CD49B, alpha 2 subunit of VLA-2 receptor)); ITGA3 (Integrin, alpha 3 (antigen CD49C, alpha 3 subunit of VLA-3 receptor)); ITGA4 (Integrin, alpha 4 (antigen CD49D, alpha 4 subunit of VLA-4 receptor)); ITGA5 (Integrin, alpha 5 (fibronectin receptor, alpha polypeptide)); ITGA6 (Integrin, alpha 6); ITGA7 (Integrin, alpha 7); ITGA9 (Integrin, alpha 9); ITGAE (Integrin, alpha E (antigen CD103, human mucosal lymphocyte antigen 1; alpha polypeptide)); ITGAL (Integrin, alpha L (antigen CD11A (p180), lymphocyte function-associated antigen 1; alpha polypeptide)); ITGAM (Integrin, alpha M (complement component receptor 3, alpha; also known as CD11b (p170), macrophage antigen alpha polypeptide)); ITGAV (Integrin, alpha V (vitronectin receptor, alpha polypeptide, antigen CD51)); ITGAX (Integrin, alpha X (antigen CD11C (p150), alpha polypeptide)); ITGB1 (Integrin, beta 1 (fibronectin receptor, beta polypeptide, antigen CD29 includes MDF2, MSK12)); ITGB2 (Integrin, beta 2 (antigen CD18 (p95), lymphocyte function-associated antigen 1; macrophage antigen 1 (mac-1) beta subunit)); ITGB3 (Integrin, beta 3 (platelet glycoprotein IIIa, antigen CD61)); ITGB4 (Integrin, beta 4); ITGB5 (Integrin, beta 5); ITGB7 (Integrin, beta 7); ITGB8 (Integrin, beta 8); JAG1 (Jagged1 (Alagille syndrome)); JAG2 (Jagged 2); KAI1 (Kangai 1 (suppression of tumorigenicity 6, prostate; CD82 antigen (R2 leukocyte antigen, antigen detected by monoclonal and antibody IA4))); KPNB1 (Karyopherin (importin) beta 1); LAG3 (Lymphocyte-activation gene 3); LY64 (Lymphocyte antigen 64 (mouse) homolog, radioprotective, 105kD); LYZ (Lysozyme (renal amyloidosis)); MAP1B (Microtubule-associated protein 1B); MDU1 (Antigen identified by monoclonal antibodies 4F2, TRA1.10, TROP4, and T43); MIC2 (Antigen identified by monoclonal antibodies 12E7, F21 and 013); MICA (MHC class I polypeptide-related sequence A); MME (Membrane metallo-endopeptidase (neutral endopeptidase, enkephalinase, CALLA, CD10)); MYL2 (Myosin, light polypeptide 2, regulatory, cardiac, slow); MYL3 (Myosin, light polypeptide 3, alkali; ventricular, skeletal, slow); NCAM1 (Neural cell adhesion molecule 1); NEO1 (Neogenin (chicken) homolog 1); NME3 (Non-metastatic cells 3, protein expressed in); PCDH1 (Protocadherin 1 (cadherin-like 1)); PDNP1 (Phosphodiesterase I/nucleotide pyrophosphatase 1 (homologous to mouse Ly-41 antigen)); PECAM1 (Platelet/endothelial cell adhesion molecule (CD31 antigen)); PKD1 (Polycystic kidney disease I (autosomal dominant)); PTCH (Patched (Drosophila) homolog); RSN (Restin (Reed-Steinberg cell-expressed intermediate filament-associated protein)); RTN1 (Reticulon 1); SDC1 (Syndecan 1); SDC2 (Syndecan 2 (heparan sulfate proteoglycan 1, cell surface-associated, fibroglycan)); SDC4 (Syndecan 4 (amphiglycan, ryudocan)); SELE (Selectin E (endothelial adhesion molecule 1)); SELL (Selectin L (lymphocyte adhesion molecule 1)); SELP (Selectin P (granule membrane protein 140kD, antigen CD62)); SELPLG (Selectin P ligand); SIAT1 (Sialyltransferase 1 (beta-galactoside alpha-2,6-sialytransferase)); SLAM (Signaling lymphocytic activation molecule); SPTA1 (Spectrin, alpha, erythrocytic 1 (elliptocytosis 2)); SPTB (Spectrin, beta, erythrocytic (includes sperocytosis, clinical type I)); ST2 (Suppression of tumorigenicity 2); TBCC (Tubulin-specific chaperone c); THBS1 (Thrombospondin 1); TM4SF2 (Transmembrane 4 superfamily member 2); TM4SF3 (Transmembrane 4 superfamily member 3); TNFSF4 (Tumor necrosis factor (ligand) superfamily, member 4 (tax-transcriptionally activated glycoprotein 1, 34kD)); TNFSF5 (Tumor necrosis factor (ligand) superfamily, member 5); TNFSF7 (Tumor necrosis factor (ligand) superfamily, member 7); TTN (Titin); TUBA1 (Tubulin, alpha 1 (testis specific)); TUBG (Tubulin, gamma polypeptide); VCAM1 (Vascular cell adhesion molecule 1); VCL (Vinculin); VIM (Vimentin);

### Miscellaneous

AREG (Amphiregulin (schwannoma-derived growth factor)); BCGF1 (B-cell growth factor 1 (12D)); CTGF (Connective tissue growth factor); CTGF-L (Connective tissue growth factor-like protein); ECGF1 (Endothelial cell growth factor 1 (platelet-derived)); EGF (Epidermal growth factor); EPS15 (Epidermal growth factor receptor pathway substrate 15); EPS8 (Epidermal growth factor receptor pathway substrate 8); FGF1 (Fibroblast growth factor 1 (acidic)); FGF10 (Fibroblast growth factor 10); FGF11 (Fibroblast growth factor 11); FGF12B (Fibroblast growth factor 12B); FGF13 (Fibroblast growth factor 13); FGF14 (Fibroblast growth factor 14); FGF16 (Fibroblast growth factor 16); FGF6 (Fibroblast growth factor 6); FGF7 (Fibroblast growth factor 7 (keratinocyte growth factor)); FGF8 (Fibroblast growth factor 8 (androgen-induced)); FGF9 (Fibroblast growth factor 9 (glia-activating factor)); FGFR1 (Fibroblast growth factor receptor 1 (fms-related tyrosine kinase 2, Pfeiffer syndrome)); FGFR2 (Fibroblast growth factor receptor 2 (bacteria-expressed kinase, keratinocyte growth factor receptor, craniofacial dysostosis 1, Crouzon syndrome, Pfeiffer syndrome, Jackson-Weiss syndrome)); FGFR3 (Fibroblast growth factor receptor 3 (achondroplasia, thanatophoric dwarfism)); FGFR4 (Fibroblast growth factor receptor 4); FIBP (Fibroblast growth factor (acidic) intracellular binding protein); FIGF (C-fos induced growth factor (vascular endothelial growth factor D)); HDGF (Hepatoma-derived, growth factor (high-mobility group protein 1-like)); IGF1 (Insulin-like growth factor 1 (somatomedin C)); IGF2 (Insulin-like growth factor 2 (somatomedin A)); IGFALS (Insulin-like growth factor binding protein, acid labile subunit); NGFB (Nerve growth factor, beta polypeptide); PDGFA (Platelet-derived growth factor alpha polypeptide); PDGFRB (Platelet-derived growth factor receptor, beta polypeptide); PGF (Placental growth factor, vascular endothelial growth factor-related protein); PTN (Pleiotrophin (heparin binding growth factor 8, neurite growth-promoting factor 1)); QSCN6 (Quiescin Q6); TAK1 (Transforming growth factor beta-activated kinase 1); TIEG (TGFB inducible early growth response); TIEG2 (TGFB inducible early growth response 2); VEGF (Vascular endothelial growth factor); VEGFB (Vascular endothelial growth factor B); VGF (VGF nerve growth factor inducible); VTN (Vitronectin (serum spreading factor, somatomedin B, complement S-protein)); A2M (Alpha-2-macroglobulin); ADAM17 (A disintegrin and metalloproteinase domain 17 (tumor necrosis factor, alpha, converting enzyme)); ADSL (Adenylosuccinate lyase); AOAH (Acyloxyacyl hydrolase (neutrophil)); AOX1 (Aldehyde oxidase 1); ATP5J (ATP synthase, H+ transporting, mitochondrial F0 complex, subunit F6); BCAT2 (Branched chain aminotransferase 2, mitochondrial); BCKDHA (Branched chain keto acid dehydrogenase E1, alpha polypeptide (maple syrup urine disease)); BCL6 (B-cell CLL/lymphoma 6 (zinc finger protein 51)); BCL7 (B-cell CLL/lymphoma 7); BHMT (Betaine-homocysteine methyltransferase); BSG (Basigin); BTG1 (B-cell translocation gene 1, anti-proliferative); C5 (Complement component 5); CAD (Carbamoyl-phosphate synthetase 2, aspartate transcarbamylase, and dihydroorotase); CATR1 (CATR tumorigenicity conversion 1); CDR1 (Cerebellar degeneration-related protein (34kD)); CHGB (Chromogranin B (secretogranin 1)); CHIT1 (Chitinase 1); COL9A2 (Collagen, type IX, alpha 2); COX10 (Cytochrome c oxidase subunit X (heme A: famesyltransferase)); COX11 (Cytochrome c oxidase subunit 11); COX15 (Cytochrome c oxidase subunit 15); COX17 (Human homolog of yeast mitochondrial copper recruitment gene); COX5A (Cytochrome c oxidase subunit Va); COX5B (Cytochrome c oxidase subunit Vb); COX6A1 (Cytochrome c oxidase subunit VIa polypeptide 1); COX6A2 (Cytochrome c oxidase subunit VIa polypeptide 2); COX6B (Cytochrome c oxidase subunit VIb); COX6C (Cytochrome c oxidase subunit VIc); COX7C (Cytochrome c oxidase subunit VIIc); COX7RP (Cytochrome c oxidase subunit VII-related protein); COX8 (Cytochrome c oxidase subunit VIII); CPA1 (Carboxypeptidase A1 (pancreatic)); CRHBP (Corticotropin releasing hormone-binding protein); CRIP2 (Cysteine-rich protein 2); CTH (Cystathionase (cystathionine gamma-lyase)); CTSB (Cathepsin B); CTSL (Cathepsin L); CYP (Clk-associating RS-cyclophilin); DARS (Aspartyl-tRNA synthetase); DEFA1 (Defensin, alpha 1, myeloid-related sequence); DSCAM (Down syndrome cell adhesion molecule); DUSP2 (Dual specificity phosphatase 2); DYT1 (Dystonia 1, torsion (autosomal dominant)); EDN1 (Endothelin 1); EGFL2 (EGF-like-domain, multiple 2); F5 (Coagulation factor V (proaccelerin, labile factor)); FMO1 (Flavin containing monooxygenase 1); FUS (Fusion, derived from t(12;16) malignant liposarcoma); FVT1 (Follicular lymphoma variant translocation 1); GARS (Glycyl-tRNA synthetase); GZMB (Granzyme B (granzyme 2, cytotoxic T-lymphocyte-associated serine esterase 1)); GZMK (Granzyme K (serine protease, granzyme 3; tryptase II)); HGFAC (HGF activator); HRMT1L2 (HMT1 (hnRNP methyltransferase, S. cerevisiae)-like 2); HSJ1 (Heat shock protein, neuronal DNAJ-like 1); HSJ2 (Heat shock protein, DNAJ-like 2); IDUA (Iduronidase, alpha-L-); IGFBP10 (Insulin-like growth factor binding protein 10); LDHA (Lactate dehydrogenase A); LDHB (Lactate dehydrogenase B); LGALS1 (Lectin, galactoside-binding, soluble, I (galectin 1)); LMO1 (LIM domain only 1 (rhombotin 1)); LNPEP (Leucyl/cystinyl aminopeptidase); LPP (LIM domain-containing preferred translocation partner in lipoma); MANA2 (Mannosidase, alpha type II); MAOA (Monoamine oxidase A); MFNG (Manic fringe (Drosophila) homolog); MMP-20 (Enamelysin); MMP10 (Matrix metalloproteinase 10 (stromelysin 2)); MMP19 (Matrix metalloproteinase 19); MMP8 (Matrix metalloproteinase 8 (neutrophil collagenase)); MT7SDNA (7S DNA); NB (Neuroblastoma (neuroblastoma suppressor)); NB4S (Neuroblastoma stage 4S gene); NPAT (Nuclear protein, ataxia-telangiectasia locus); OAS2 (2'-5'oligoadenylate synthetase 2); ORCTL3 (Organic cationic transporter-like 3); PDHA1 (Pyruvate dehydrogenase (lipoamide) alpha 1); PGAM1 (Phosphoglycerate mutase 1 (brain)); PITX2 (Paired-like homeodomain transcription factor 2); PRNP (Prion protein (p27-30) (Creutzfeld-Jakob disease, Gerstmann-Strausler-Scheinker syndrome, fatal familial insomnia)); PRSM1 (Protease, metallo, 1, 33kD); PTGS2 (Prostaglandin-endoperoxide synthase 2 (prostaglandin G/H synthase and cyclooxygenase)); RAD50 (RAD50 (S. cerevisiae) homolog); RAD51C (RAD51 (S. cerevisiae) homolog C); RCN2 (Reticulocalbin 2, EF-hand calcium binding domain); SET (SET translocation (myeloid leukemia-associated)); SIAT4A (Sialyltransferase 4A (beta-galactosidase alpha-2,3-sialytransferase)); SPARC (Secreted protein, acidic, cysteine-rich (osteonectin)); TAL1 (T-cell acute lymphocytic leukemia 1); TBG (Thyroxin-binding globulin); TFF1 (Trefoil factor 1 (breast cancer, estrogen-inducible sequence expressed in)); TIMP4 (Tissue inhibitor of metalloproteinase 4); TSC1 (Tuberous sclerosis 1); TSC2 (Tuberous sclerosis 2); UCP2 (Uncoupling protein 2 (mitochondrial, proton carrier)); UROS (Uroporphyrinogen III synthase (congenital erythropoietic porphyria)); VWF (Von Willebrand factor); ANT2 (Adenine nucleotide translocator 2 (fibroblast)); BSEP (Bile salt export pump (ABC member 16, MDR/TAP subfamily)); GJB1 (Gap junction protein, beta 1, 32kD (connexin 32, Charcot-Marie-Tooth neuropathy, X-linked)); KCNN3 (Potassium intermediate/small conductance calcium-activated channel, subfamily N, member 3); OCLN (Occludin); PGY3 (P glycoprotein 3/multiple drug resistance 3); SLC2A3 (Solute carrier family 2 (facilitated glucose transporter), member 3); SLC2A5 (Solute carrier family 2 (facilitated glucose transporter), member 5); TAP1 (Transporter 1, ABC (ATP binding cassette)); TAPBP (TAP binding protein (tapasin)); ARHGDIB (Rho GDP dissociation inhibitor (GDI) beta); ATRX (Alpha thalassemia/mental retardation syndrome X-linked); BMZF2 (Zinc finger 2, bone marrow); BMZF3 (Bone marrow zinc finger 3); BPI (Bactericidal/permeability-increasing protein); CD14 (CD14 antigen); EPB41 (Erythrocyte membrane protein band 4.1 (elliptocytosis 1, RH-linked)); EPB42 (Erythrocyte membrane protein band 4.2); GATA1 (GATA-binding protein 1 (globin transcription factor 1)); GATA2 (GATA-binding protein 2); GATA3 (GATA-binding protein 3); GATA4 (GATA-binding protein 4); GATA6 (GATA-binding protein 6); GZMA (Granzyme A (granzyme 1, cytotoxic T-lymphocyte-associated serine esterase 3)); HBA1 (Hemoglobin, alpha 1); HBB (Hemoglobin, beta); HBG1 (Hemoglobin, gamma A); HBZ (Hemoglobin, zeta); HCF2 (Heparin cofactor II); HHEX (Hematopoietically expressed homeobox); HK2 (Hexokinase 2); HP (Haptoglobin); HPS (Hermansky-Pudlak syndrome); HPX (Hemopexin); LAF4 (Lymphoid nuclear protein related to AF4); LCP1 (Lymphocyte cytosolic protein 1 (L-plastin)); LRMP (Lymphoid-restricted membrane protein); MAL (Mal, T-cell differentiation protein); MLL (Myeloid/lymphoid or mixed-lineage leukemia (trithorax (Drosophila) homolog)); MLL2 (Myeloid/lymphoid or mixed-lineage leukemia 2); MLLT1 (Myeloid/lymphoid or mixed-lineage leukemia (trithorax (Drosophila) homolog); translocated to, 1); MLLT2 (Myeloid/lymphoid or mixed-lineage leukemia (trithorax (Drosophila) homolog); translocated to, 2); MLLT3 (Myeloid/lymphoid or mixed-lineage leukemia (trithorax (Drosophila) homolog); translocated to, 3); MLLT4 (Myeloid/lymphoid or mixed-lineage leukemia (trithorax (Drosophila) homolog); translocated to, 4); MLLT6 (Myeloid/lymphoid or mixed-lineage leukemia (trithorax (Drosophila) homolog); translocated to, 6); MLLT7 (Myeloid/lymphoid or mixed-lineage leukemia (trithorax (Drosophila) homolog); translocated to, 7); MPO (Myeloperoxidase); MYD88 (Myeloid differentiation primary response gene (88)); POU2F1 (POU domain, class 2, transcription factor 1); POU2F2 (POU domain, class 2, transcription factor 2); POU3F1 (POU domain, class 3, transcription factor 1); SCYC1 (Small inducible cytokine subfamily C, member 1 (lymphotactin)); TNFRSF17 (Tumor necrosis factor receptor superfamily, member 17); VWF (Von Willebrand factor);

### Pharmacology

ALDH1 (Aldehyde dehydrogenase 1, soluble); ALDH10 (Aldehyde dehydrogenase 10 (fatty aldehyde dehydrogenase)); ALDH2 (Aldehyde dehydrogenase 2, mitochondrial); ALDH3 (Aldehyde dehydrogenase 3); ALDH6 (Aldehyde dehydrogenase 6); ALDH7 (Aldehyde dehydrogenase 7); ALDH8 (Aldehyde dehydrogenase 8); ANT2 (Adenine nucleotide translocator 2 (fibroblast)); APEX (APEX nuclease (multifunctional DNA repair enzyme)); BCHE (Butyrylcholinesterase); BLMH (Bleomycin hydrolase); CAT (Catalase); CDA (Cytidine deaminase); CYP11A (Cytochrome P450, subfamily XIA (cholesterol side chain cleavage)); CYP11B1 (Cytochrome P450, subfamily XIB (steroid 11-beta-hydroxylase), polypeptide 1); CYP11B2 (Cytochrome P450, subfamily XIB (steroid 11-beta-hydroxylase), polypeptide 2); CYP17 (Cytochrome P450, subfamily XVII (steroid 17-alpha-hydroxylase), adrenal hyperplasia); CYP19 (Cytochrome P450, subfamily XIX (aromatization of androgens)); CYP1A1 (Cytochrome P450, subfamily I (aromatic compound-inducible), polypeptide 1); CYP1A2 (Cytochrome P450, subfamily I (aromatic compound-inducible), polypeptide 2); CYP1B1 (Cytochrome P450, subfamily I (dioxin-inducible), polypeptide 1 (glaucoma 3, primary infantile)); CYP21 (Cytochrome P450, subfamily XXI (steroid 21-hydroxylase, congenital adrenal hyperplasia)); CYP27A1 (Cytochrome P450, subfamily XXVIIA (steroid 27-hydroxylase, cerebrotendinous xanthomatosis), polypeptide 1); CYP2A6 (Cytochrome P450, subfamily IIA (phenobarbital-inducible), polypeptide 6); CYP2B (Cytochrome P450, subfamily IIB (phenobarbital-inducible)); CYP2B6 (Cytochrome P450, subfamily IIB (phenobarbital-inducible), polypeptide 6); CYP2C18 (Cytochrome P450, subfamily IIC (mephenytoin 4-hydroxylase), polypeptide 18); CYP2C19 (Cytochrome P450, subfamily IIC (mephenytoin 4-hydroxylase)); CYP2C9 (Cytochrome P450, subfamily IIC (mephenytoin 4-hydroxylase), polypeptide 9); CYP2D6 (Cytochrome P450, subfamily IID (debrisoquine, sparteine, etc., - metabolizing), polypeptide 6); CYP2E (Cytochrome P450, subfamily IIE (ethanol-inducible)); CYP2F1 (Cytochrome P450, subfamily IIF, polypeptide 1); CYP2J2 (Cytochrome P450, subfamily IIJ (arachidonic acid epoxygenase) polypeptide 2); CYP3A3 (Cytochrome P450, subfamily IIIA (niphedipine oxidase), polypeptide 3); CYP3A5 (Cytochrome P450, subfamily IIIA (niphedipine oxidase), polypeptide 5); CYP3A7 (Cytochrome P450, subfamily IIIA, polypeptide 7); CYP4A11 (Cytochrome P450, subfamily IVA, polypeptide 11); CYP4F3 (Cytochrome P450, subfamily IVF, polypeptide 3 (leukotriene B4 omega hydroxylase)); CYP51 (Cytochrome P450, 51 (lanosterol 14-alpha-demethylase)); DCK (Deoxycytidine kinase); DGUOK (Deoxyguanosine kinase); DHFR (Dihydrofolate reductase); DIA4 (Diaphorase (NADH/NADPH) (cytochrome b-5 reductase)); DPYD (Dihydropyrimidine dehydrogenase); EPHX1 (Epoxide hydrolase 1, microsomal (xenobiotic)); EPHX2 (Epoxide hydrolase 2, cytoplasmic); GJB1 (Gap junction protein, beta 1, 32kD (connexin 32, Charcot-Marie-Tooth neuropathy, X-linked)); GLRX (Glutaredoxin (thioltransferase)); GPX1 (Glutathione peroxidase 1); GPX2 (Glutathione peroxidase 2 (gastrointestinal)); GPX3 (Glutathione peroxidase 3 (plasma)); GPX4 (Glutathione peroxidase 4 (phospholipid hydro-peroxidase)); GSR (Glutathione reductase); GSS (Glutathione synthetase); GSTA2 (Glutathione S-transferase A2); GSTA3 (Glutathione S-transferase A3); GSTM1 (Glutathione S-transferase M1); GSTM2 (Glutathione S-transferase M2 (muscle)); GSTM3 (Glutathione S-transferase M3 (brain)); GSTM4 (Glutathione S-transferase M4); GSTM5 (Glutathione S-transferase M5); GSTP1 (Glutathione S-transferase pi); GSTT1 (Glutathione S-transferase theta 1); GSTT2 (Glutathione S-transferase theta 2); GSTTLp28 (Glutathione-S-transferase like); GSTZ1 (Glutathione S-transferase Zeta 1 (maleylacetoacetate isomerase)); HPRT1 (Hypoxanthine phosphoribosyltransferase 1 (Lesch-Nyhan syndrome)); IMPDH1 (IMP (inosine monophosphate) dehydrogenase 1); IMPDH2 (IMP (inosine monophosphate) dehydrogenase 2); KCNN3 (Potassium intermediate/small conductance calcium-activated channel, subfamily N, member 3); MGST1 (Microsomal glutathione S-transferase 1); MGST2 (Microsomal glutathione S-transferase 2); MGST3 (Microsomal glutathione S-transferase 3); MRP1 (Multiple drug resistance protein 1); NAT1 (N-acetyltransferase 1 (arylamine N-acetyltransferase)); NMOR2 (NAD(P)H menadione oxidoreductase 2, dioxin-inducible); OCLN (Occludin); PGY1 (P glycoprotein 1/multiple drug resistance 1); PGY3 (P glycoprotein 3/multiple drug resistance 3); RRM1 (Ribonucleotide reductase M1 polypeptide); RRM2 (Ribonucleotide reductase M2 polypeptide); SLC2A3 (Solute carrier family 2 (facilitated glucose transporter), member 3); SLC2A5 (Solute carrier family 2 (facilitated glucose transporter), member 5); SOD1 (Superoxide dismutase 1, soluble (amyotrophic lateral sclerosis 1 (adult))); SOD2 (Superoxide dismutase 2, mitochondrial); SOD3 (Superoxide dismutase 3, extracellular); TAP1 (Transporter 1, ABC (ATP binding cassette)); TAPBP (TAP binding protein (tapasin)); TK1 (Thymidine kinase 1, soluble); TPMT (Thiopurine S-methyltransferase); TXNRD1 (Thioredoxin reductase 1); TYMS (Thymidylate synthetase); UGALT (UDP-galactose translocator); UGT2B10 (UDP glycosyltransferase 2 family, polypeptide B10); UGT2B15 (UDP glycosyltransferase 2 family, polypeptide B15); UGT2B4 (UDP glycosyltransferase 2 family, polypeptide B4); UGT2B7 (UDP glycosyltransferase 2 family, polypeptide B7); UGT8 (UDP glycosyltransferase 8 (UDP-galactose ceramide galactosyltransferase)); UMPS (Uridine monophosphate synthetase (orotate phosphoribosyl transferase and orotidine-5'-decarboxylase)); XDH (Xanthene dehydrogenase);

### Signal transduction

ABL1 (V-abl Abelson murine leukemia viral oncogene homolog 1); ABL2 (V-abl Abelson murine leukemia viral oncogene homolog 2 (arg, Abelson-related gene)); ABR (Active BCR-related gene); ACVR1B (Activin A receptor, type IB); ADK (Adenosine kinase); ADRBK1 (Adrenergic, beta, receptor kinase 1); AK1 (Adenylate kinase 1); AK2 (Adenylate kinase 2); AK3 (Adenylate kinase 3); AKT1 (V-akt murine thymoma viral oncogene homolog 1); AKT2 (V-akt murine thymoma viral oncogene homolog 2); ALOX12 (Arachidonate 12-lipoxygenase); ALOX5 (Arachidonate 5-lipoxygenase); ARAF1 (V-raf murine sarcoma 3611 viral oncogene homolog 1); ARHA (Ras homolog gene family, member A); ARHC (Ras homolog gene family, member C); ARHGDIB (Rho GDP dissociation inhibitor (GDI) beta); BLK (B lymphoid tyrosine kinase); BMPR2 (Bone morphogenetic protein receptor, type II (serine/threonine kinase)); BMX (BMX non-receptor tyrosine kinase); BRAF (V-raf murine sarcoma viral oncogene homolog B1); BTK (Bruton agammaglobulinemia tyrosine kinase); CAK (Cell adhesion kinase); CALM1 (Calmodulin 1 (phosphorylase kinase, delta)); CAMK4 (Calcium/calmodulin-dependent protein kinase IV); CBLB (Cas-Br-M (murine) ectropic retroviral transforming sequence b); CCNH (Cyclin H); CDC2L1 (Cell division cycle 2-like 1 (PITSLRE proteins)); CDC42 (Cell division cycle 42 (GTP-binding protein, 25kD)); CDC42 (Cell division cycle 42 (GTP-binding protein, 25kD)); CDC7L1 (CDC7 (cell division cycle 7, S. cerevisiae, homolog)-like 1); CDK2 (Cyclin-dependent kinase 2); CDK5 (Cyclin-dependent kinase 5); CDK6 (Cyclin-dependent kinase 6); CDK7 (Cyclin-dependent kinase 7 (homolog of Xenopus MO15 cdk-activating kinase)); CDK8 (Cyclin-dependent kinase 8); CDKN2B (Cyclin-dependent kinase inhibitor 2B (p15, inhibits CDK4)); CHEK1 (CHK1 (checkpoint, S.pombe) homolog); CHN1 (Chimerin (chimaerin) 1); CHN2 (Chimerin (chimaerin) 2); CHUK (Conserved helix-loop-helix ubiquitous kinase); CIS4 (STAT induced STAT inhibitor-4); CKS1 (CDC28 protein kinase 1); CKS2 (CDC28 protein kinase 2); CLGN (Calmegin); CLK2 (CDC-like kinase 2); CLK3 (CDC-like kinase 3); CNK (Cytokine-inducible kinase); COT (Cot (cancer Osaka thyroid) oncogene); CSK (C-src tyrosine kinase); CSNK1A1 (Casein kinase 1, alpha 1); CSNK1D (Casein kinase 1, delta); CSNK1E (Casein kinase 1, epsilon); CSNK2A2 (Casein kinase 2, alpha prime polypeptide); CSNK2B (Casein kinase 2, beta polypeptide); CTNNA1 (Catenin (cadherin-associated protein), alpha 1 (102kD)); CTNNB1 (Catenin (cadherin-associated protein), beta 1 (88kD)); CTNND2 (Catenin (cadherin-associated protein), delta 2 (neural plakophilin-related arm-repeat protein)); DGKA (Diacylglycerol kinase, alpha (80kD)); DGKG (Diacylglycerol kinase, gamma (90kD)); DGKQ (Diacylglycerol kinase, theta (110kD)); DMPK (Dystrophia myotonica-protein kinase); DRG2 (Developmentally regulated GTP-binding protein 2); DVL3 (Dishevelled 3 (homologous to Drosophila dsh)); DYRK1 (Dual-specificity tyrosine-(Y)-phosphorylation regulated kinase 1); DYRK1B (Dual-specificity tyrosine-(Y)-phosphorylation regulated kinase 1B); DYRK2 (Dual-specificity tyrosine-(Y)-phosphorylation regulated kinase 2); DYRK3 (Dual-specificity tyrosine-(Y)-phosphorylation regulated kinase 3); DYRK4 (Dual-specificity tyrosine-(Y)-phosphorylation regulated kinase 4); EFNA1 (Ephrin-A1); EFNA5 (Ephrin-A5); EFNB1 (Ephrin-B1); EFNB2 (Ephrin-B2); EGFR (Epidermal growth factor receptor (avian erythroblastic leukemia viral (v-erb-b) oncogene homolog)); EPHA2 (EphA2); EPHA4 (EphA4); EPHA5 (EphA5); EPHA7 (EphA7); EPHB3 (EphB3); EPHB4 (EphB4); EPHB6 (EphB6); ERBB2 (V-erb-b2 avian erythroblastic leukemia viral oncogene homolog 2 (neuro/glioblastoma derived oncogene homolog)); ERBB3 (V-erb-b2 avian erythroblastic leukemia viral oncogene homolog 3); ERBB4 (V-erb-a avian erythroblastic leukemia viral oncogene homolog-like 4); FBL (Fibrillarin); FER (Fer (fps/fes related) tyrosine kinase (phosphoprotein NCP94)); FES (Feline sarcoma (Snyder-Theilen) viral (v-fes)/Fujinami avian sarcoma (PRCII) viral (v-fps) oncogene homolog); FGFR1 (Fibroblast growth factor receptor 1 (fms-related tyrosine kinase 2, Pfeiffer syndrome)); FGFR2 (Fibroblast growth factor receptor 2 (bacteria-expressed kinase, keratinocyte growth factor receptor, craniofacial dysostosis 1, Crouzon syndrome, Pfeiffer syndrome, Jackson-Weiss syndrome)); FGR (Gardner-Rasheed feline sarcoma viral (v-fgr) oncogene homolog); FLT1 (Fms-related tyrosine kinase 1 (vascular endothelial growth factor/vascular permeability factor receptor)); FLT3LG (Fms-related tyrosine kinase 3 ligand); FLT4 (Fms-related tyrosine kinase 4); FRK (Fyn-related kinase); FYB (FYN-binding protein (FYB-120/130)); FYN (FYN oncogene related to SRC, FGR, YES); GLA (Galactosidase, alpha); GNAI1 (Guanine nucleotide binding protein (G protein), alpha inhibiting activity polypeptide 1); GNG10 (Guanine nucleotide binding protein 10); GPRK6 (G protein-coupled receptor kinase 6); GRB2 (Growth factor receptor-bound protein 2); GUCY1B3 (Guanylate cyclase 1, soluble, beta 3); HCK (Hemopoietic cell kinase); HIPK3 (Homeodomain-interacting protein kinase 3); HRK (Harakiri, BCL2-interacting protein (contains only BH3 domain)); ILK (Integrin-linked kinase); IRAK1 (Interleukin-1 receptor-associated kinase 1); IRAK2 (Interleukin-1 receptor-associated kinase 2); ITPKB (Inositol 1,4,5-trisphosphate 3-kinase B); JAK1 (Janus kinase 1 (a protein tyrosine kinase)); JAK2 (Janus kinase 2 (a protein tyrosine kinase)); JAK3 (Janus kinase 3 (a protein tyrosine kinase, leukocyte)); KDR (Kinase insert domain receptor (a type III receptor tyrosine kinase)); KIAA0641 (Apoptosis-associated tyrosine kinase); LCAT (Lecithin-cholesterol acyltransferase); LCK (Lymphocyte-specific protein tyrosine kinase); LIMK1 (LIM domain kinase 1); LIMK2 (LIM domain kinase 2); LTK (Leukocyte tyrosine kinase); LYN (V-yes-1 Yamaguchi sarcoma viral related oncogene homolog); MACS (Myristoylated alanine-rich protein kinase C substrate (MARCKS, 80K-L)); MADH1 (MAD (mothers against decapentaplegic, Drosophila) homolog 1); MADH2 (MAD (mothers against decapentaplegic, Drosophila) homolog 2); MADH3 (MAD (mothers against decapentaplegic, Drosophila) homolog 3); MAPKAPK2 (Mitogen-activated protein kinase-activated protein kinase 2); MAPKAPK5 (Mitogen-activated protein kinase-activated protein kinase 5); MATK (Megakaryocyte-associated tyrosine kinase); MEKK3 (MAP/ERK kinase kinase 3); MEKK4 (MAP/ERK kinase kinase 4); MEKK5 (MAP/ERK kinase kinase 5); MST1R (Macrophage stimulating 1 receptor (c-met-related tyrosine kinase)); NCK1 (NCK adaptor protein 1); NEK3 (NIMA (never in mitosis gene a)-related kinase); NME1 (Non-metastatic cells 1, protein (NM23A) expressed in); NME2 (Non-metastatic cells 2, protein (NM23B) expressed in); NMI (N-myc (and STAT) interactor); NPM1 (Nucleophosmin (nucleolar phosphoprotein B23, numatrin)); NTRK1 (Neurotrophic tyrosine kinase, receptor, type 1); NTRK2 (Neurotrophic tyrosine kinase, receptor, type 2); NTRK3 (Neurotrophic tyrosine kinase, receptor, type 3); NTRKR1 (Neurotrophic tyrosine kinase, receptor-related 1); NTRKR2 (Neurotrophic tyrosine kinase, receptor-related 2); NTRKR3 (Neurotrophic tyrosine kinase, receptor-related 3); PCTK3 (PCTAIRE protein kinase 3); PDE4B (Phosphodiesterase 4B, cAMP-specific (dunce (Drosophila)-homolog phosphodiesterase E4)); PDGFA (Platelet-derived growth factor alpha polypeptide); PDGFB (Platelet-derived growth factor beta polypeptide (simian sarcoma viral (v-sis) oncogene homolog)); PDGFRA (Platelet-derived growth factor receptor, alpha polypeptide); PDGFRB (Platelet-derived growth factor receptor, beta polypeptide); PDGFRL (Platelet-derived growth factor receptor-like); PDK2 (Pyruvate dehydrogenase kinase, isoenzyme 2); PDK4 (Pyruvate dehydrogenase kinase, isoenzyme 4); PDPK1 (3-phosphoinositide dependent protein kinase-1); PHKA2 (Phosphorylase kinase, alpha 2 (liver), glycogen storage disease IX); PHKG2 (Phosphorylase kinase, gamma 2 (testis)); PIASX-BETA (Protein inhibitor of activated STAT X); PIK3C3 (Phosphoinositide-3-kinase, class 3); PIK3CA (Phosphoinositide-3-kinase, catalytic, alpha polypeptide); PIK3CA (Phosphoinositide-3-kinase, catalytic, alpha polypeptide); PIK3CG (Phosphoinositide-3-kinase, catalytic, gamma polypeptide); PIM1 (Pim-1 oncogene); PLA2G2A (Phospholipase A2, group IIA (platelets, synovial fluid)); PLCB4 (Phospholipase C, beta 4); PLCE (Phospholipase C, epsilon); PLCG2 (Phospholipase C, gamma 2 (phosphatidylinositol-specific)); PPP2R5A (Protein phosphatase 2, regulatory subunit B (B56), alpha isoform); PPP2R5B (Protein phosphatase 2, regulatory subunit B (B56), beta isoform); PPP2R5C (Protein phosphatase 2, regulatory subunit B (B56), gamma isoform); PPP2R5D (Protein phosphatase 2, regulatory subunit B (B56), delta isoform); PPP2R5E (Protein phosphatase 2, regulatory subunit B (B56), epsilon isoform); PRKAA2 (Protein kinase, AMP-activated, alpha 2 catalytic subunit); PRKACA (Protein kinase, cAMP-dependent, catalytic, alpha); PRKACB (Protein kinase, cAMP-dependent, catalytic, beta); PRKACG (Protein kinase, cAMP-dependent, catalytic, gamma); PRKAG1 (Protein kinase, AMP-activated, gamma 1 non-catalytic subunit); PRKAR1A (Protein kinase, cAMP-dependent, regulatory, type I, alpha (tissue specific extinguisher 1)); PRKAR1B (Protein kinase, cAMP-dependent, regulatory, type I, beta); PRKAR2B (Protein kinase, cAMP-dependent, regulatory, type II, beta); PRKCA (Protein kinase C, alpha); PRKCB1 (Protein kinase C, beta 1); PRKCG (Protein kinase C, gamma); PRKCI (Protein kinase C, iota); PRKCL1 (Protein kinase C-like 1); PRKCL2 (Protein kinase C-like 2); PRKCM (Protein kinase C, mu); PRKCQ (Protein kinase C, theta); PRKCZ (Protein kinase C, zeta); PRKG1 (Protein kinase, cGMP-dependent, type I); PRKG2 (Protein kinase, cGMP-dependent, type II); PRKM1 (Protein kinase, mitogen-activated 1 (MAP kinase 1; p40, p41)); PRKM10 (Protein kinase mitogen-activated 10 (MAP kinase)); PRKM11 (Protein kinase mitogen- activated 11); PRKM13 (Protein kinase mitogen- activated 13); PRKM3 (Protein kinase, mitogen-activated 3 (MAP kinase 3; p44)); PRKM4 (Protein kinase, mitogen-activated 4 (MAP kinase 4; p63)); PRKM6 (Protein kinase, mitogen-activated 6 (extracellular signal-regulated kinase, p97)); PRKM7 (Protein kinase mitogen-activated 7 (MAP kinase)); PRKM8 (Protein kinase mitogen-activated 8 (MAP kinase)); PRKM9 (Protein kinase mitogen-activated 9 (MAP kinase)); PRKMK1 (Protein kinase, mitogen-activated, kinase 1 (MAP kinase kinase 1)); PRKMK1 (Protein kinase, mitogen-activated, kinase 1 (MAP kinase kinase 1)); PRKMK2 (Protein kinase, mitogen-activated, kinase 2, p45 (MAP kinase kinase 2)); PRKMK3 (Protein kinase, mitogen-activated, kinase 3 (MAP kinase kinase 3)); PRKMK5 (Protein kinase, mitogen-activated, kinase 5 (MAP kinase kinase 5)); PRKMK7 (Protein kinase, mitogen-activated, kinase 7 (MAP kinase kinase 7)); PRKR (Protein kinase, interferon-inducible double stranded RNA dependent); PTK2 (PTK2 protein tyrosine kinase 2); PTK2B (Protein tyrosine kinase 2 beta); PTK7 (PTK7 protein tyrosine kinase 7); PTK9 (Protein tyrosine kinase 9); RAB8IP (Rab8 interacting protein (GC kinase)); RASA1 (RAS p21 protein activator (GTPase activating protein) 1); RET (Ret proto-oncogene (multiple endocrine neoplasia MEN2A, MEN2B and medullary thyroid carcinoma 1, Hirschsprung disease)); RGS1 (Regulator of G-protein signalling 1); RGS16 (Regulator of G-protein signalling 16); RGS7 (Regulator of G-protein signalling 7); RHO7 (GTP-binding protein Rho7); RHOK (Rhodopsin kinase); ROCK1 (Rho-associated, coiled-coil containing protein kinase 1); RPS6KA2 (Ribosomal protein S6 kinase, 90kD, polypeptide 2); RPS6KB 1 (Ribosomal protein S6 kinase, 70kD, polypeptide 1); RYK (RYK receptor-like tyrosine kinase); SAPK3 (Stress-activated protein kinase 3); SERK1 (SAPK/Erk kinase 1); SFN (Stratifin); SH2D1A (SH2 domain protein 1A, Duncan's disease (lymphoproliferative syndrome)); SH3D1B (SH3 domain protein 1B); SRC (V-src avian sarcoma (Schmidt-Ruppin A-2) viral oncogene homolog); SRPK2 (SFRS protein kinase 2); SSI-1 (JAK binding protein); SSI-3 (STAT induced STAT inhibitor 3); STAT1 (Signal transducer and activator of transcription 1, 91kD); STAT2 (Signal transducer and activator of transcription 2, 113kD); STAT3 (Signal transducer and activator of transcription 3 (acute-phase response factor)); STAT4 (Signal transducer and activator of transcription 4); STAT5A (Signal transducer and activator of transcription 5A); STAT6 (Signal transducer and activator of transcription 6, interleukin-4 induced); STATI2 (STAT induced STAT inhibitor-2); STK11 (Serine/threonine kinase 11 (Peutz-Jeghers syndrome)); STK15 (Serine/threonine kinase 15); STK17A (Serine/threonine kinase 17a (apoptosis-inducing)); STK17B (Serine/threonine kinase 17b (apoptosis-inducing)); STK2 (Serine/threonine kinase 2); STK3 (Serine/threonine kinase 3 (Ste20, yeast homolog)); STK4 (Serine/threonine kinase 4); SUB1.5 (Guanine nucleotide exchange factor; 115-kD; mouse Lsc homolog); SYK (Spleen tyrosine kinase); TESK1 (Testis-specific kinase 1); TIAM1 (T-cell lymphoma invasion and metastasis 1); TK2 (Thymidine kinase 2, mitochondrial); TNFAIP1 (Tumor necrosis factor, alpha-induced protein 1 (endothelial)); TRK (Oncogene TRK); TRK1 (TRNA lysine 1); TTK (TTK protein kinase); TXK (TXK tyrosine kinase); TYK2 (Tyrosine kinase 2); TYRO3 (TYRO3 protein tyrosine kinase); TYROBP (TYRO protein tyrosine kinase binding protein); UBE1L (Ubiquitin-activating enzyme E1, like); UBE2A (Ubiquitin-conjugating enzyme E2A (RAD6 homolog)); UBE2B (Ubiquitin-conjugating enzyme E2B (RAD6 homolog)); VASP (Vasodilator-stimulated phosphoprotein); VAV2 (Vav 2 oncogene); VRK2 (Vaccinia related kinase 2); WAS (Wiskott-Aldrich syndrome (ecezema-thrombocytopenia)); YES1 (V-yes-1 Yamaguchi sarcoma viral oncogene homolog 1); YESP (V-yes-1 Yamaguchi sarcoma viral oncogene homolog pseudogene); ZAP70 (Zeta-chain (TCR) associated protein kinase (70 kD)); ZPK (Zipper (leucine) protein kinase);

### Transcription

ADA (Adenosine deaminase); ADPRT (ADP-ribosyltransferase (NAD+; poly (ADP-ribose) polymerase)); ADSS (Adenylosuccinate synthase); AHR (Aryl hydrocarbon receptor); ATBF1 (AT-binding transcription factor 1); ATF1 (Activating transcription factor 1); ATF3 (Activating transcription factor 3); ATF4 (Activating transcription factor 4 (tax-responsive enhancer element B67)); BARD1 (BRCA1 associated RING domain 1); BCL6 (B-cell CLL/lymphoma 6 (zinc finger protein 51)); BMZF2 (Zinc finger 2, bone marrow); BMZF3 (Bone marrow zinc finger 3); CBF2 (CCAAT-box-binding transcription factor); CBFA2 (Core-binding factor, runt domain, alpha subunit 2 (acute myeloid leukemia 1; aml1 oncogene)); CBFA3 (Core-binding factor, runt domain, alpha subunit 3); CBFB (Core-binding factor, beta subunit); CEBPA (CCAAT/enhancer binding protein (C/EBP), alpha); CEBPB (CCAAT/enhancer binding protein (C/EBP), beta); CEBPD (CCAAT/enhancer binding protein (C/EBP), delta); CEBPE (CCAAT/enhancer binding protein (C/EBP), epsilon); CEBPG (CCAAT/enhancer binding protein (C/EBP), gamma); CENPE (Centromere protein E (312kD)); CHD1 (Chromodomain helicase DNA binding protein 1); CHD2 (Chromodomain helicase DNA binding protein 2); CHD3 (Chromodomain helicase DNA binding protein 3); CHD4 (Chromodomain helicase DNA binding protein 4); CREB2 (CAMP responsive element binding protein 2); CREBBP (CREB binding protein (Rubinstein-Taybi syndrome)); CSE1L (Chromosome segregation 1 (yeast homolog)-like); CSTF3 (Cleavage stimulation factor, 3' pre-RNA, subunit 3, 77kD); CTPS (CTP synthase); DCK (Deoxycytidine kinase); DCTD (DCMP deaminase); DDIT3 (DNA-damage-inducible transcript 3); DGUOK (Deoxyguanosine kinase); DR1 (Down-regulator of transcription 1, TBP-binding (negative cofactor 2)); DUT (DUTP pyrophosphatase); E2F1 (E2F transcription factor 1); E2F2 (E2F transcription factor 2); E2F5 (E2F transcription factor 5, p130-binding); EGR1 (Early growth response 1); EIF3S6 (Eukaryotic translation initiation factor 3, subunit 6 (48kD)); ELF1 (E74-like factor 1 (ets domain transcription factor)); ELF3 (E74-like factor 3 (ets domain transcription factor)); ELK4 (ELK4, ETS-domain protein (SRF accessory protein 1) NOTE: Symbol and name provisional); EP300 (E1A binding protein p300); ERV3 (Endogenous retroviral sequence 3 (includes zinc finger protein H-plk/HPF9)); ESR1 (Estrogen receptor 1); ETS2 (V-ets avian erythroblastosis virus E26 oncogene homolog 2); ETV1 (Ets variant gene 1); ETV3 (Ets variant gene 3); ETV4 (Ets variant gene 4 (E1A enhancer-binding protein, E1AF)); ETV5 (Ets variant gene 5 (ets-related molecule)); EZF (Endothelial Kruppel-like zinc finger protein); FKHR (Forkhead (Drosophila) homolog 1 (rhabdomyosarcoma)); FLI1 (Friend leukemia virus integration 1); FSRG1 (Female sterile homeotic-related gene 1 (mouse homolog)); GART (Phosphoribosylglycinamide formyltransferase, phosphoribosylglycinamide synthetase, phosphoribosylaminoimidazole synthetase); GATA1 (GATA-binding protein 1 (globin transcription factor 1)); GATA2 (GATA-binding protein 2); GATA3 (GATA-binding protein 3); GATA4 (GATA-binding protein 4); GLI (Glioma-associated oncogene homolog (zinc finger protein)); GRSF1 (G-rich RNA sequence binding factor 1); GTF2H2 (General transcription factor IIH, polypeptide 2 (44kD subunit)); H4FI (H4 histone family, member I); HHEX (Hematopoietically expressed homeobox); HIF1A (Hypoxia-inducible factor 1, alpha subunit (basic helix-loop-helix transcription factor)); HIVEP1 (Human immunodeficiency virus type I enhancer-binding protein 1); HLF (Hepatic leukemia factor); HMG17 (High-mobility group (nonhistone chromosomal) protein 17); HMG2 (High-mobility group (nonhistone chromosomal) protein 2); HNRPK (Heterogeneous nuclear ribonucleoprotein K); HZF2 (Zinc finger (C2H2)); ICSBP1 (Interferon consensus sequence binding protein 1); ID1 (Inhibitor of DNA binding 1, dominant negative helix-loop-helix protein); ID2 (Inhibitor of DNA binding 2, dominant negative helix-loop-helix protein); ID3 (Inhibitor of DNA binding 3, dominant negative helix-loop-helix protein); ID4 (Inhibitor of DNA binding 4, dominant negative helix-loop-helix protein); IRF1 (Interferon regulatory factor 1); IRF2 (Interferon regulatory factor 2); IRF4 (Interferon regulatory factor 4); IRF5 (Interferon regulatory factor 5); IRF7 (Interferon regulatory factor 7); JUN (V-jun avian sarcoma virus 17 oncogene homolog); JUND (Jun D proto-oncogene); KIAA0646 (C3HC4-type zinc finger protein); LAF4 (Lymphoid nuclear protein related to AF4); LKLF (Lung Kruppel-like zinc finger transcription factor); LYF1 (Zinc finger protein, subfamily 1A, 1 (Ikaros)); LYL1 (Lymphoblastic leukemia derived sequence 1); MAFG (V-maf musculoaponeurotic fibrosarcoma (avian) oncogene family, protein G); MAX (MAX protein); MAZ (MYC-associated zinc finger protein (purine-binding transcription factor)); MBLL (C3H-type zinc finger protein; similar to D. melanogaster muscleblind B protein); MDM2 (Mouse double minute 2, human homolog of; p53-binding protein); MHC2TA (MHC class II transactivator); MKI67 (Antigen identified by monoclonal antibody Ki-67); MNDA (Myeloid cell nuclear differentiation antigen); MSX1 (Msh (Drosophila) homeo box homolog 1 (formerly homeo box 7)); MTHFD (5,10-methylenetetrahydrofolate dehydrogenase, 5,10-methylenetetrahydrofolate cyclohydrolase, 10-formyltetrahydrofolate synthetase); MYC (V-myc avian myelocytomatosis viral oncogene homolog); NCBP (Nuclear cap binding protein, 80kD); NCBP (Nuclear cap binding protein, 80kD); NDP52 (Nuclear domain 10 protein); NFE2 (Nuclear factor (erythroid-derived 2), 45kD); NFKB 1 (Nuclear factor of kappa light polypeptide gene enhancer in B-cells 1 (p 105)); NFKB2 (Nuclear factor of kappa light polypeptide gene enhancer in B-cells 2 (p49/p100)); NFYA (Nuclear transcription factor Y, alpha); NP (Nucleoside phosphorylase); NURR1 (Nuclear receptor related 1 (transcriptionally inducible)); ODC1 (Ornithine decarboxylase 1); PAX3 (Paired box gene 3 (Waardenburg syndrome 1)); PBX1 (Pre-B-cell leukemia transcription factor 1); PBX3 (Pre-B-cell leukemia transcription factor 3); PCNA (Proliferating cell nuclear antigen); PEX6 (Peroxisomal biogenesis factor 6); PITX2 (Paired-like homeodomain transcription factor 2); PML (Promyelocytic leukemia); POU1F1 (POU domain, class 1, transcription factor 1 (Pit1, growth hormone factor 1)); POU2AF1 (POU domain, class 2, associating factor 1); POU2F1 (POU domain, class 2, transcription factor 1); POU2F2 (POU domain, class 2, transcription factor 2); POU3F1 (POU domain, class 3, transcription factor 1); POU3F2 (POU domain, class 3, transcription factor 2); POU5F1 (POU domain, class 5, transcription factor 1); PPAT (Phosphoribosyl pyrophosphate amidotransferase); PROP1 (Prophet of Pit1, paired-like homeodomain transcription factor); PRPS1 (Phosphoribosyl pyrophosphate synthetase 1); PTB (Polypyrimidine tract binding protein (heterogeneous nuclear ribonucleoprotein I)); RANBP2 (RAN binding protein 2); RARB (Retinoic acid receptor, beta); RARG (Retinoic acid receptor, gamma); RECQL (RecQ protein-like (DNA helicase Q1-like)); RENBP (Renin-binding protein); RFX1 (Regulatory factor X, 1 (influences HLA class II expression)); RFX2 (Regulatory factor X, 2 (influences HLA class II expression)); RFX3 (Regulatory factor X, 3 (influences HLA class II expression)); RFX4 (Regulatory factor X, 4 (influences HLA class II expression)); RFX5 (Regulatory factor X, 5 (influences HLA class II expression)); RFXAP (Regulatory factor X-associated protein); RXRA (Retinoid X receptor, alpha); SATB1 (Special AT-rich sequence binding protein 1 (binds to nuclear matrix/scaffold-associating DNA's)); SFRS7 (Splicing factor, arginine/serine-rich 7 (35kD)); SKIL (SKI-like); SLUG (Slug (chicken homolog), zinc finger protein); SMARCA2 (SWI/SNF related, matrix associated, actin dependent regulator of chromatin, subfamily a, member 2); SON (SON DNA binding protein); SOX4 (SRY (sex determining region Y)-box 4); SP100 (Nuclear antigen Sp100); SPIB (Spi-B transcription factor (Spi-1/PU.1 related)); SRF (Serum response factor (c-fos serum response element-binding transcription factor)); STAT3 (Signal transducer and activator of transcription 3 (acute-phase response factor)); STAT4 (Signal transducer and activator of transcription 4); STAT5A (Signal transducer and activator of transcription 5A); TAF2C2 (TATA box binding protein (TBP)-associated factor, RNA polymerase II, C2, 105kD); TAL2 (T-cell acute lymphocytic leukemia 2); TCEB1L (Transcription elongation factor B (SIII), polypeptide 1-like); TCF1 (Transcription factor 1, hepatic; LF-B1, hepatic nuclear factor (HNF1), albumin proximal factor); TCF12 (Transcription factor 12 (HTF4, helix-loop-helix transcription factors 4)); TCF3 (Transcription factor 3 (E2A immunoglobulin enhancer binding factors E12/E47)); TCF7 (Transcription factor 7 (T-cell specific, HMG-box)); TCF8 (Transcription factor 8 (represses interleukin 2 expression)); TCF9 (Transcription factor 9 (binds GC-rich sequences)); TEGT (Testis enhanced gene transcript); TFAP2B (Transcription factor AP-2 beta (activating enhancer-binding protein 2 beta)); TFAP4 (Transcription factor AP-4 (activating enhancer-binding protein 4)); TFDP2 (Transcription factor Dp-2 (E2F dimerization partner 2)); THRA (Thyroid hormone receptor, alpha (avian erythroblastic leukemia viral (v-erb-a) oncogene homolog)); TIAL1 (TIA1 cytotoxic granule-associated RNA-binding protein-like 1); TK1 (Thymidine kinase 1, soluble); TOP1 (Topoisomerase (DNA) I); TOP2B (Topoisomerase (DNA) II beta (180kD)); TP53 (Tumor protein p53 (Li-Fraumeni syndrome)); UBL1 (Ubiquitin-like 1 (sentrin)); WT1 (Wilms tumor 1); WT1 (Wilms tumor 1); XPO1 (Exportin 1 (CRM1, yeast, homolog)); ZFP103 (Zinc finger protein homologous to Zfp103 in mouse); ZFP36 (Zinc finger protein homologous to Zfp-36 in mouse); ZFP37 (Zinc finger protein homologous to Zfp37 in mouse); ZFP93 (Zinc finger protein); ZFX (Zinc finger protein, X-linked); ZFY (Zinc finger protein, Y-linked); ZK1 (Kruppel-type zinc finger (C2H2)); ZNF10 (Zinc finger protein 10 (KOX 1)); ZNF121 (Zinc finger protein 121 (clone ZHC32)); ZNF124 (Zinc finger protein 124 (HZF-16)); ZNF127 (Zinc finger protein 127); ZNF 131 (Zinc finger protein 131 (clone pHZ-10)); ZNF132 (Zinc finger protein 132 (clone pHZ-12)); ZNF133 (Zinc finger protein 133 (clone pHZ-13)); ZNF134 (Zinc finger protein 134 (clone pHZ-15)); ZNF135 (Zinc finger protein 135 (clone pHZ-17)); ZNF136 (Zinc finger protein 136 (clone pHZ-20)); ZNF137 (Zinc finger protein 137 (clone pHZ-30)); ZNF139 (Zinc finger protein 139 (clone pHZ-37)); ZNF140 (Zinc finger protein 140 (clone pHZ-39)); ZNF141 (Zinc finger protein 141 (clone pHZ-44)); ZNF142 (Zinc finger protein 142 (clone pHZ-49)); ZNF143 (Zinc finger protein 143 (clone pHZ-1)); ZNF144 (Zinc finger protein 144 (Mel-18)); ZNF145 (Zinc finger protein 145 (Kruppel-like, expressed in promyelocytic leukemia)); ZNF147 (Zinc finger protein 147 (estrogen-responsive finger protein)); ZNF148 (Zinc finger protein 148 (pHZ-52)); ZNF151 (Zinc finger protein 151 (pHZ-67)); ZNF154 (Zinc finger protein 154 (pHZ-92)); ZNF155 (Zinc finger protein 155 (pHZ-96)); ZNF157 (Zinc finger protein 157 (HZF22)); ZNF162 (Zinc finger protein 162); ZNF165 (Zinc finger protein 165); ZNF169 (Zinc finger protein 169); ZNF 173 (Zinc finger protein 173); ZNF 177 (Zinc finger protein 177); ZNF 189 (Zinc finger protein 189); ZNF198 (Zinc finger protein 198); ZNF2 (Zinc finger protein 2); ZNF20 (Zinc finger protein, C2H2, rapidly turned over); ZNF200 (Zinc finger protein 200); ZNF202 (Zinc finger protein 202); ZNF204 (Zinc finger protein 204); ZNF205 (Zinc finger protein 205); ZNF206 (Zinc finger protein 206); ZNF207 (Zinc finger protein 207); ZNF239 (Zinc finger protein 239); ZNF259 (Zinc finger protein 259); ZNF261 (Zinc finger protein 261); ZNF262 (Zinc finger protein 262); ZNF263 (Zinc finger protein 263); ZNF264 (Zinc finger protein 264); ZNF3 (Zinc finger protein 3 (A8-51)); ZNF35 (Zinc finger protein 35 (clone HF.10)); ZNF37A (Zinc finger protein 37a (KOX 21)); ZNF42 (Zinc finger protein 42 (myeloid-specific retinoic acid- responsive)); ZNF44 (Zinc finger protein 44 (KOX 7)); ZNF45 (Zinc finger protein 45 (a Kruppel-associated box (KRAB) domain polypeptide)); ZNF6 (Zinc finger protein 6 (CMPX1)); ZNF7 (Zinc finger protein 7 (KOX 4, clone HF.16)); ZNF74 (Zinc finger protein 74 (Cos52)); ZNF76 (Zinc finger protein 76 (expressed in testis)); ZNF8 (Zinc finger protein 8 (clone HF.18)); ZNF84 (Zinc finger protein 84 (HPF2)); ZNF85 (Zinc finger protein 85); ZNF9 (Zinc finger protein 9 (a cellular retroviral nucleic acid binding protein)); ZNF91 (Zinc finger protein 91 (HPF7, HTF10));

### Tumor Suppressor/Oncogenes

ABL1 (V-abl Abelson murine leukemia viral oncogene homolog 1); ABL2 (V-abl Abelson murine leukemia viral oncogene homolog 2 (arg, Abelson-related gene)); AKT1 (V-akt murine thymoma viral oncogene homolog 1); AKT2 (V-akt murine thymoma viral oncogene homolog 2); APC (Adenomatosis polyposis coli); ARAF1 (V-raf murine sarcoma 3611 viral oncogene homolog 1); ARAF2 (V-raf murine sarcoma 3611 viral oncogene homolog 2); ARHA (Ras homolog gene family, member A); ARHB (Ras homolog gene family, member B); ARHC (Ras homolog gene family, member C); AXL (AXL receptor tyrosine kinase); BCL2 (B-cell CLL/lymphoma 2); BCL3 (B-cell CLL/lymphoma 3); BCR (Breakpoint cluster region); BLYM (Avian lymphoma virus-derived transforming sequence); BMI1 (Murine leukemia viral (bmi-1) oncogene homolog); BRAF (V-raf murine sarcoma viral oncogene homolog B1); BRAFP (V-raf murine sarcoma viral oncogene homolog B1 pseudogene); CBFA2 (Core-binding factor, runt domain, alpha subunit 2 (acute myeloid leukemia 1; aml1 oncogene)); CBL (Cas-Br-M (murine) ecotropic retroviral transforming sequence); CCND1 (Cyclin D1 (PRAD1: parathyroid adenomatosis 1)); CDH1 (Cadherin 1, E-cadherin (epithelial)); CDK4 (Cyclin-dependent kinase 4); CDKN1A (Cyclin-dependent kinase inhibitor 1A (p21, Cip1)); CDKN1C (Cyclin-dependent kinase inhibitor 1C (p57, Kip2)); CDKN2A (Cyclin-dependent kinase inhibitor 2A (melanoma, p16, inhibits CDK4)); CHES1 (Checkpoint suppressor 1); COT (Cot (cancer Osaka thyroid) oncogene); CRK (V-crk avian sarcoma virus CT10 oncogene homolog); CRKL (V-crk avian sarcoma virus CT10 oncogene homolog-like); CSF1R (Colony stimulating factor 1 receptor, formerly McDonough feline sarcoma viral (v-fms) oncogene homolog); D10S170 (DNA segment, single copy, probe pH4 (transforming sequence, thyroid-1,); DCC (Deleted in colorectal carcinoma); DDX6 (DEAD/H (Asp-Glu-Ala-Asp/His) box polypeptide 6 (RNA helicase, 54kD)); E2F1 (E2F transcription factor 1); ECT2 (Epithelial cell transforming sequence 2 oncogene); EGFR (Epidermal growth factor receptor (avian erythroblastic leukemia viral (v-erb-b) oncogene homolog)); EIF3S6 (Eukaryotic translation initiation factor 3, subunit 6 (48kD)); ELE1 (RET-activating gene ELE1); ELK1 (ELK1, member of ETS oncogene family); ELK2P1 (ELK2, member of ETS oncogene family, pseudogene 1); ELK3 (ELK3, ETS-domain protein (SRF accessory protein 2) NOTE: Symbol and name provisional.); EMP1 (Epithelial membrane protein 1); EMS1 (Ems1 sequence (mammary tumor and squamous cell carcinoma-associated (p80/85 src substrate)); EPHA1 (EphA1); EPHA3 (EphA3); ERBA2L (V-erb-a avian erythroblastic leukemia viral oncogene homolog 2-like); ERBAL2 (V-erb-a avian erythroblastic leukemia viral oncogene homolog-like 2); ERBB2 (V-erb-b2 avian erythroblastic leukemia viral oncogene homolog 2 (neuro/glioblastoma derived oncogene homolog)); ERBB3 (V-erb-b2 avian erythroblastic leukemia viral oncogene homolog 3); ERBB4 (V-erb-a avian erythroblastic leukemia viral oncogene homolog-like 4); ERG (V-ets avian erythroblastosis virus E26 oncogene related); ETS1 (V-ets avian erythroblastosis virus E26 oncogene homolog 1); ETS2 (V-ets avian erythroblastosis virus E26 oncogene homolog 2); ETV 1 (Ets variant gene 1); ETV3 (Ets variant gene 3); ETV6 (Ets variant gene 6 (TEL oncogene)); EVI1 (Ecotropic viral integration site 1); EWSR1 (Ewing sarcoma breakpoint region 1); FAT (FAT tumor suppressor (Drosophila) homolog); FER (Fer (fps/fes related) tyrosine kinase (phosphoprotein NCP94)); FES (Feline sarcoma (Snyder-Theilen) viral (v-fes)/Fujinami avian sarcoma (PRCII) viral (v-fps) oncogene homolog); FGF3 (Fibroblast growth factor 3 (murine mammary tumor virus integration site (v-int-2) oncogene homolog)); FGF4 (Fibroblast growth factor 4 (heparin secretory transforming protein 1, Kaposi sarcoma oncogene)); FGF6 (Fibroblast growth factor 6); FGR (Gardner-Rasheed feline sarcoma viral (v-fgr) oncogene homolog); FKHL1 (Forkhead (Drosophila)-like 1); FLI1 (Friend leukemia virus integration 1); FLT1 (Fms-related tyrosine kinase 1 (vascular endothelial growth factor/vascular permeability factor receptor)); FOS (V-fos FBJ murine osteosarcoma viral oncogene homolog); FOSB (FBJ murine osteosarcoma viral oncogene homolog B); FOSL1 (FOS-like antigen-1); FOSL2 (FOS-like antigen 2); FYN (FYN oncogene related to SRC, FGR, YES); GLI (Glioma-associated oncogene homolog (zinc finger protein)); GLI2 (GLI-Kruppel family member GLI2); GLI3 (GLI-Kruppel family member GLI3 (Greig cephalopolysyndactyly syndrome)); GRF2 (Guanine nucleotide-releasing factor 2 (specific for crk proto-oncogene)); GRO1 (GRO1 oncogene (melanoma growth stimulating activity, alpha)); GRO2 (GRO2 oncogene); GRO3 (GRO3 oncogene); HCK (Hemopoietic cell kinase); HKR3 (GLI-Kruppel family member HKR3); HRAS (V-Ha-ras Harvey rat sarcoma viral oncogene homolog); HRASP (V-Ha-ras Harvey rat sarcoma viral oncogene homolog pseudogene); INT6P1 (Murine mammary tumor integration site 6 (oncogene homolog) pseudogene 1); IRF4 (Interferon regulatory factor 4); JUN (V-jun avian sarcoma virus 17 oncogene homolog); JUNB (Jun B proto-oncogene); JUND (Jun D proto-oncogene); KAI1 (Kangai 1 (suppression of tumorigenicity 6, prostate; CD82 antigen (R2 leukocyte antigen, antigen detected by monoclonal and antibody IA4))); KIT (V-kit Hardy-Zuckerman 4 feline sarcoma viral oncogene homolog); KRAS1P (V-Ki-rasl Kirsten rat sarcoma 1 viral oncogene homolog, processed pseudogene); KRAS2 (V-Ki-ras2 Kirsten rat sarcoma 2 viral oncogene homolog); LBC (Lymphoid blast crisis oncogene); LCK (Lymphocyte-specific protein tyrosine kinase); LCN2 (Lipocalin 2 (oncogene 24p3)); LCO (Liver cancer oncogene); LPSA (Oncogene liposarcoma (DNA segment, single copy, expressed, probes); LTA (Lymphotoxin alpha (TNF superfamily, member 1)); LTB (Lymphotoxin beta (TNF superfamily, member 3)); LYN (V-yes-1 Yamaguchi sarcoma viral related oncogene homolog); M1S1 (Membrane component, chromosome 1, surface marker 1 (40kD glycoprotein, identified by monoclonal antibody GA733)); M4S1 (Membrane component, chromosomal 4, surface marker (35kD glycoprotein)); MADH4 (MAD (mothers against decapentaplegic, Drosophila) homolog 4); MAF (V-maf musculoaponeurotic fibrosarcoma (avian) oncogene homolog); MAFG (V-maf musculoaponeurotic fibrosarcoma (avian) oncogene family, protein G); MAFK (V-maf avian musculoaponeurotic fibrosarcoma oncogene family, protein K); MAS1 (MAS1 oncogene); MAX (MAX protein); MCC (Mutated in colorectal cancers); MCF2 (MCF.2 cell line derived transforming sequence); MDM2 (Mouse double minute 2, human homolog of; p53-binding protein); MEL (Mel transforming oncogene (derived from cell line NK14)- RAB8 homolog); MELL1 (Mel transforming oncogene-like 1); MET (Met proto-oncogene (hepatocyte growth factor receptor)); MLH1 (MutL (E. coli) homolog 1 (colon cancer, nonpolyposis type 2)); MOS (V-mos Moloney murine sarcoma viral oncogene homolog); MPL (Myeloproliferative leukemia virus oncogene); MSH2 (MutS (E. coli) homolog 2 (colon cancer, nonpolyposis type 1)); MUM1 (Multiple myeloma oncogene 1); MYB (V-myb avian myeloblastosis viral oncogene homolog); MYBL1 (V-myb avian myeloblastosis viral oncogene homolog-like 1); MYBL2 (V-myb avian myeloblastosis viral oncogene homolog-like 2); MYC (V-myc avian myelocytomatosis viral oncogene homolog); MYCL1 (V-myc avian myelocytomatosis viral oncogene homolog 1, lung carcinoma derived); MYCL2 (V-myc avian myelocytomatosis viral oncogene homolog 2); MYCLK1 (V-myc avian myelocytomatosis viral oncogene homolog-like 1); MYCN (V-myc avian myelocytomatosis viral related oncogene, neuroblastoma derived); MYCP (V-myc avian myelocytomatosis viral oncogene homolog pseudogene); NBL1 (Neuroblastoma candidate region, suppression of tumorigenicity 1); NF1 (Neurofibromin 1 (neurofibromatosis, von Recklinghausen disease, Watson disease)); NF2 (Neurofibromin 2 (bilateral acoustic neuroma)); NFKB2 (Nuclear factor of kappa light polypeptide gene enhancer in B-cells 2 (p49/p100)); NKTR (Natural killer-tumor recognition sequence); NOTCH4 (Notch (Drosophila) homolog 4); NOV (Nephroblastoma overexpressed gene); NRAS (Neuroblastoma RAS viral (v-ras) oncogene homolog); NRASL1 (Neuroblastoma RAS viral (v-ras) oncogene homolog-like 1); NRASL2 (Neuroblastoma RAS viral (v-ras) oncogene homolog-like 2); NRASL3 (Neuroblastoma RAS viral (v-ras) oncogene homolog-like 3); NTRK1 (Neurotrophic tyrosine kinase, receptor, type 1); OVC (Oncogene OVC (ovarian adenocarcinoma oncogene)); PACE (Paired basic amino acid cleaving enzyme (furin, membrane associated receptor protein)); PDGFB (Platelet-derived growth factor beta polypeptide (simian sarcoma viral (v-sis) oncogene homolog)); PIM1 (Pim-1 oncogene); PVTI (Pvt-1 (murine) oncogene homolog, MYC activator); RAB1 (RAB1, member RAS oncogene family); RAB11A (RAB 11A, member oncogene family); RAB11A (RAB11A, member RAS oncogene family); RAB 13 (RAB 13, member RAS oncogene family); RAB2 (RAB2, member RAS oncogene family); RAB27A (RAB27A, member RAS oncogene family); RAB27B (RAB27B, member RAS oncogene family); RAB2L (RAB2, member RAS oncogene family-like); RAB3A (RAB3A, member RAS oncogene family); RAB3B (RAB3B, member RAS oncogene family); RAB4 (RAB4, member RAS oncogene family); RAB5A (RAB5A, member RAS oncogene family); RAB5B (RAB5B, member RAS oncogene family); RAB6 (RAB6, member RAS oncogene family); RAB7L1 (RAB7, member RAS oncogene family-like 1); RABL (RAB, member of RAS oncogene family-like); RAF1 (V-raf-1 murine leukemia viral oncogene homolog 1); RAF1P1 (V-raf-1 murine leukemia viral oncogene homolog 1 pseudogene 1); RALA (V-ral simian leukemia viral oncogene homolog A (ras related)); RALB (V-ral simian leukemia viral oncogene homolog B (ras related; GTP binding protein)); RAN (RAN, member RAS oncogene family); RAP1A (RAP1A, member of RAS oncogene family); RAP1AP (RAP1A, member of RAS oncogene family pseudogene); RAP1B (RAP1B, member of RAS oncogene family); RAP2A (RAP2A, member of RAS oncogene family); RAP2B (RAP2B, member of RAS oncogene family); RB1 (Retinoblastoma I (including osteosarcoma)); REL (V-rel avian reticuloendotheliosis viral oncogene homolog); RELA (V-rel avian reticuloendotheliosis viral oncogene homolog A (nuclear factor of kappa light polypeptide gene enhancer in B-cells 3 (p65))); RELB (V-rel avian reticuloendotheliosis viral oncogene homolog B (nuclear factor of kappa light polypeptide gene enhancer in B-cells 3)); RET (Ret proto-oncogene (multiple endocrine neoplasia MEN2A, MEN2B and medullary thyroid carcinoma 1, Hirschsprung disease)); ROS1 (V-ros avian UR2 sarcoma virus oncogene homolog 1); RRAS (Related RAS viral (r-ras) oncogene homolog); SEA (S13 avian erythroblastosis oncogene homolog); SKI (V-ski avian sarcoma viral oncogene homolog); SMARCB1 (SWI/SNF related, matrix associated, actin dependent regulator of chromatin, subfamily b, member 1); SPI1 (Spleen focus forming virus (SFFV) proviral integration oncogene spi1); SPINK1 (Serine protease inhibitor, Kazal type 1); SRC (V-src avian sarcoma (Schmidt-Ruppin A-2) viral oncogene homolog); ST5 (Suppression of tumorigenicity 5); SUPT3H (Suppressor of Ty (S.cerevisiae) 3 homolog); SUPT5H (Suppressor of Ty (S.cerevisiae) 5 homolog); SUPT6H (Suppressor of Ty (S.cerevisiae) 6 homolog); TAL1 (T-cell acute lymphocytic leukemia 1); TGFBR2 (Transforming growth factor, beta receptor II (70-80kD)); THPO (Thrombopoietin (myeloproliferative leukemia virus oncogene ligand, megakaryocyte growth and development factor)); THRA (Thyroid hormone receptor, alpha (avian erythroblastic leukemia viral (v-erb-a) oncogene homolog)); THRB (Thyroid hormone receptor, beta (avian erythroblastic leukemia viral (v-erb-a) oncogene homolog 2)); TIAM1 (T-cell lymphoma invasion and metastasis 1); TIM (Oncogene TIM); TM4SF1 (Transmembrane 4 superfamily member 1); TNF (Tumor necrosis factor (TNF superfamily, member 2)); TP53BP2 (Tumor protein p53-binding protein, 2); TP73 (Tumor protein p73); TPR (Translocated promoter region (to activated MET oncogene)); TRE17 (Tre-2 oncogene); USP4 (Ubiquitin specific protease 4 (proto-oncogene)); USP6 (Ubiquitin specific protease 6 (Tre-2 oncogene)); VAV1 (Vav 1 oncogene); VAV2 (Vav 2 oncogene); VHL (Von Hippel-Lindau syndrome); WNT1 (Wingless-type MMTV integration site family, member 1); WNT2 (Wingless-type MMTV integration site family member 2); WNT5A (Wingless-type MMTV integration site family, member 5A); WT1 (Wilms tumor 1); YES1 (V-yes-1 Yamaguchi sarcoma viral oncogene homolog 1); YESP (V-yes-1 Yamaguchi sarcoma viral oncogene homolog pseudogene); AMPHL (Amphiphysin-like); APC (Adenomatosis polyposis coli); ARHA (Ras homolog gene family, member A); ARHB (Ras homolog gene family, member B); ARHC (Ras homolog gene family, member C); AXL (AXL receptor tyrosine kinase); BCL2 (B-cell CLL/lymphoma 2); BCL3 (B-cell CLL/lymphoma 3); BCR (Breakpoint cluster region); BLYM (Avian lymphoma virus-derived transforming sequence); BRCA1 (Breast cancer 1, early onset); BRCA2 (Breast cancer 2, early onset); CBL (Cas-Br-M (murine) ecotropic retroviral transforming sequence); CCND1 (Cyclin D1 (PRAD1: parathyroid adenomatosis 1)); CDH1 (Cadherin 1, E-cadherin (epithelial)); CDK4 (Cyclin-dependent kinase 4); CDKN1A (Cyclin-dependent kinase inhibitor 1A (p21, Cip1)); CDKN1C (Cyclin-dependent kinase inhibitor 1C (p57, Kip2)); CDKN2A (Cyclin-dependent kinase inhibitor 2A (melanoma, p16, inhibits CDK4)); CDKN2B (Cyclin-dependent kinase inhibitor 2B (p15, inhibits CDK4)); CHES1 (Checkpoint suppressor 1); D10S170 (DNA segment, single copy, probe pH4 (transforming sequence, thyroid-1,); DCC (Deleted in colorectal carcinoma); DDX6 (DEAD/H (Asp-Glu-Ala-Asp/His) box polypeptide 6 (RNA helicase, 54kD)); E2F1 (E2F transcription factor 1); EIF3S6 (Eukaryotic translation initiation factor 3, subunit 6 (48kD)); ELE1 (RET-activating gene ELE1); ELK3 (ELK3, ETS-domain protein (SRF accessory protein 2) NOTE: Symbol and name provisional.); EMP1 (Epithelial membrane protein 1); EMS1 (Ems1 sequence (mammary tumor and squamous cell carcinoma-associated (p80/85 src substrate)); EPHA1 (EphA1); EPHA3 (EphA3); ETV3 (Ets variant gene 3); EVI1 (Ecotropic viral integration site 1); EWSR1 (Ewing sarcoma breakpoint region 1); FAT (FAT tumor suppressor (Drosophila) homolog); FER (Fer (fps/fes related) tyrosine kinase (phosphoprotein NCP94)); FHIT (Fragile histidine triad gene); FKHL1 (Forkhead (Drosophila)-like 1); FLT1 (Fms-related tyrosine kinase 1 (vascular endothelial growth factor/vascular permeability factor receptor)); FOSL1 (FOS-like antigen-1); FOSL2 (FOS-like antigen 2); GLI2 (GLI-Kruppel family member GLI2); GLI3 (GLI-Kruppel family member GLI3 (Greig cephalopolysyndactyly syndrome)); HCK (Hemopoietic cell kinase); HKR3 (GLI-Kruppel family member HKR3); ING1 (Inhibitor of growth 1); IRF4 (Interferon regulatory factor 4); KAI1 (Kangai 1 (suppression of tumorigenicity 6, prostate; CD82 antigen (R2 leukocyte antigen, antigen detected by monoclonal and antibody IA4))); LCK (Lymphocyte-specific protein tyrosine kinase); LTA (Lymphotoxin alpha (TNF superfamily, member 1)); LTB (Lymphotoxin beta (TNF superfamily, member 3)); M1S1 (Membrane component, chromosome 1, surface marker 1 (40kD glycoprotein, identified by monoclonal antibody GA733)); M4S1 (Membrane component, chromosomal 4, surface marker (35kD glycoprotein)); MADH4 (MAD (mothers against decapentaplegic, Drosophila) homolog 4); MAX (MAX protein); MCC (Mutated in colorectal cancers); MEN1 (Multiple endocrine neoplasia I); MLH1 (MutL (E. coli) homolog 1 (colon cancer, nonpolyposis type 2)); MSH2 (MutS (E. coli) homolog 2 (colon cancer, nonpolyposis type 1)); NBL1 (Neuroblastoma candidate region, suppression of tumorigenicity 1); NF1 (Neurofibromin 1 (neurofibromatosis, von Recklinghausen disease, Watson disease)); NF2 (Neurofibromin 2 (bilateral acoustic neuroma)); NFKB2 (Nuclear factor of kappa light polypeptide gene enhancer in B-cells 2 (p49/p100)); NKTR (Natural killer-tumor recognition sequence); NME1 (Non-metastatic cells 1, protein (NM23A) expressed in); NOV (Nephroblastoma overexpressed gene); NTRK1 (Neurotrophic tyrosine kinase, receptor, type 1); PDGFRL (Platelet-derived growth factor receptor-like); PLA2G2A (Phospholipase A2, group IIA (platelets, synovial fluid)); PTCH (Patched (Drosophila) homolog); PTEN (Phosphatase and tensin homolog (mutated in multiple advanced cancers 1)); RB1 (Retinoblastoma 1 (including osteosarcoma)); SMARCB1 (SWI/SNF related, matrix associated, actin dependent regulator of chromatin, subfamily b, member 1); SPINK1 (Serine protease inhibitor, Kazal type 1); ST5 (Suppression of tumorigenicity 5); SUPT3H (Suppressor of Ty (S.cerevisiae) 3 homolog); SUPT5H (Suppressor of Ty (S.cerevisiae) 5 homolog); SUPT6H (Suppressor of Ty (S.cerevisiae) 6 homolog); TAL1 (T-cell acute lymphocytic leukemia 1); TGFBR2 (Transforming growth factor, beta receptor II (70-80kD)); TIAMI (T-cell lymphoma invasion and metastasis 1); TIM (Oncogene TIM); TM4SF1 (Transmembrane 4 superfamily member 1); TNF (Tumor necrosis factor (TNF superfamily, member 2)); TP53 (Tumor protein p53 (Li-Fraumeni syndrome)); TP53BP2 (Tumor protein p53-binding protein, 2); TP73 (Tumor protein p73); VHL (Von Hippel-Lindau syndrome); WNT1 (Wingless-type MMTV integration site family, member 1); WNT2 (Wingless-type MMTV integration site family member 2); WNT5A (Wingless-type MMTV integration site family, member 5A); WT1 (Wilms tumor 1)

## Claims

1. An in vitro method for the development of gene panels for diagnostic and therapeutic purposes, comprising the steps of:
a) providing at least one biological sample from each of at least two groups of biological material containing mRNA and/or proteins;
b) analysing the expression level of at least two genes in at least one of the biological samples;
c) selecting the gene(s) exhibiting a different expression level between said at least two groups of biological material,
whereby a first knowledge base is generated;
d) analysing the level of cytosine methylation in the methylation relevant regions of at least two genes of at least one of the biological samples of step a), wherein the gene is selected on the basis of the first knowledge base;
e) selecting the gene(s) exhibiting a different level of cytosine methylation between said at least two groups of biological material,
whereby a second knowledge base is generated; and
f) adding selected genes from the second knowledge base to a gene panel.

2. Method according to claim 1, comprising that the biological material that is provided from a biopsy, an operation on an individual, a dissection, derived from a preserved biological sample, collected from body fluid(s) and/or collected directly from the environment.

3. Method according to claim 1 or 2, **characterised in that** the biological material comprises a eucaryotic and/or procaryotic cell line, a biopsy sample, blood, sputum, faeces, urine, cerebral liquid, tissue embedded in paraffin, tissue derived from eyes, intestine, brain, heart, prostata, kidney, lung, breast or liver, histological samples or a combination thereof.

4. Method according to any of claims 1 to 3, **characterised in that** at least one of the biological samples is derived from biological material of healthy and/or diseased individuals.

5. Method according to any of claims 1 to 4, **characterised in that** the isolation of said biological sample comprises isolating subcellular compartments, organelles, macromolecular structures and multiprotein complexes, partial or complete preparation of the mRNA, reverse transcription or partial digestion of the material with an enzyme selected from proteases, RNAses and/or DNAses or combinations thereof.

6. Method according to any of claims 1 to 5, **characterised in that** the analysis of the expression level of the at least two genes in the biological sample comprises determining the relative amount of mRNA or protein derived from said at least two genes.

7. Method according to claim 6, **characterised in that** the analysis comprises one- or two-dimensional gel electrophoresis, differential display, analysis of selected sets of tumour markers, subtractive hybridisation, mass spectrometry, comparative expressed sequence tag sequencing, representational difference analysis, cDNA or oligonucleotide arrays, serial analysis of gene expression, enzymatic, fluorescent, radioactive, dye and/or antibody labelling.

8. Method according to claim **7, characterised in that** the analysis further comprises measuring intensities of expression during one- or two-dimensional gel electrophoresis, differential display, subtractive hybridisation, DNA, RNA or protein sequencing, mass spectrometry, and enzymatic, radioactive, dye and/or antibody labelling.

9. Method according to any of claims 6 to 8, **characterised in that** the analysis is at least partially performed by means of a suited automate, for example a robot.

10. Method according claim 9, **characterised in that** the expression levels of at least 100 genes are analysed.

11. Method according to any of claims 1 to 10, **characterised in that** the selection is based on a combination of the analysis of both mRNA level and protein expression.

12. Method according to any of claims 1 to 11, **characterised in that** the selection is based on the result of at least two individual rows of analyses.

13. Method according to any of claims 1 to 12, **characterised in that** the selection is performed in such a way as to give a first knowledge base comprising only one set of selected genes.

14. Method according to any of claims 1 to 12, **characterised in that** the selection is performed in such a way as to give a first knowledge base comprising different subsets of selected genes.

15. Method according to any of claims 1 to 12, **characterised in that** the selection is at least partially performed automatically by means of a suited automate, such as a computer device.

16. Method according to any of claims 1 to 15, **characterised in that** at least two genes are selected.

17. Method according to claim 16, **characterised in that** at least 100 genes are selected.

18. Method according to any of claims 1 to 17, **characterised in that** the methylation relevant regions comprise the complete genes and/or promoters, introns, first exons and/or enhancers of the genes to be analysed.

19. Method according to any of claims 1 to 18, **characterised in that** the analysis of the level of cytosine methylation comprises chemical treatment with bisulphite, hydrogen sulphite or disulphite, polymerase chain reaction (PCR), hybridisation analyses, sequencing, mass spectrometry and fluorescent, enzymatic, radioactive, dye and/or antibody labelling.

20. Method according to any of claims 1 to 19**, characterised in that** the analysis is at least partially performed by means of a suited automate, for example a robot.

21. Method according claim 20, **characterised in that** the level of cytosine methylation of at least 100 genes are analysed.

22. Method according to any of claims 1 to 21, **characterised in that** the selection is based on the result of at least two individual rows of analyses.

23. Method according to any of claims 1 to 22, **characterised in that** the selection is performed in such a way as to give a second knowledge base comprising only one set of selected genes.

24. Method according to any of claims 1 to 23, **characterised in that** the selection is performed in such a way as to give a second knowledge base comprising different subsets of selected genes.

25. Method according to any of claims 1 to 24, **characterised in that** the selection is at least partially performed automatically by means of a suited automate, such as a computer device.

26. Method according to any of claims 1 to 25, **characterised in that** at least two genes are selected in parallel.

27. Method according to claim 26, **characterised in that** at least 100 genes are selected in parallel.

28. Method according to any of claims 1 to 27, **characterised in that** all or a part of the genes of the second knowledge base are added to the gene panel.

29. Method according to any of claims 1 to 28, **characterised in that** additional information about methylation relevant regions of the selected genes is added to the gene panel.

30. Method according to any of claims 1 to 29, **characterised in that** steps a) to f) are repeated.

31. Method according to claim 30, **characterised in that** it is repeated for at least 100 times.

32. Method according to any of claims 30 or 31,**characterised in that** the identical biological material, different biological material or a combination thereof is used in step a).

33. Method according to any of claims 1 to 32, **characterised in that** the steps are performed in the following order: step a), step d), step e), step b), step c), and step f).

34. Method according to any of claims 1 to 33, **characterised in that** it is at least partially performed by means of a suited automate, for example a robot.

35. An in vitro method for diagnosing a disease, comprising the following steps:
a) providing a gene panel according to any of claims 1 to 34;
b) analysing the level of cytosine methylation at selected sites of the DNA based on said gene panel in biological material of at least one diseased individual with a known disease or medical condition and at least one healthy individual, thereby generating a first knowledge base;
c) analysing the level of cytosine methylation at selected sites of the DNA based on said gene panel in biological material of at least one diseased individual with an unknown disease or medical condition, thereby generating a second knowledge base; and
d) providing a third knowledge base comprising a plurality of expert rules for comparing the first and second knowledge base;
e) selecting a type of disease or medical condition for the at least one diseased individual with an unknown disease or medical condition based on said first to third knowledge bases.

36. Method according to claim 35, wherein the disease is selected from unwanted side effects of medicaments, cancers, dysfunctions, damages or diseases of the central nerval system (CNS), aggressive symptoms or behavioural disorders, clinical, and psychological consequences of brain injuries, psychotic disorders, dementia and/or associates syndromes, cardiovascular diseases, diseases, malfunctions or damages of the gastrointestine, diseases, malfunctions or damages of the respiratory system, injury, inflammation, infection, immunity and/or reconvalescence, diseases, malfunctions or damages as consequences of modifications in the developmental process, diseases, malfunctions or damages of the skin, muscles, connective tissue or bones, endocrine or metabolic diseases, malfunctions or damages, headache, and sexual malfunctions or combinations thereof, leukemia, head and neck cancer, Hodgkin's disease, gastric cancer, prostate cancer, renal cancer, bladder cancer, breast cancer, Burkitt's lymphoma, Wilms tumor, Prader-Willi/Angelman syndrome, ICF syndrome, dermatofibroma, hypertension, pediatric neurobiological diseases, autism, ulcerative colitis, fragile X syndrome, and Huntington's disease.

## Patentansprüche

1. In vitro Verfahren zur Entwicklung von Genpanels für diagnostische und therapeutische Zwecke, umfassend die Schritte von:
a) Zur Verfügung stellen von mindestens einer biologischen Probe von jeder von mindestens zwei Gruppen an biologischem Material enthaltend mRNA und/oder Proteine;
b) Analysieren des Expressionsspiegels von mindestens zwei Genen in mindestens einer der biologischen Proben;
c) Auswählen des/der Gens/Gene, der/die einen unterschiedlichen Expressionsspiegel zwischen den mindestens zwei Gruppen an biologischem Material zeigt/zeigen,
wodurch eine erste Wissensdatenbank erzeugt wird;
d) Analysieren des Spiegels an Cytosinmethylierung in den Methylierungs-relevanten Regionen von mindestens zwei Genen von mindestens einer der biologischen Proben aus Schritt a), wobei das Gen auf der Basis der ersten Wissensdatenbank ausgewählt wird;
e) Auswählen der/des Gens/Gene, das/die einen unterschiedlichen Spiegel an Cytosinmethylierung zwischen den mindestens zwei Gruppen an biologischem Material aufweisen/aufweist,
wodurch eine zweite Wissensdatenbank erzeugt wird; und
f) Hinzufügen von ausgewählten Genen aus der zweiten Wissensdatenbank zu einem Genpanel.

2. Verfahren nach Anspruch 1, umfassend, dass das biologische Material, das zur Verfügung gestellt wird, aus einer Biopsie, einer Operation an einem Individuum, einer Präparation, aus einer konservierten biologischen Probe abgeleitet ist, aus Körperflüssigkeit(en) entnommen und/oder direkt aus der Umgebung entnommen ist.

3. Verfahren nach Anspruch 1 oder 2**, dadurch gekennzeichnet, dass** das biologische Material eine eukaryontische und/oder prokaryontische Zelllinie, eine Biopsieprobe, Blut, Sputum, Faeces, Urin, Cerebralflüssigkeit, tissue embedded in Paraffin eingebettetes Gewebe, von Augen, Darm, Hirn, Herz, Prostata, Niere, Lunge, Brust oder Leber abgeleitetes Gewebe, histologische Proben oder eine Kombination davon umfasst.

4. Verfahren nach einem der Ansprüche 1 bis 3, **dadurch gekennzeichnet, dass** mindestens eine der biologischen Proben von biologischen Material von gesunden und/oder erkrankten Individuen abgeleitet ist.

5. Verfahren nach einem der Ansprüche 1 bis 4, **dadurch gekennzeichnet, dass** die Isolierung der biologischen Probe ein Isolieren subzellulärer Kompartimente, Organellen, makromolekularer Strukturen und Multiproteinkomplexe, partielle oder vollständige Präparation der mRNA, reverse Transkription oder partiellen Verdau des Materials mit einem Enzym ausgewählt aus Proteasen, RNAsen und/oder DNAsen oder Kombinationen davon umfasst.

6. Verfahren nach einem der Ansprüche 1 bis 5, **dadurch gekennzeichnet, dass** die Analyse des Expressionsspiegel der mindestens zwei Gene in der biologischen Probe ein Bestimmen der relativen Menge an mRNA oder Protein abgeleitet von den mindestens zwei Genen umfasst.

7. Verfahren nach Anspruch 6, **dadurch gekennzeichnet, dass** die Analyse ein- oder zweidimensionale Gelelektrophorese, Differential Display, Analyse von ausgewählten Sets an Tumormarkern, subtraktive Hybridisierung, Massenspektrometrie, vergleichende exprimierte Sequenzmarker-Sequenzierung, repräsentative Differenzanalyse, cDNA- oder Oligonukleotidarrays, serielle Analyse der Genexpression, enzymatische, fluoreszente, radioaktive, Farbstoff- und/oder Antikörper-Markierung umfasst.

8. Verfahren nach Anspruch 7, **dadurch gekennzeichnet, dass** die Analyse weiter ein Messen von Intensitäten der Expression während ein- oder zwei-dimensionaler Gelelektrophorese, Differential Display, subtraktiver Hybridisierung, DNA, RNA oder Protein-Sequenzierung, Massenspektrometrie und enzymatischer, radioaktiver, Farbstoff- und/oder Antikörper-Markierung umfasst.

9. Verfahren nach einem der Ansprüche 6 bis 8, **dadurch gekennzeichnet, dass** die Analyse zumindest teilweise mittels eines geeigneten Automaten durchgeführt wird, zum Beispiel einem Roboter.

10. Verfahren nach Anspruch 9, **dadurch gekennzeichnet, dass** die Expressionsspiegel von mindestens 100 Genen analysiert werden.

11. Verfahren nach einem der Ansprüche 1 bis 10, **dadurch gekennzeichnet, dass** die Auswahl auf einer Kombination der Analyse von sowohl mRNA Spiegel als auch Proteinexpression basiert wird.

12. Verfahren nach einem der Ansprüche 1 bis 11, **dadurch gekennzeichnet, dass** die Auswahl auf dem Ergebnis von mindestens zwei individuellen Reihen an Analysen basiert wird.

13. Verfahren nach einem der Ansprüche 1 bis 12, **dadurch gekennzeichnet, dass** die Auswahl auf solche Weise durchgeführt wird, dass sie eine erste Wissensdatenbank ergibt, umfassend nur ein Set von ausgewählten Genen.

14. Verfahren nach einem der Ansprüche 1 bis 12, **dadurch gekennzeichnet, dass** die Auswahl auf solche Weise durchgeführt wird, dass sie eine erste Wissensdatenbank ergibt, umfassend verschiedene Subsets von ausgewählten Genen.

15. Verfahren nach einem der Ansprüche 1 bis 12, **dadurch gekennzeichnet, dass** die Auswahl zumindest teilweise automatisiert mittels eines geeigneten Automaten durchgeführt wird, zum Beispiel einer Computervorrichtung.

16. Verfahren nach einem der Ansprüche 1 bis 15, **dadurch gekennzeichnet, dass** mindestens zwei Gene ausgewählt werden.

17. Verfahren nach Anspruch 16, **dadurch gekennzeichnet, dass** mindestens 100 Gene ausgewählt werden.

18. Verfahren nach einem der Ansprüche 1 bis 17, **dadurch gekennzeichnet, dass** die Methylierungs-relevanten Regionen die vollständigen Gene und/oder Promotoren, Introns, erste Exons und/oder Enhancer der zu analysierenden Gene umfassen.

19. Verfahren nach einem der Ansprüche 1 bis 18, **dadurch gekennzeichnet, dass** die Analyse des Spiegels an Cytosinmethylierung die chemische Behandlung mit Bisulfit, Hydrogensulfit oder Disulfit, Polymerase-Kettenreaktion (PCR), Hybridisierungsanalysen, Sequenzierung, Massenspektrometrie und enzymatische, fluoreszente, radioaktive, Farbstoff- und/oder Antikörper-Markierung umfasst.

20. Verfahren nach einem der Ansprüche 1 bis 19, **dadurch gekennzeichnet, dass** die Analyse zumindest teilweise mittels eines geeigneten Automaten durchgeführt wird, zum Beispiel einem Roboter.

21. Verfahren nach Anspruch 20, **dadurch gekennzeichnet, dass** der Spiegel an Cytosinmethylierung von mindestens 100 Genen analysiert wird.

22. Verfahren nach einem der Ansprüche 1 bis 21, **dadurch gekennzeichnet, dass** die Auswahl auf dem Ergebnis von mindestens zwei individuellen Reihen an Analysen basiert wird.

23. Verfahren nach einem der Ansprüche 1 bis 22, **dadurch gekennzeichnet, dass** die Auswahl auf solche Weise durchgeführt wird, dass sie eine zweite Wissensdatenbank ergibt, umfassend nur ein Set von ausgewählten Genen.

24. Verfahren nach einem der Ansprüche 1 bis 23, **dadurch gekennzeichnet, dass** die Auswahl auf solche Weise durchgeführt wird, dass sie eine zweite Wissensdatenbank ergibt, umfassend verschiedene Subsets von ausgewählten Genen.

25. Verfahren nach einem der Ansprüche 1 bis 24, **dadurch gekennzeichnet, dass** die Auswahl zumindest teilweise automatisiert mittels eines geeigneten Automaten durchgeführt wird, zum Beispiel einer Computervorrichtung.

26. Verfahren nach einem der Ansprüche 1 bis 25, **dadurch gekennzeichnet, dass** mindestens zwei Gene parallel ausgewählt werden.

27. Verfahren nach Anspruch 26, **dadurch gekennzeichnet, dass** mindestens 100 Gene parallel ausgewählt werden.

28. Verfahren nach einem der Ansprüche 1 bis 27, **dadurch gekennzeichnet, dass** alle oder ein Teil der Gene der zweiten Wissensdatenbank zu dem Genpanel hinzugefügt werden.

29. Verfahren nach einem der Ansprüche 1 bis 28, **dadurch gekennzeichnet, dass** zusätzliche Information über die Methylierungs-relevanten Regionen der ausgewählten Gene zu dem Genpanel hinzugefügt wird.

30. Verfahren nach einem der Ansprüche 1 bis 29, **dadurch gekennzeichnet, dass** die Schritte a) bis f) wiederholt werden.

31. Verfahren nach Anspruch 30, **dadurch gekennzeichnet, dass** es mindestens 100 mal wiederholt wird.

32. Verfahren nach einem der Ansprüche 30 oder 31, **dadurch gekennzeichnet, dass** das identische biologische Material, unterschiedliches biologisches Material oder eine Kombination davon in Schritt a) verwendet wird.

33. Verfahren nach einem der Ansprüche 1 bis 32, **dadurch gekennzeichnet, dass** die Schritte in der folgenden Reihenfolge durchgeführt werden: Schritt a), Schritt d), Schritt e), Schritt b), Schritt c) und Schritt f).

34. Verfahren nach einem der Ansprüche 1 bis 33, **dadurch gekennzeichnet, dass** es zumindest teilweise mittels eines geeigneten Automaten durchgeführt wird, zum Beispiel einem Roboter.

35. In vitro Verfahren zur Diagnose einer Erkrankung, umfassend die folgenden Schritte:
a) Zur Verfügung stellen eines Genpanel nach einem der Ansprüche 1 bis 34;
b) Analysieren der Spiegel an Cytosinmethylierung an ausgewählten Stellen der DNA basierend auf dem Genpanel in biologischem Material von mindestens einem erkrankten Individuum mit einer bekannten Erkrankung oder medizinischem Zustand und mindestens einem gesunden Individuum, wodurch eine erste Wissensdatenbank erzeugt wird;
c) Analysieren der Spiegel an Cytosinmethylierung an ausgewählten Stellen der DNA basierend auf dem Genpanel in biologischem Material von mindestens einem erkrankten Individuum mit einer unbekannten Erkrankung oder medizinischem Zustand, wodurch eine zweite Wissensdatenbank erzeugt wird; und
d) Zur Verfügung stellen einer dritten Wissensdatenbank umfassend eine Vielzahl von Expertenregeln zum Vergleichen der ersten und zweiten Wissensdatenbank;
e) Auswählen eines Typs an Erkrankung oder medizinischem Zustand für das mindestens eine erkrankte Individuum mit einer unbekannten Erkrankung oder medizinischem Zustand basierend auf den ersten bis dritten Wissensdatenbanken.

36. Verfahren nach Anspruch 35, wobei die Erkrankung ausgewählt ist aus unerwünschten Nebenwirkungen von Medikamenten, Krebserkrankungen, Dysfunktionen, Beschädigungen oder Erkrankungen des zentralen Nervensystems (CNS), aggressiven Symptomen oder Verhaltensstörungen, klinischen und psychologischen Konsequenzen von Hirnverletzungen, psychotischen Störungen, Demenz und/oder assoziierten Syndromen, kardiovaskulären Erkrankungen, Erkrankungen, Fehlfunktionen oder Beschädigungen des gastrointestinalen Systems, Erkrankungen, Fehlfunktionen oder Beschädigungen des respiratorischen Systems, Verletzung, Entzündung, Infektion, Immunität und/oder Rekonvaleszenz, Erkrankungen, Fehlfunktionen oder Beschädigungen als Konsequenzen von Modifikationen im Entwicklungsprozess, Erkrankungen, Fehlfunktionen oder Beschädigungen der Haut, Muskeln, Bindegewebe oder Knochen, endokrine oder metabolischen Erkrankungen, Fehlfunktionen oder Beschädigungen, Kopfschmerzen, und sexuelle Fehlfunktionen oder Kombinationen davon, Leukämie, Kopf und Nackenkrebs, Morbus Hodgkin, Magenkrebs, Prostatakrebs, Nierenkrebs, Blasenkrebs, Brustkrebs, Burkitt's Lymphom, Wilms Tumor, Prader-Willi/Angelman Syndrom, ICF Syndrom, Dermatofibroma, Hochdruck, pädiatrischen neurobiologischen Erkrankungen, Autismus, ulzerative Colitis, fragiles X Syndrom und Morbus Huntington.

## Revendications

1. Procédé *in vitro* de développement de panels de gènes pour des objectifs diagnostiques et thérapeutiques, comprenant les étapes consistant à :
a) fournir au moins un échantillon biologique à partir de chacun d'au moins deux groupes de matériau biologique contenant de l'ARNm et / ou des protéines ;
b) analyser le taux d'expression d'au moins deux gènes dans au moins l'un des échantillons biologiques ;
c) sélectionner le(s) gène(s) présentant un taux d'expression différent entre lesdits au moins deux groupes de matériau biologique,
moyennant quoi une première base de connaissance est générée ;
d) analyser le taux de méthylation des cytosines dans les régions pertinentes de la méthylation d'au moins deux gènes d'au moins l'un des échantillons biologiques de l'étape a), où le gène est sélectionné sur la base de la première base de connaissance ;
e) sélectionner le(s) gène(s) présentant un taux différent de méthylation des cytosines entre lesdits au moins deux groupes de matériau biologique,
moyennant quoi une seconde base de connaissance est générée ; et
f) ajouter les gènes sélectionnés à partir de la seconde base de connaissance à un panel de gènes.

2. Procédé selon la revendication 1, comprenant que le matériau biologique est fourni à partir d'une biopsie, d'une opération sur un individu, d'une dissection, dérivé d'un échantillon biologique préservé, recueilli à partir de liquide(s) corporel(s) et / ou recueilli directement à partir de l'environnement.

3. Procédé selon la revendication 1 ou 2, **caractérisé en ce que** le matériau biologique comprend une lignée cellulaire eucaryote et / ou procaryote, un échantillon de biopsie, du sang, des crachats, des selles, des urines, du liquide cérébral, un tissu noyé dans de la paraffine, du tissu dérivé des yeux, de l'intestin, du cerveau, du coeur, de la prostate, du rein, du poumon, du sein ou du foie, des échantillons histologiques ou une combinaison de ceux-ci.

4. Procédé selon l'une quelconque des revendications 1 à 3, **caractérisé en ce qu'**au moins l'un des échantillons biologiques est dérivé de matériau biologique d'individus en bonne santé et / ou malades.

5. Procédé selon l'une quelconque des revendications 1 à 4, **caractérisé en ce que** l'isolement dudit échantillon biologique comprend l'isolement de compartiments subcellulaires, d'organites, de structures macromoléculaires et de complexes de protéines multiples, la préparation partielle ou complète de l'ARNm, la transcription inverse ou la digestion partielle du matériau avec une enzyme choisie parmi des protéases, des ARNases et / ou des ADNases ou des combinaisons de celles-ci.

6. Procédé selon l'une quelconque des revendications 1 à 5, **caractérisé en ce que** l'analyse du taux d'expression des au moins deux gènes dans l'échantillon biologique comprend la détermination de la quantité relative d'ARNm ou de protéine dérivé(e) desdits au moins deux gènes.

7. Procédé selon la revendication 6, **caractérisé en ce que** l'analyse comprend une électrophorèse sur gel mono- ou bi-dimensionnelle, une présentation différentielle, une analyse d'ensembles choisis de marqueurs tumoraux, une hybridation soustractive, une spectrométrie de masse, un séquençage comparatif d'étiquette de séquence exprimée, une analyse de différence représentationnelle, des puces d'ADNc ou d'oligonucléotides, une analyse en série de l'expression génique, un marquage enzymatique, fluorescent, radioactif, avec un colorant et / ou un anticorps.

8. Procédé selon la revendication 7, **caractérisé en ce que** l'analyse comprend en outre la mesure des intensités d'expression au cours de l'électrophorèse sur gel mono- ou bi-dimensionnelle, de la présentation différentielle, de l'hybridation soustractive, du séquençage d'ADN, d'ARN ou de protéine, de la spectrométrie de masse, et du marquage enzymatique, radioactif, avec un colorant et / ou un anticorps.

9. Procédé selon l'une quelconque des revendications 6 à 8, **caractérisé en ce que** l'analyse est réalisée au moins partiellement au moyen d'un automate adapté, par exemple un robot.

10. Procédé selon la revendication 9, **caractérisé en ce que** les taux d'expression d'au moins 100 gènes sont analysés.

11. Procédé selon l'une quelconque des revendications 1 à 10, **caractérisé en ce que** la sélection est basée sur une combinaison de l'analyse à la fois du taux d'ARNm et de l'expression de la protéine.

12. Procédé selon l'une quelconque des revendications 1 à 11, **caractérisé en ce que** la sélection est basée sur le résultat d'au moins deux rangées individuelles d'analyses.

13. Procédé selon l'une quelconque des revendications 1 à 12, **caractérisé en ce que** la sélection est réalisée de telle manière à donner une première base de connaissance comprenant seulement un ensemble de gènes sélectionnés.

14. Procédé selon l'une quelconque des revendications 1 à 12, **caractérisé en ce que** la sélection est réalisée de telle manière à donner une première base de connaissance comprenant différents sous-ensembles de gènes sélectionnés.

15. Procédé selon l'une quelconque des revendications 1 à 12, **caractérisé en ce que** la sélection est réalisée au moins partiellement de manière automatique au moyen d'un automate adapté, tel qu'un dispositif informatique.

16. Procédé selon l'une quelconque des revendications 1 à 15, **caractérisé en ce qu'**au moins deux gènes sont sélectionnés.

17. Procédé selon la revendication 16, **caractérisé en ce qu'**au moins 100 gènes sont sélectionnés.

18. Procédé selon l'une quelconque des revendications 1 à 17, **caractérisé en ce que** les régions pertinentes de méthylation comprennent les gènes complets et / ou les promoteurs, les introns, les premiers exons et / ou des amplificateurs des gènes à analyser.

19. Procédé selon l'une quelconque des revendications 1 à 18, **caractérisé en ce que** l'analyse du taux de méthylation des cytosines comprend un traitement chimique avec du bisulfite, du sulfite ou du disulfite d'hydrogène, une réaction en chaîne de la polymérase (PCR), des analyses d'hybridation, un séquençage, une spectrométrie de masse et un marquage fluorescent, enzymatique, radioactif, avec un colorant et / ou un anticorps.

20. Procédé selon l'une quelconque des revendications 1 à 19, **caractérisé en ce que** l'analyse est réalisée au moins partiellement au moyen d'un automate adapté, par exemple un robot.

21. Procédé selon la revendication 20, **caractérisé en ce que** le taux de méthylation des cytosines d'au moins 100 gènes est analysé.

22. Procédé selon l'une quelconque des revendications 1 à 21, **caractérisé en ce que** la sélection est basée sur le résultat d'au moins deux rangées individuelles d'analyses.

23. Procédé selon l'une quelconque des revendications 1 à 22, **caractérisé en ce que** la sélection est réalisée de telle manière à donner une seconde base de connaissance comprenant seulement un ensemble de gènes sélectionnés.

24. Procédé selon l'une quelconque des revendications 1 à 23, **caractérisé en ce que** la sélection est réalisée de telle manière à donner une seconde base de connaissance comprenant différents sous-ensembles de gènes sélectionnés.

25. Procédé selon l'une quelconque des revendications 1 à 24, **caractérisé en ce que** la sélection est réalisée au moins partiellement de manière automatique au moyen d'un automate adapté, tel qu'un dispositif informatique.

26. Procédé selon l'une quelconque des revendications 1 à 25, **caractérisé en ce qu'**au moins deux gènes sont sélectionnés en parallèle.

27. Procédé selon la revendication 26, **caractérisé en ce qu'**au moins 100 gènes sont sélectionnés en parallèle.

28. Procédé selon l'une quelconque des revendications 1 à 27, **caractérisé en ce que** la totalité ou une partie des gènes de la seconde base de connaissance sont ajoutés au panel des gènes.

29. Procédé selon l'une quelconque des revendications 1 à 28, **caractérisé en ce que** les informations supplémentaires au sujet des régions pertinentes de méthylation des gènes sélectionnés sont ajoutées au panel des gènes.

30. Procédé selon l'une quelconque des revendications 1 à 29, **caractérisé en ce que** les étapes a) à f) sont répétées.

31. Procédé selon la revendication 30, **caractérisé en ce qu'**il est répété pour au moins 100 fois.

32. Procédé selon l'une quelconque des revendications 30 ou 31, **caractérisé en ce que** le matériau biologique identique, le matériau biologique différent ou une combinaison de ceux-ci est utilisé dans l'étape a).

33. Procédé selon l'une quelconque des revendications 1 à 32**, caractérisé en ce que** les étapes sont réalisées dans l'ordre suivant : étape a), étape d), étape e), étape b), étape c) et étape f).

34. Procédé selon l'une quelconque des revendications 1 à 33, **caractérisé en ce qu'**il est réalisé au moins partiellement au moyen d'un automate adapté, par exemple un robot.

35. Procédé in vitro de diagnostic d'une maladie, comprenant les étapes suivantes :
a) la fourniture d'un panel de gènes selon l'une quelconque des revendications 1 à 34 ;
b) l'analyse du taux de méthylation des cytosines au niveau de sites sélectionnés sur l'ADN en se basant sur ledit panel de gènes dans le matériau biologique d'au moins un individu malade avec une maladie ou une affection médicale connue et d'au moins un sujet en bonne santé, générant de cette manière une première base de connaissance ;
c) l'analyse du taux de méthylation des cytosines au niveau de sites sélectionnés sur l'ADN en se basant sur ledit panel de gènes dans le matériau biologique d'au moins un individu malade avec une maladie ou une affection médicale inconnue, générant de cette manière une deuxième base de connaissance; et
d) la fourniture d'une troisième base de connaissance comprenant une pluralité de règles expertes pour comparer la première et la deuxième bases de connaissance ;
e) la sélection d'un type de maladie ou d'affection médicale pour le au moins un individu malade avec une maladie ou une affection médicale inconnue en se basant sur la première à la troisième bases de connaissance.

36. Procédé selon la revendication 35, dans lequel la maladie est sélectionnée à partir d'effets secondaires indésirables de médicaments, de cancers, de dysfonctionnements, de lésions ou de maladies du système nerveux central (SNC), de symptômes d'agressivité ou de troubles comportementaux, de conséquences cliniques et psychologiques de lésions cérébrales, de troubles psychotiques, d'une démence et / ou de troubles associés, de maladies cardiovasculaires, de maladies, de mauvais fonctionnements ou de lésions du tube digestif, de maladies, de mauvais fonctionnements ou de lésions du système respiratoire, d'une lésion, d'une inflammation, d'une infection, d'une immunité et / ou d'une reconvalescence, de maladies, de mauvais fonctionnements ou de lésions comme conséquences de modifications dans le processus développemental, de maladies, de mauvais fonctionnements ou de lésions de la peau, des muscles, du tissu conjonctif ou des os, de maladies, de mauvais fonctionnements ou de lésions endocrines ou métaboliques, de céphalée, et de mauvais fonctionnements sexuels ou de combinaisons de ceux-ci, d'une leucémie, d'un cancer de la tête et du cou, de la maladie de Hodgkin, d'un cancer gastrique, d'un cancer de la prostate, d'un cancer rénal, d'un cancer de la vessie, d'un cancer du sein, d'un lymphome de Burkitt, d'une tumeur de Wilms, d'un syndrome de Prader-Willi / Angelman, d'un syndrome ICF, d'un dermatofibrome, d'une hypertension, de maladies neurobiologiques pédiatriques, d'un autisme, d'une rectocolite hémorragique, d'un syndrome du X fragile et d'une maladie de Huntington.
